# EUROPEAN PATENT APPLICATION

(11) **EP 1 469 308 A1**
(43) Date of publication of application: **20.10.2004**
(21) Application number: 02786130.1
(22) Date of filing: 17.12.2002
(51) Int. Cl.: G01N 33/15, G01N 33/50

(54) **METHOD OF SCREENING DRUG**

(30) Priority: 18.12.2001 JP 2001384683
(71) Applicant: National Institute of Advanced Industrial Science and Technology, Tokyo 100-8901 (JP); Japan Science and Technology Agency, Kawaguchi-shi, Saitama 332-0012 (JP)
(72) Inventor: SUZUKI, Masashi; Nat.Inst.of Adv.Ind.Sc.& Technlgy, Tsukuba-shi, Ibaraki 305-8566 (JP); KOIKE, Hideaki; Nat.Inst.of Adv.Ind.Sc.& Technlgy, Tsukuba-shi, Ibaraki 305-8566 (JP)
(74) Representative: VOSSIUS & PARTNER
(86) International application number: PCT/JP2002/013193
(87) International publication number: WO 2003/052408

(57) **Abstract**

This invention provides a method of screening for pharmaceutical agents that regulate at least one of metabolism or growth of, or infection by microorganisms, which comprises selecting ligands of an Lrp-like protein based on 3D structural information of the protein.

## Description

### Technical Field

The present invention relates to a method of screening for pharmaceutical agents that regulate at least one of metabolism or growth of, or infection by microorganisms.

### Background Art

Analyses of genomic DNA sequences of microorganisms have revealed that a group of proteins that are very similar to each other are commonly found in many microorganisms. Among this group of proteins, function of *E. coli* Lrp (leucine responsive regulatory protein) has been elucidated to the greatest extent. Another *E. coli* protein, AsnC, is classified into the same group. The amino acid sequences of the two proteins are similar, and their functions too are similar. Thus, AsnC is regarded as an Lrp-like protein. Hereinafter, these homologous proteins are collectively referred to as the "group of Lrp proteins" or "Lrp-like proteins."

In response to extracellular nutritional conditions indicated by the extracellular concentration of leucine, *E. coli* Lrp regulates its metabolism. This mechanism is referred to as the "feast/famine" regulation. If extracellular nutritional conditions improve, *E. coli* shifts from the "famine" state to the "feast" state. Specifically, *E. coli* suspends movement to other sites, actively absorbs nutrients through the cell membrane, terminates autotrophic metabolic pathways for amino acid synthesis and the like, and initiates cell division and growth. Finally, its infectivity too is changed. Experimentally, several dozen metabolism-associated operons have been identified as being regulated by Lrp, the number of such genes may well reach 10% of the whole genes coded in the *E. coli* genome. Similarly, the AsnC protein, an *E.coli* Lrp-like protein, regulates amino acid synthesis and DNA replication in response to the concentration of asparagine.

In order to achieve such regulation, Lrp-like proteins recognize promoter DNA sites positioned upstream of the genes regulated, thereby activating or inactivating transcription of these genes. Association with or dissociation from promoter DNA sites by Lrp-like proteins is regulated, in many cases, by extracellular nutritional conditions. Types of small molecules that mediate extracellular nutritional conditions (hereafter referred to as "ligands") will introduce structural changes into Lrp-like proteins, thereby affecting their ability to associate with promoter DNA sites.

However, 3D (i.e., three dimensional) structural information of Lrp-like proteins adequate for elucidating the aforementioned mechanisms, or that adequate for modeling or screening of novel ligands was not elucidated. In general, X-ray diffraction analysis, NMR spectroscopy, electron microscopy, and the like are available for studying the 3D structures of proteins. However, the sizes of the target molecules are restricted in NMR spectroscopy and the accuracy of electron microscopy is insufficient. Thus, X-ray diffraction analysis is the method most suitable for analyzing the 3D structures of assemblies of Lrp-like proteins. Crystallization of proteins, that is required in X-ray diffraction analysis, is generally difficult.

Accordingly, crystallization of *E. coli* Lrp has been attempted in Europe and the United States, but there has been no report of success. An Lrp-like protein derived from another type of microorganism has been crystallized in Europe, and the 3D combination of secondary structural elements has been reported for the protein. However, the resolution is as low as approximately 3 Å, and the R factor is higher than 30%. No detailed experimental information is available for the geometry of amino acid side chains, which is important for analyzing interaction with potential ligands.

### Disclosure of the Invention

The aim of the present invention is by analyzing the 3D structure of an Lrp-like protein using the 3D coordinates of atoms in side chains thereof, to identify part of the 3D structure that can be targeted upon modeling or screening ligands. Furthermore, the present invention provides a method of screening for pharmaceutical agents that can regulate one of metabolism or growth of, or infection by microorganisms based on the aforementioned 3D structural information.

By intensive studies, the present inventors have succeeded in crystallizing one of the fourteen Lrp-like proteins present in *Pyrococcus* sp. OT3 (alias: *Pyrococcus* *horikoshii* or *Pyrococcus shinkaii*, Japan Collection of Microorganisms; registration number: JCM 9974), and the 3D structure thereof has been determined at a high resolution using X-ray diffraction analysis. Based on this 3D structural information, the present inventors have established a method of screening for pharmaceutical agents that will function as antibiotics by targeting transcriptional regulation by Lrp-like proteins of the genes that are involved in metabolism or growth of, or infection by the microorganisms.

More specifically, the present invention relates to the following (1) to (12).
(1) A method of screening for pharmaceutical agents that regulate at least one of metabolism or growth of; or infection by microorganisms, which comprises selecting ligands of an Lrp-like protein based on the 3D structural information of the protein.
(2) The method according to (1) above, wherein the 3D structural information is information concerning gaps or pockets generated by assembly of Lrp-like protein monomers.
(3) The method according to (1) or (2) above, wherein the 3D structural information is information obtained from 3D atomic coordinates determined by X-ray diffraction analysis.
(4) The method according to (3) above, which comprises the following steps:
   1) determination of the 3D atomic coordinates of the Lrp-like protein by applying X-ray diffraction analysis to the protein crystallized;
   2) processing of the 3D atomic coordinates using a program designed for analyzing protein 3D structures, thereby obtaining graphical and/or numerical information concerning the 3D structure of the Lrp-like protein;
   3) deduction of the 3D structural information on gaps or pockets generated by assembling of monomers of the Lrp-like protein, by using the graphical and/or numerical information; and
   4) identification of ligands which will interact with the Lrp-like protein and thus will be usable as pharmaceutical agents, by using the 3D structural information.
(5) The method according to any one of (1) to (4) above, wherein the pharmaceutical agents that regulate at least one of metabolism or growth of, or infection by microorganisms, are antimicrobial agents.
(6) The method according to any one of (1) to (5) above, wherein the Lrp-like protein is derived from a microorganism belonging to the genus *Pyrococcus.*
(7) The method according to (6) above, wherein the microorganism is *Pyrococcus* sp. OT3 (JCM 9974).
(8) The method according to (7) above, wherein the 3D atomic coordinates comprise the data shown in Table 1.
(9) A recording medium in which the following a) and/or b) are recorded:
   a) 3D atomic coordinates of an Lrp-like protein; and
   b) graphical and/or numerical information concerning the 3D structure of an Lrp-like protein obtained by analyzing the 3D atomic coordinates using a program designed for analyzing protein 3D structures.
(10) The recording medium according to (9) above, wherein the Lrp-like protein is derived from a microorganism belonging to the genus *Pyrococcus.*
(11) The recording medium according to (10) above, wherein the microorganism is *Pyrococcus* sp. OT3 (JCM 9974).
(12) The recording medium according to (11) above, wherein the 3D atomic coordinates comprise the data shown in Table 1.

The present invention relates to a method of screening for pharmaceutical agents that regulate at least one of metabolism or growth of, or infection by microorganisms, wherein a ligand interacting with an Lrp-like protein is selectively identified using 3D structural information concerning the aforementioned protein.

Hereafter, the present invention is described in detail.

### 1. An Lrp-like protein

In the present invention, the term "Lrp-like protein" refers to the leucine responsive regulatory protein (Lrp) of *E. coli* or a homologue thereof derived from any microorganism. Lrp is an *E. coli* protein, which regulates metabolism of this organism in response to the extracellular concentration of leucine. The term "Lrp homologues" refers to a group of proteins whose amino acid sequences and functions (i.e., response to extracellular nutritions and regulation of metabolisms) are similar to those of *E*. *coli* Lrp. Examples of Lrp homologues include *E. coli* AsnC protein, LrpA of the genus *Pyrococcus*, and Sa-lrp of the genus *Sulfolobus.*

Lrp-like proteins function by binding to promoter DNA sites positioned upstream of the genes regulated by Lrp-like proteins, thereby activating or inactivating transcription of these genes. It is known that association with or dissociation from promoter DNA sites by Lrp-like proteins is regulated by types of small molecules that reflect the extracellular nutritional condition. These small molecules cause structural changes of the Lrp-like proteins, thereby affecting their abilities to associate with the promoter DNAs. In this specification, "small molecules that bind to Lrp-like proteins and cause structural changes of the proteins" are referred to as "ligands".

Application of the present invention is not restricted to any particular Lrp-like protein, but this invention can be applied to any organism which has an Lrp-like protein. Such microorganisms include, *E. coli*, *Bacillus subtilis, Thermoplasma*, and *Sulfolobus.* Microorganisms in the genus *Pyrococcus,* particularly *Pyrococcus* sp. OT3 (JCM 9974), can be targeted.

### 2. Three dimensional structural information

In the present invention, the term "3D structural information of an Lrp-like protein" refers to information concerning the 3D structure of an Lrp-like protein in a crystal. This 3D structural information includes information concerning both the monomeric form of the Lrp-like protein and its assembly form. The information also includes information concerning the Lrp-like protein both in a form associating with a ligand and the form dissociating.

In the present invention, information concerning gaps or pockets created upon assembling of monomers of an Lrp-like protein is particularly useful. The term "gap" refers to an empty space created between monomers of an Lrp-like protein, between atoms or between amino acid residues. The term "pocket" refers to another empty space created around the center of the assembly of an Lrp-like protein. These spaces are important for interaction with "ligands", and thus are used for screening of pharmaceutical agents.

The 3D structural information can be obtained using, for example, X-ray diffraction analysis, NMR spectroscopy, or electron microscopy. Since the size of molecules that can be analyzed by NMR spectroscopy is limited, and since the resolution obtainable by electron microscopy is limited, use of X-ray diffraction analysis is appropriate. In X-ray diffraction analysis, phase of diffraction spots can be determined, if not only the original crystals of protein but also their isomorphous derivatives with heavy-atoms are made. For obtaining such derivatives, the original crystals are soaked into at least two solutions each containing a heavy atom (for example, platinum or gold). Diffraction from the original and derivatives are analyzed as a set.

Using the obtained diffraction, an electron density map is computed, using commercially available programs such as programs in the CCP4 package, and the 3D atomic coordinates can be determined, so that they best fits the electron density map. An example of such "3D atomic coordinates" is described using the Protein Data Bank (PDB) format in Table 1. The PDB format is an international standard for describing the 3D coordinates of atoms in proteins.

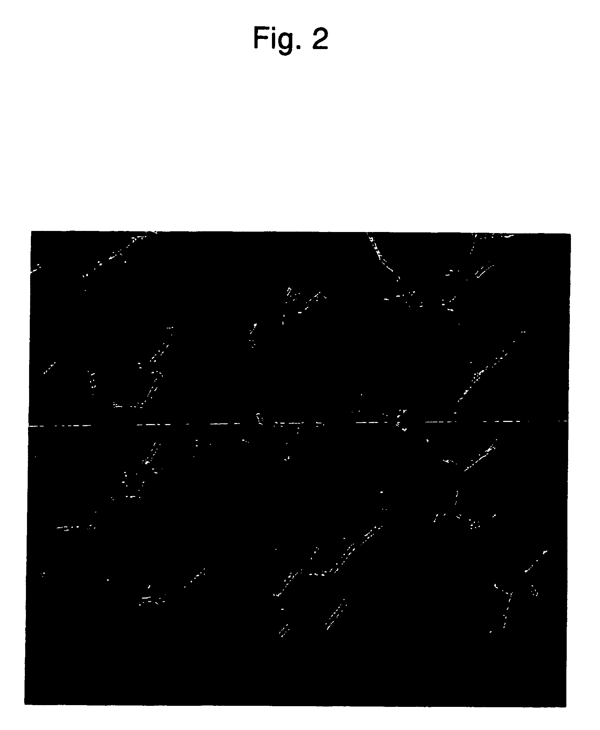

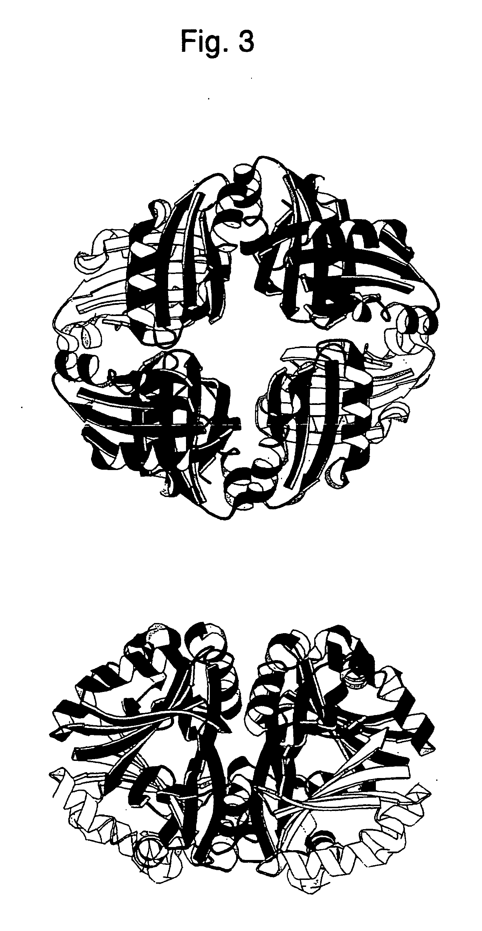

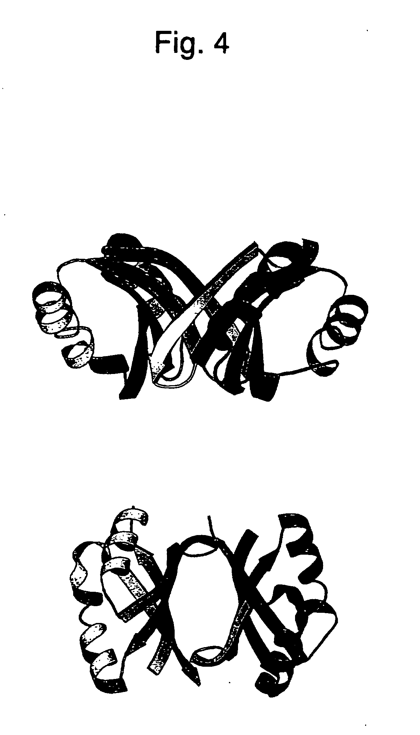

Subsequently, the 3D atomic coordinates can be processed using a program designed for analyzing protein 3D structures, thereby obtaining graphical information and/or numerical information concerning the 3D structure of an Lrp-like protein. Examples of such a "program designed for analyzing protein 3D structures" include CCP4, O, X-PLOR, MolScript, Insight II, and Grasp Programs. The term "graphical information" refers to any information represented as images, e.g., in the form of ribbon diagrams (e.g., Figs. 3 and 4) or in the form of wire models (e.g., Fig. 2). Similarly, "numerical information" refers to any information expressed by combining numbers, e.g., values such as those defining the diameters of crystals, the widths or depths of pockets or gaps.

### 3. Screening for ligands

The atomic coordinates and the graphical and/or numerical information concerning the 3D structure of the Lrp-like protein will determine a large continuous empty space expanding from a pocket to gaps, thereby defining the coordinates of hydrophobic or hydrophilic chemical groups facing the empty space. Thus, they can be useful for screening for ligands that will act on the Lrp-like protein.

As is described in the "Examples", association with a natural ligand can introduce 3D structural changes to an Lrp-like protein, affecting association with or dissociation from a promoter DNA site by the Lrp-like protein. Accordingly, such a ligand can be used as a pharmaceutical agent that artificially regulates metabolism or growth of, or infection by the microorganism. Preferably, the pharmaceutical agent will terminate metabolism or growth of, or infection by the microorganism, thereby preventing pathogenic function of the microorganism.

No Lrp-like protein has been found in an eukaryote, including of humans. Thus, it is likely that a ligand interacting with an Lrp-like protein will not cause any serious side effect to humans. If such a ligand function as an antibiotic, its target protein and mechanism of action will become different from those of known antibiotics. This might prevent infection by microorganisms which are resistant to known antibiotics; such infection is becoming more and more serious.

### 4. A recording medium

The present invention also provides a medium in which the 3D structural information of the Lrp-like protein is recorded. Specifically, this medium records the following a) and/or b) thereon:
a) the 3D atomic coordinates of the Lrp-like protein; and
b) graphical and/or numerical information concerning the 3D structure of the Lrp-like protein obtained by analyzing the 3D atomic coordinates using a program designed for analyzing protein 3D structures.

In this specification, the 3D structural information of a particular Lrp-like protein derived from *Pyrococcus* sp. OT3 is shown as a preferable example of such data recorded on a medium. The term "medium" indicates any one of the media that can record the aforementioned information, such as a CD-ROM, an optical disk, an optical file, a MO, a minidisk, a flexible disk, and a computer hard disk.

The aforementioned medium provides information necessary for screening for ligands interacting with Lrp-like proteins, and thus can be used as a tool for screening for pharmaceutical agents that regulate at least one of metabolism or growth of, or infection by microorganisms.

### Brief Description of the Drawings

Fig. 1 shows crystals of the Lrp-like protein.
Fig. 2 shows part of the electron density map calculated for the Lrp-like protein (i.e., the 2 Fo-Fc map; counter level: 1.0) and the final model of the protein fitted into the map.
Fig. 3 shows two views of a ribbon diagram showing the 3D structure of an octameric assembly of the Lrp-like protein, determined by X-ray crystallography (*see* Fig. 2).
Fig. 4 shows two views of another ribbon diagram showing the 3D structure of a dimer of the Lrp-like protein in the crystal.
Fig. 5 shows profiles of gel filtration of the Lrp-like protein: black-purified by a mild method (1), red-purified by a rigorous condition (2): *see* Examples for understanding these purification methods.
Fig. 6 indicates the electron densities (red) identified as corresponding to two molecules of the natural ligand most likely, that had been absorbed into octamers of the Lrp-like protein: subfigure b is a side view, while the top views shown in (a), and subfigure c is an enlargement of (b).
Fig. 7 shows crystals of the Lrp-like protein before or immediately after (upper), or several hours after (lower) soaked into solution containing 10 mM Leu (a) or 10 mM Asp (b).Fig. 8 shows profiles of gel filtration of the Lrp-like protein in in the presence of various amino acids (a and b) or some other compounds (c), i.e., malic acid (red), 2-oxoglutaric acid (green), and oxaloacetic acid (blue), or in their absence (black).
This description includes the contents disclosed in the description of Japanese Patent Application No. 2001-384683, which is a priority document of the present application.

### Best Mode for Carrying out the Invention

The present invention is hereafter described in more detail, referring to particular examples, although the present invention is not limited to this particular form.

### [Example 1] Crystallization of the Lrp-like protein

### (1) Construction of a vector for expressing the Lrp-like protein

The following primers were synthesized in order to amplify a DNA fragment coding the Lrp-like protein (*llp*) gene by PCR using the genomic DNA of *Pyrococcus* sp. OT3 (JCM 9974) as the template. PCR was carried out using the reaction solution, 20 µl, LA Tag (Takara Shuzo Co., Ltd.) and GeneAmp PCR system 9600 (Perkin Elmer): each cycle consisting of denaturation at 94°C, annealing at 55°C, and elongation at 72°C, and repeating this cycle 30 times.

The amplified DNA fragment was cleaved using restriction enzymes *Nde*I (Takara Shuzo Co., Ltd.) and *Bam*HI (Takara Shuzo Co., Ltd.), and the fragment coding the gene was separated from the rest by electrophoresis using an agarose gel. The DNA fragment coding the *llp* gene was ligated with an expression vector, pET3 (Novagen), cleaved using restriction enzymes, *Nde*I and *Bam*HI, thereby constructing a expression vector (pET3-LRPS01): 10 µl of the reaction solution, 660 mM Tris-HCl buffer (pH 7.6) containing 66 mM MgCl₂, 100 mM DTT, and 1 mM ATP, was kept at 16°C overnight, in the presence of T4 DNA ligase (Takara Shuzo Co., Ltd.).
Primers:

ForwardStrand: 5'-TGGTGATGACATATGGTGACGGCCTTTATCCTG-3'
(SEQ ID NO: 1)

Reverse Strand: 5'-GAACGGATCCATCAAATTGCTATCATAGTCGAGGTC-3'
(SEQ ID NO: 2)

### (2) Expression of the Lrp-like protein using E. coli cells

The vector designed for expressing the *llp* gene, pET3-LRPS01, was introduced into *E. coli* cells *strain* BL21 (DE3) (Novagen). The *E. coli* cells were cultured in 8 ml of the 2 x YT medium containing 50 mg/ml ampicillin at 37°C overnight, inoculated into 8 1 of the same medium, and allowed to grow until the absorbance of the medium reached approximately 0.75 at 600 nm. Subsequently, 2 mM isopropylthiogalactoside (IPTG) was added to induce expression of the Lrp-like protein. After additional culture for 4 hours, cells were collected by centrifugation at 500 rpm for 5 minutes using a centrifuge (Beckman Avanti J-25, 7,).

### (3) Purification of the Lrp-like protein

The cells expressing the Lrp-like protein were suspended into 250 ml of 100 mM Tris-HCl buffer (pH 7.0) containing 1 mM EDTA, and treated using a French press (SLM). After centrifugation for 30 minutes at 4°C at 25,000 rpm, using a centrifuge (Beckman Avanti J-25) and a rotor (JA-25.5), the supernatant was subjected to a thermal treatment at 75°C for 1 hour. After another centrifugation at 4°C at 25,000 rpm for 30 minutes, the supernatant was dialyzed against 30 mM Tris-HCl buffer (pH 7.0), and subjected to further purification: by twice repeating the following process, i.e., anion exchange column chromatography followed by gel filtration.

The supernatant was applied to an anion exchanger (1.6 x 18 cm, Resource Q, Pharmacia), in a column, equilibrated with 30 mM Tris-HCl buffer (pH 7.0), and the column was washed with the same buffer. Subsequently, the protein was eluted with an linear gradient, 0-2 M of NaCl in 30 mM Tris-HCl buffer (pH 7.0), with a flow rate of 2 ml/min, using FPLC column (Pharmacia). Each solution, The solution eluted from the column was fractionated into fractions of 4 ml, was collected, each, and those identified as containing the Lrp-like protein were identified using SDS electrophoresis and were stored.

The stored protein once stored was dialyzed against 100 mM Tris-HCl (pH 7.0) and subject to gel filtration. The protein solution was applied to a gel column (2.6 x 60 cm, Superdex 75, Pharmacia) in a column (2.6 x 60 cm), equilibrated with 100 mM Tris-HCl buffer (pH 7.0), and eluted with the same buffer with the flow rate 1 ml/min using the (FPLC system, (Pharmacia). The fractions, obtained (each of, 2 ml each,) were subjected to SDS electrophoresis, and those containing the Lrp-like protein were identified and stored. The above processes of anion exchange chromatography and gel filtration were alternately carried out two times alternatively, and the protein was purified to a quality with which so that a single band was observed when subjected to SDS electrophoresis.

### (4) Crystallization of the Lrp-like protein

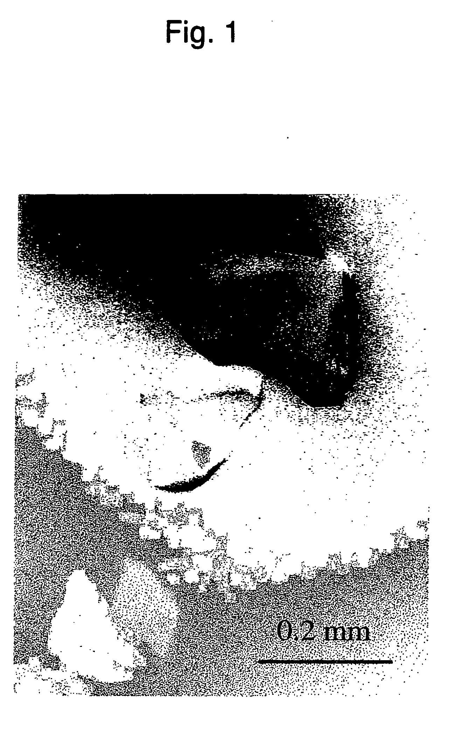

The purified Lrp-like protein, 20 mg/ml in 10 mM Tris-HCl buffer (pH 7.0), was used for crystallization. Crystallization was carried out using 24-well crystallization plates (Hampton Research) by a vapor-diffusion method, using a sitting-drop technique. Specifically, 4 (l of the protein solution was mixed with 4 (l of the same solution as placed in the reservoir (100 mM citrate buffer (pH 4.0) containing 10% by weight of PEG 6000 and 1.0 M lithium chloride). This crystallization solution was allowed to equilibrated with 0.8 ml of the 100 mM citrate buffer (pH 4.0) containing 10% by weight of PEG 6000 and 1.0 M lithium chloride placed in the reservoir solution, by vapor diffusion at a constant temperature of 5°C. In approximately a week, crystals in pyramid-like hexagonal forms (of the sizes of approximately 0.2 mm) were obtained (Fig. 1).

### [Example 2] Analysis of the 3D structure of the Lrp-like protein

### (1) X-ray diffraction analysis

In order to determine the 3D structure of the Lrp-like protein by a heavy atom isomorphous replacement method using heavy atom derivatives, the original crystals obtained (*see* Example 1) were soaked into solutions containing 10 mM K₂[Pt(CN)₆] or 0.1 mM K[AuCl₄] at 5°C for approximately 2 days.

Four sets of diffraction were measured from a total of four sets, i.e., two sets of the original crystals and one set of each two sets, each from of the two heavy atom derivatives (platinum and or gold), were measured by using 1) an ordinary (i.e., conventional a rotating anode type) X-ray source and 2) two sets from the original crystals using the ordinary X-ray source or a synchlotron radiation X-ray source.

### 1) Analysis using an ordinary (i.e., a conventional rotating anode type) X-ray source

Diffraction from a set of the original crystals, and the two sets of heavy atom derivatives (platinum and gold), were measured, using an ordinary (i.e., a conventional rotating anode type) X-ray source at room temperature. Each crystal was placed inside a quartz capillary (1.5 mm in the diameter, TOHO), and an excess solvent was removed. After sealing the both ends of the capillary with wax, the capillary was placed inside an X-ray diffractometer (R-AXIS IV, Rigaku Denki). An X-ray source was produced by rotating thean anode at 50 kV and 100 mA (UltraX 18, Rigaku Denki), and the diffraction was recorded using adiffractometer.

### 2) Analysis using a synchlotron radiation X-ray source

In order to obtain more diffraction containing information at higher resolutions, the original crystals were frozen and kept in this state while measuring their diffraction using a synchrotron radiation X-ray source, i.e., the Hyogo beamline BL24XU at using a synchrotron radiation X-ray source, i.e., the Hyogo beamline BL24XU at SPring8 (Hyogo, Japan). The crystals were soaked into a solution containing a cryo-protectant, 100 mM citrate buffer (pH 4.0) containing 20%, by weight, of glycerol, 14%, by weight, of PEG 6000, and 1.0 M lithium chloride for several minutes, placed inside a 0.2 mm-mount loop (Cryoloop, Hampton Research), and frozen using liquid nitrogen at -196°C. During the measurements, the crystals were kept at another temperature of -173°C using a device (Rigaku Denki) designed to spray cold air to crystals. Diffraction was recorded by a diffractometer (R-AXIS IV, Rigaku Denki) using synchrotron X-ray of a wave length of 0.834 Å.

### (2) Data analysis

The recorded diffraction was processed using a program designed for the diffractometer and the programs in the CCP4 package (Collaborative Computational Project, Number 4, Acta Crystallographica D50, 760-763, 1994). Consequently, the space group of the crystals was identified to P3₂21 or P3₁21, and the unit cell lengths were identified: a = b = 96.3 Å, and c = 97.1 Å. Based on these lengths, it was concluded that each asymmetric unit of these crystals contained three to six molecules of the Lrp-like protein.

### (3) Building an initial model

The diffraction from the original and the two heavy atom derivatives (platinum and gold) were integrated using programs in the CCP4 package, and used for the subsequent computational analysis. Positions of the heavy atoms in the derivatives were determined using difference Patterson maps. The gold and platinum derivatives, respectively, had two and three heavy metal atoms per asymmetric units. Numbers, positions, and occupancies of the heavy atoms were refined using the MLPHARE program in the CCP4 package. The overall figure of merit at this stage was calculated as 0.43 at 3.0 Å.

By using the phase obtained as above, and by using the isomorphous replacement method, an electron density map was calculated. By examining the map, the phase was improved by the methods of solvent flattening and histogram matching, using the DM program in the CCP4 package. In the revised electron density map, calculated using the improved phase, electron densities corresponding to right-handed α-helices were identified appropriately only when four molecules of the protein were assumed in each asymmetric unit and when these units were related by the space group P3₂21. A model was made by assembling four identical monomers (each consisting of 72 alanine residues), so that the model would best fit to the electron density map using program O (Jones T. A., Zou J. Y., Cowan S. W., and Kjeldgaard M., Improved methods for binding protein models in electron density maps and the location of errors in these models, Acta Crystallographica A47, 110-119, 1991).

### (4) Refinement of the model

The initial model was refined using the X-PLORE program (Brünger, A. X-PLORE v3.1 Manual (Yale University, New Haven, 1992)), so that the model would best satisfy both the diffraction data experimentally obtained and the standard geometric parameters (Engh and Huber) that were expected for protein 3D structures in general. The model was further refined using program O to correct the part of the model where large deviations from the electron density map were observed. Until this stage, the four monomers in the asymmetric unit were kept identical to each other. The R factor indicating the difference between the experimental data and the model was 39.1% at this stage.

In order to further refine the model, the high-resolution data obtained at SPring8 BL24XU were used. The aforementioned alanine model was refined using the X-PLORE program using data up to 3.0 Å, and other data up to 2.0 Å were incorporated while improving the phase using the WARP program (Perrakis, A., Sixma, T. K., Wilson, K. S., and Lamzin, V. S., WARP: Improvement and extension of crystallographic phases by weighted averaging multiple refined dummy atomic models, Acta Crystallographica D53, 448-455, 1997). Using the improved phase, a new electron density map was calculated, where electron densities of most of the atoms in the protein, including the atoms of side chains of the amino acid residues, were clearly identified. Based on this electron density map, another 3D structural model was made by including the amino acid side chains.

Further refinements were carried out at a resolution of 1.8 Å, by modeling solvent molecules so that there would match with electron densities that were clearly identified but not interpretable as any atoms in the protein, which will be discussed further in the following paragraph. At the final stage of refinement, the four monomers in the asymmetric unit were allowed to adopt non-identical 3D structures.

### (5) Determination of the final model

The final model (Figs. 2 and 3) was assembled by four independent monomers of the Lrp-like protein. Each monomer comprises all the residues except for the first amino acid residue, i.e., methionine, at the N-terminal end. The R factor, evaluating the consistency between the experimental data (20 - 1.8 Å) and the final model, was calculated as 21.2%, indicating a high accuracy of the 3D model. The PROCHECK program in the CCP4 package was used to calculate a Ramachandran plot. In the plot, 98.5% of all the residues were found in energetically-most stable regions. The average temperature factor (B-factor) was as small as 17.3, indicating that all the atoms were determined unambiguously, except for some side chains, which were positioned on the surface, and thus expected as possessing real flexibilities for movements.

### [Example 3] Analysis of the assembling of the Lrp-like protein by gel filtration

It has been reported that many Lrp-like proteins derived from microorganisms, including *E. coli* form dimers or tetramers in solution. The Lrp-like protein expressed in *E. coli* was analyzed by gel filtration in order to estimate sizes of its assemblies in solution.

Specifically, the Lrp-like protein purified for the crystallization was diluted by 50 mM Tris-HCl buffer (pH 7.0) containing 300 mM sodium chloride, yielding a concentration of approximately 100 µM. Gel filtration was carried out at a flow rate 1 ml/min (LC Module I plus System, Waters) using a matrix (Protein Pak 125, 7.8 x 300 mm, Waters) and the same buffer, i.e., 50 mM Tris-HCl buffer (pH 7.0) containing 300 mM sodium chloride. Retention of the protein was recorded by measuring the UV absorption at 220 nm.

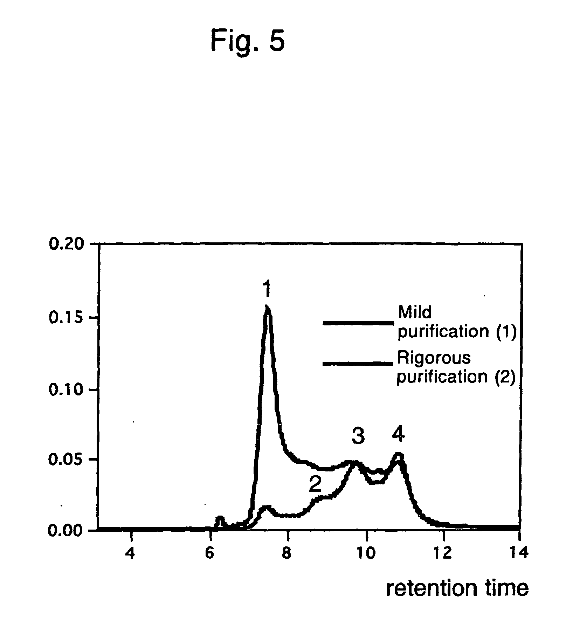

A multiple number of peaks were observed (Fig. 5, purification 1). In general upon gel filtration, assemblies of larger sizes will be retained for a shorter time. Accordingly, the first peak (peak 1 in Fig. 5) might correspond to an octamer, and other peaks retained longer should correspond to smaller assemblies. This protein forms a variety of assemblies up to an octamer depending on the condition. The function of the octamer may be different from those of smaller assemblies.

### [Example 4] Analysis of the 3D coordinates of atoms in the Lrp-like protein

### (1) The 3D coordinates of atoms in the Lrp-like protein

The atomic coordinates of the 3D structure of the Lrp-like protein determined are shown using the protein data bank (PDB) format (Table 1), describing the four independent monomers of the protein (A to D) in the asymmetric unit and 196 water molecules. Lines 1 to 7 of Table 1 specify the crystal symmetry to repeat the asymmetric unit, as is generally the case of any crystals. From line 8, eleven parameters (i) - (xi) described from left to right are: (i) serial numbers of the atoms (1 to 2,556); (ii) types of atoms, e.g., C the carbon, further differentiated as, for example, CA, CB, indicating the positions inside the amino acid residues, also including information concerning chemical bondings; (iii) assignments of the atoms to 20 types of amino acid residues, using the 3 letter code, e.g., VAL for valine) or, (HOH) for the atoms belonging not to amino acid residues but to water molecules, and, in addition, their assignments to the four monomers (A to D) or to water molecule (W); (iv) amino acid residue numbers counted from the N terminus in each monomer, or numbers 1 to 196 for the water molecules; (v) the X coordinates in angstrom; (vi) the Y coordinates in angstrom; (vii) the Z coordinates in angstrom; (viii) occupancies (i.e., probabilities of the atoms occupying the given coordinates); (ix) isotropic temperature factors (i.e., measures of the flexibilities of atoms for thermal movements); (x) the atomic numbers defined by the periodic table (e.g., 6 for C, 7 for N, and 8 for O); and (xi) types of the atoms, the same as in (ii) (e.g., C, O, and N).

The atom types (e.g., C, O) described above are in general those described by a nomenclature of the IUPAC-IUB Nomenclature, but in (ii), A, B, C, D, E, Z, and H were used (e.g., CA or CB) instead of α, β, γ, δ, ε, ζ, and η. The additional oxygen atoms present at the C termini were labeled as OXT (OT).

### (2) Analysis of the 3D coordinates

### 1) Assembling by the Lrp-like protein

By analyzing the data shown in Table 1, it was found that each monomer of the Lrp-like protein is composed of a four-stranded β sheet and two α-helices (Fig. 4). Each pair of monomers assemble into a dimer, by forming a single β-sheet composed of eight strands, i.e., four strands from each monomer (Fig. 4). In the crystal, each set of 4 dimers further assemble, thereby forming a disk-like octamer (Fig. 3). The diameter of the octamer disk is approximately 60 Å and the thickness thereof is approximately 40 Å.

### 2) Characteristics of the 3D structure

At the center of the disk-like octamer of the Lrp-like protein, there exists a pocket, the overall shape resembling a Japanese 50-yen coin having a hole in the center. An empty space extends from this central pocket to the four gaps, each formed between a pair of dimers, as a whole, forming a cross-like shape (*see*, Fig. 3 upper). The boundary of this empty space, the types of amino acid side chains surrounding the empty space, and the coordinates of these side chains conform the 3D structural information critical for screening for ligands interacting with the Lrp-like protein.

The boundary of the central pocket resembles a cylinder of the diameter approximately 12 Å and the height approximately 30 Å, which is large enough for containing two amino acid molecules. Eight sets of amino acid side chains, each from each of the eight monomers assembling, valine 9, glutamic acid 37, tyrosine 38, methionine 68, and serine 70, are facing this pocket: (here numbers, such as 9, 37, or 38, are those of amino acid residues, counted from the N terminus in each monomer (*see* Table 1). Also, residues 37, 38, and 68 are positioned at the border connecting the pocket to the gaps.

Each of the four gaps formed between the pairs of the dimers is large enough to accommodate two amino acids, or able to do so by being expanded by the two amino acids. Each gap was surrounded by two sets of side chains of the two dimers, glutamic acid 15, valine 33, tyrosine 35, aspartic acid 39, leucine 49, aspartic acid 53, isoleucine 56, threonine 57, arginine 61, threonines 69 and 71, and isoleucines 73 and 75.

### 3) Application of the 3D information to screening for ligands

If a ligand binds to the empty space extending from the pocket to the gaps, and if the ligand is completely complementary to part of the space and thus able to fit into, the ligand will stabilize the octamer thereby activating the functions of Lrp-like protein as an octamer. However, if the ligand is larger than the space and binds there with a high constant, it will enlarge the empty space, thereby dissociating the octamer terminating the functions of the Lrp-like protein as an octamer, and activating functions as smaller assemblies. Most likely, he empty space will be the site for interacting with natural ligands.

As has been described, graphical and/or numerical information of the Lrp-like protein obtained from the 3D coordinates shown in Table 1 determine the boundary of the empty space extending from the pocket to the gaps and specify the positions of atomic groups, hydrophobic or hydrophilic, facing the space. Thus, this information provides a useful platform for screening for ligands interacting with the Lrp-like protein.

### [Example 5] The position of a natural ligand present in the crystal of Lrp-like protein

### (1) The presence of a natural ligand in the crystal of the Lrp-like protein

The presence of a natural ligand in the crystal of the Lrp-like protein (Example 1) was found and confirmed as described below.

### 1) Purification of the Lrp-like protein using a method different from what was described in Example 1

The protein was purified by a more rigorous method with additional uses of ammonium sulfate fractionation and hydrophobic column chromatography. Ammonium sulfate fractionation was carried out after the heat treatment and centrifugation. Namely, ammonium sulfate was added to the supernatant, yielding 40% saturation. The solution was centrifuged at 4°C at 18,000 rpm for 15 minutes. Ammonium sulfate was further added to the supernatant, yielding 80% saturation. After the centrifugation at 4°C at 18,000 rpm for 15 minutes, the sediment was dissolved in 30 ml of 30 mM Tris-HCl buffer (pH 7.0) and dialyzed against 30 mM Tris-HCl buffer (pH 7.0). Hydrophobic column chromatography was carried out after the anion exchange column chromatography and the gel filtration described in Example 1. Namely, after the gel filtration, ammonium sulfate was added to the protein solution, yielding a concentration of 1.6 M, and applied to a column (1.6 x 18 cm), containing Butyl-Toyopearl 650 M (Tosoh) pre-equilibrated with 50 mM Tris-HCl buffer (pH 7.0) containing 1.5 M ammonium sulfate., The column was washed with the same buffer. Subsequently, the protein was eluted using a line, 1.5-0 M, gradient of ammonium sulfate in 50 mM Tris-HCl buffer (pH 7.0) with the flow rate 2 ml/min: the FPLC column (Pharmacia) was used. Fractions, 4 ml each, were subjected to SDS electrophoresis and those identified as containing the Lrp-like protein were stored.

### 2) Changes between assembly forms

By the rigours purification the apparent molecular weight in solution of the Lrp-like protein was changed, suggesting changes in the assembly form, using gel filtration (Fig. 5, purification 2). Compared with the original method employed in Example 1, in the modified method the protein is exposed to the environments accerelating, in general, denaturation of proteins. Thus, the change in the assembly form can be explained, if a ligand present in *E. coli* cells was binding to the Lrp-like protein, thereby stabilizing its assembling, after the purification by the original method, while, if the ligand was removed from the protein by the modified method. If the above idea that a natural ligand which can accelerate assembling of the Lrp-like protein is present in *E. coli* cells and that this ligand was incorporated into the assembly of the Lrp-like protein upon expression of this protein in *E. coli* cells, it is expected that the crystals obtained using the Lrp-like protein purified by the original method should have been those of the complex of the Lrp-like protein and the *E. coli* ligand rather than those of the Lrp-like protein alone.

### (2) The position of the natural ligand present in the crystal of the Lrp-like protein

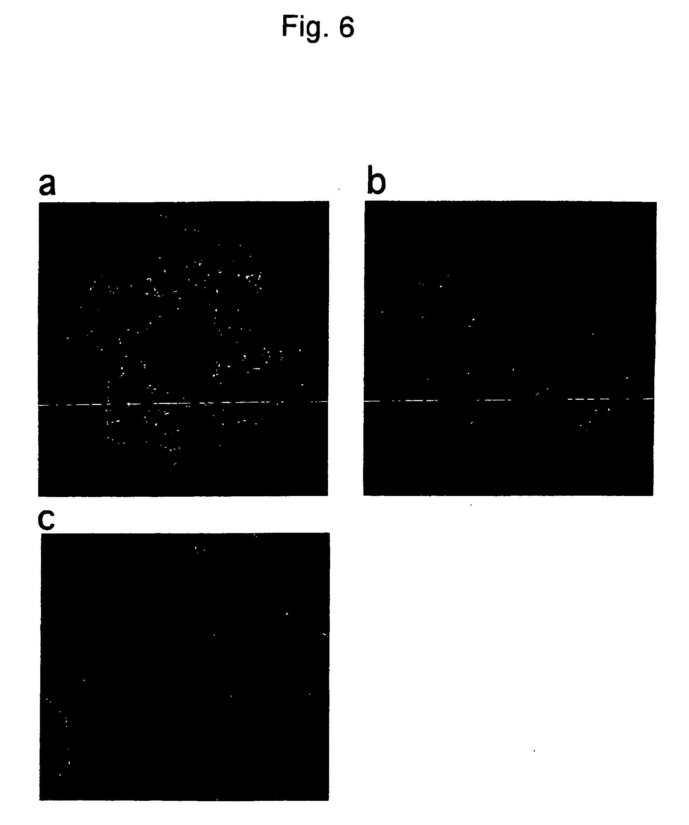

The electron density map was re-analyzed, seeking for a density which might correspond to the *E. coli* ligand. Other than the densities clearly corresponding to the protein, there existed extra densities, which then were interpreted as those of the 196 water molecules. By re-examining these extra densities, electron densities which likely corresponded to two molecules of the ligand were identified, inside the gaps formed between the dimers (Fig. 6).

As has been described, what can be determined experimentally by X-ray diffraction analysis is an electron density map, and the 3D coordinates obtained best fitting the map are still a model, (Fig. 2). A number of water molecules are contained in crystals, in general. It is extremely difficult to identify molecules as small as water in an electron density map. Of the atoms constituting a water molecule, the two hydrogens diffract X-rays very poorly, and thus, in short, they are invisible by X-ray analysis. Consequently, in order to identity a water molecule, a point-like density of the oxygen atom isolated from any other density needs to be identified in the electron density map. The extra densities present in the electron density map of the Lrp-like protein was first interpreted as 196 water molecules, or more precisely 196 oxygen atoms. If a natural ligand originated in *E. coli.* cells are present in the crystal it should be larger than a single water molecule, corresponding to one or more of such electron densities.

In order to identify the electron density of the ligand, water molecules modeled to relatively low electron densities were removed. Of these 66 were found inside the pocket at the center of the protein octamer. This was necessary to remove distortion of the map caused by possible misidentification of these atoms. Starting with this model, the process of 3D structure refinement was repeated several times, and the difference between the model and the electron density map (hereafter referred to as the difference Fourier map) was computed, expecting that a density should emerge, corresponding to the ligand. At each cycle, high densities newly found in the difference Fourier map (at counter levels of three or higher) were examined thoroughly. To these densities, which were isolated or which were close enough to hydrophilic groups in the protein so that ionic/hydrogen bonding was possible, new water molecules were modeled. Finally, an electron density larger than that expected for a water molecule, was identified in each asymmetric unit. Since an octamer is composed of two such asymmetric units two such densities were found in the octamer (Fig. 6). These two densities were interpreted as those of two molecules of the ligand, each positioned inside the gap formed between a pair of dimers. Each electron density has a size equivalent with four or more water molecules, or equivalent with a single molecule of a medium sized amino acid such as valine or isoleucine.

### [Example 6] Interaction between the Lrp-like protein and natural ligands in solution

It has been confirmed that various amino acids and, in addition, some other biomolecules (e.g., metabolic intermediates) can act as natural ligands on assemblies of the Lrp-like protein.

### (1) Depolarization of the crystals by amino acids

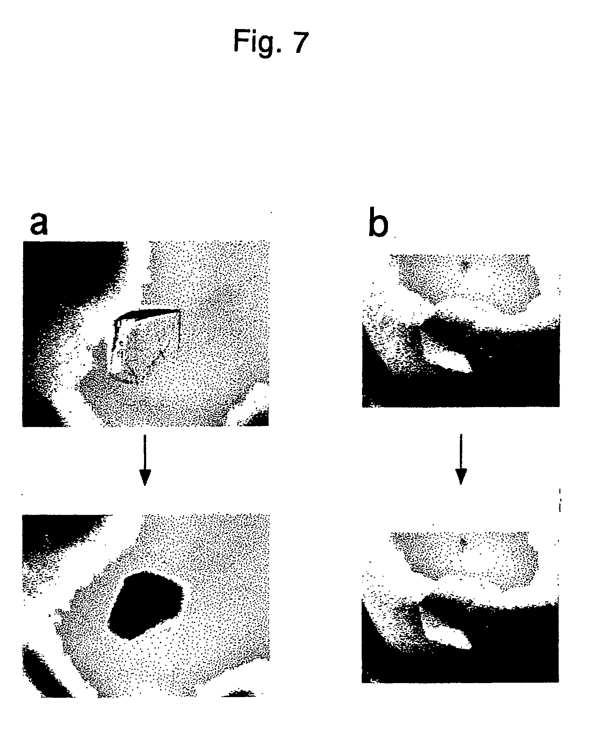

It has been known that the regularity inside a crystal might be affected, if the structure of the protein changes upon interaction with a ligand which is incorporated from outside the crystal, thereby depolarizing the crystal. Thus, crystals of the Lrp-like protein were soaked into solutions, each containing one of twenty amino acids at the same concentration 10 mM. With isoleucine, valine, methionine, and leucine, in several hours the crystals depolarized (Fig. 7). With the other amino acids, no change was observed. It is important to note that the changes caused by the four amino acids to the 3D structure of the protein are not necessarily the same, and that the other amino acids, although they showed no effect, might not have diffused into the crystal, and thus these 1 might function as ligands in solution.

### (2) Analysis using gel filtration

Possible changes in assemblies of the Lrp-like protein induced by interaction with amino acids and metabolic intermediates were analyzed in solution using gel filtration.

### 1) Experimental procedure

Gel filtration was carried out with the flow rate 1 ml/min, using a matrix (Protein Pak 125, 7.8 x 300 mm, Waters) and 50 mM Tris-HCl buffer (pH 7.0) containing 300 mM sodium chloride. Flow of the proteins was monitored by measuring the UV absorbance at 220 nm (LC Module I plus system, Waters). The Lrp-like protein purified by method 2, i.e., the rigorous method, were dissolved in 50 mM Tris-HCl buffer (pH 7.0) containing 200 mM sodium chloride, and kept at -80°C until it was diluted by 10-fold with 50 mM Tris-HCl buffer (pH 7.0) containing 300 mM sodium chloride, and mixed with each potential ligand for at least 30 minutes at room temperature (about 25°C). In the mixture, the monomer concentration of the Lrp-like protein was approximately 100 µM. While, the concentration of each-amino acid was 1 mM and that of 5 metabolic intermediates, i.e., malic acid, 2-oxoglutaric acid, oxaloacetic acid, pyruvic acid, and 3-phosphoglyceric acid, was 10 mM.

### 2) Results

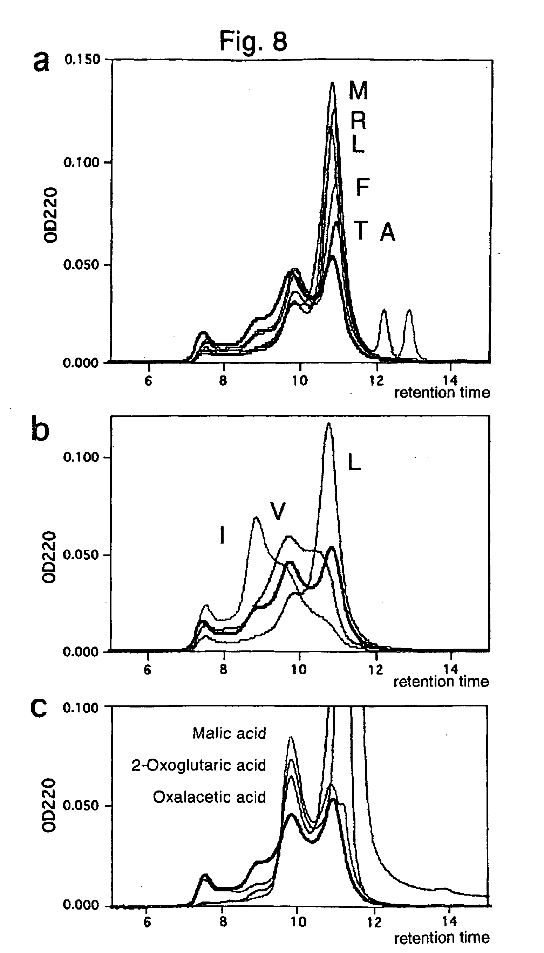

When isoleucine was mixed with the protein, the profile of gel filtration changed, increasing the second peak. While, hen valine was mixed, the third peak increased (Fig. 8a). Six other amino acids, i.e., methionine, arginine, leucine, phenylalanine, alanine, and threonine, showed effects of increasing the fourth peak (Fig. 8b). None of the other twelve amino acids showed any significant effect. When one of the three metabolic intermediates, each containing four or more carbon atoms (i.e., malic acid, 2-oxoglutaric acid, and oxaloacetic acid) was mixed, the third peak increased (Fig. 8c). In contrast, no significant effect was observed with two other metabolic intermediates, each containing three carbon atoms (i.e., pyruvic acid and 3-phosphoglyceric acid).

Peaks associated with smaller retention time should correspond to assemblies of larger molecular weights. Thus it is concluded that isoleucine accelerates assembling of the protein, six other amino acids, i.e., methionine, arginine, leucine, phenylalanine, alanine, and threonine, accelerate disassembling of the protein, and valine and three metabolic intermediates (malic acid, 2-oxoglutaric acid, and oxaloacetic acid) have intermediate effects.

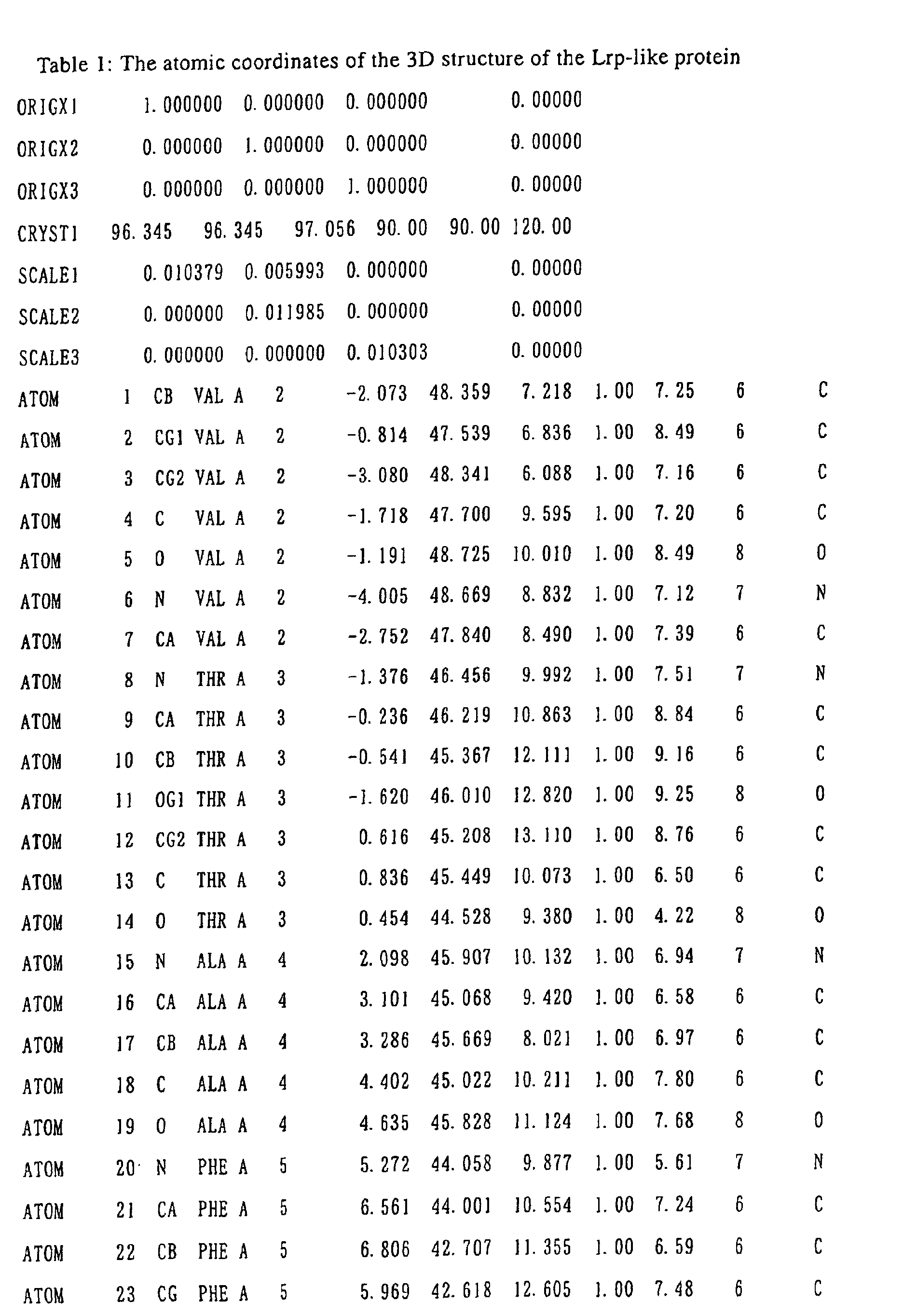

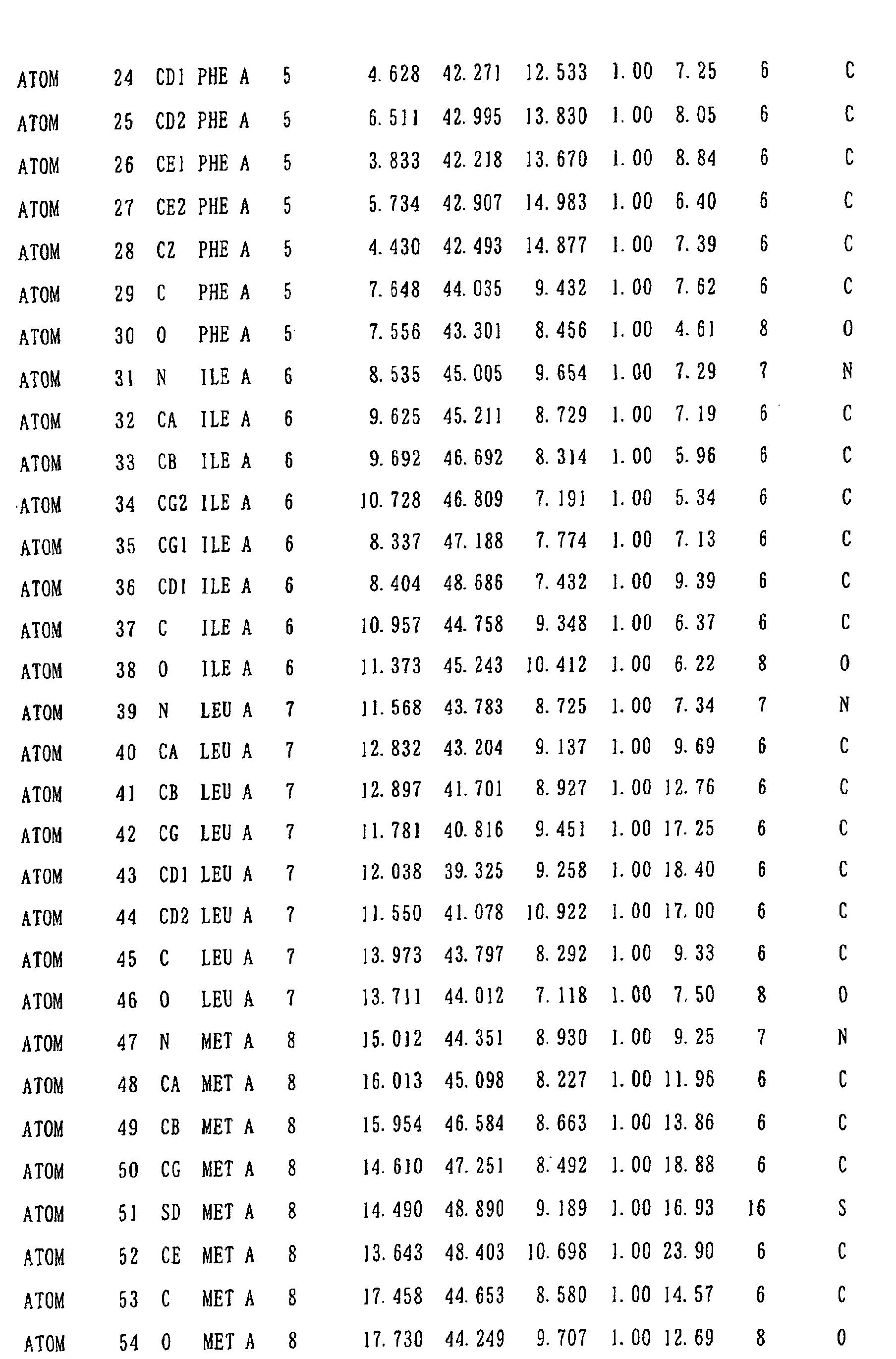

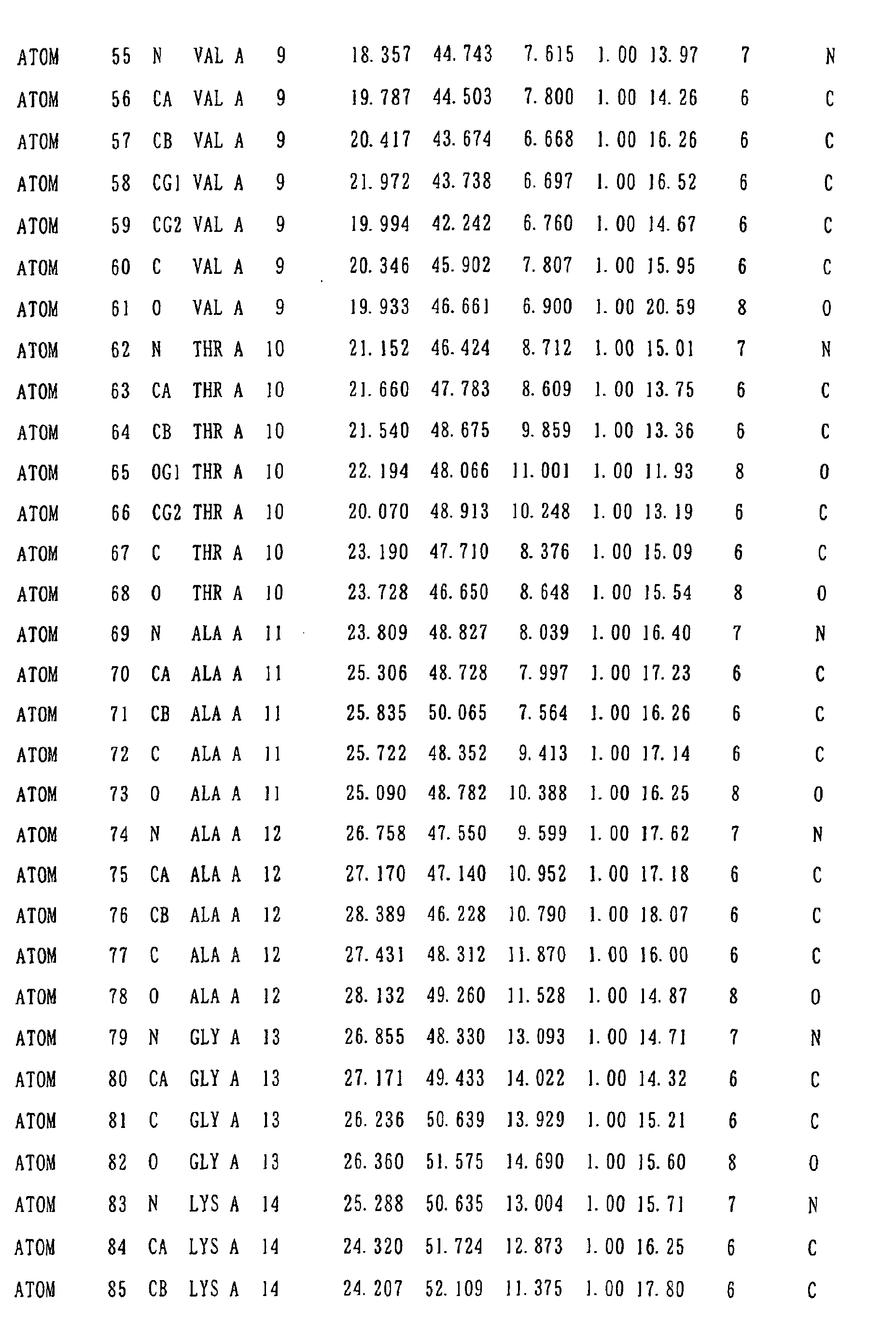

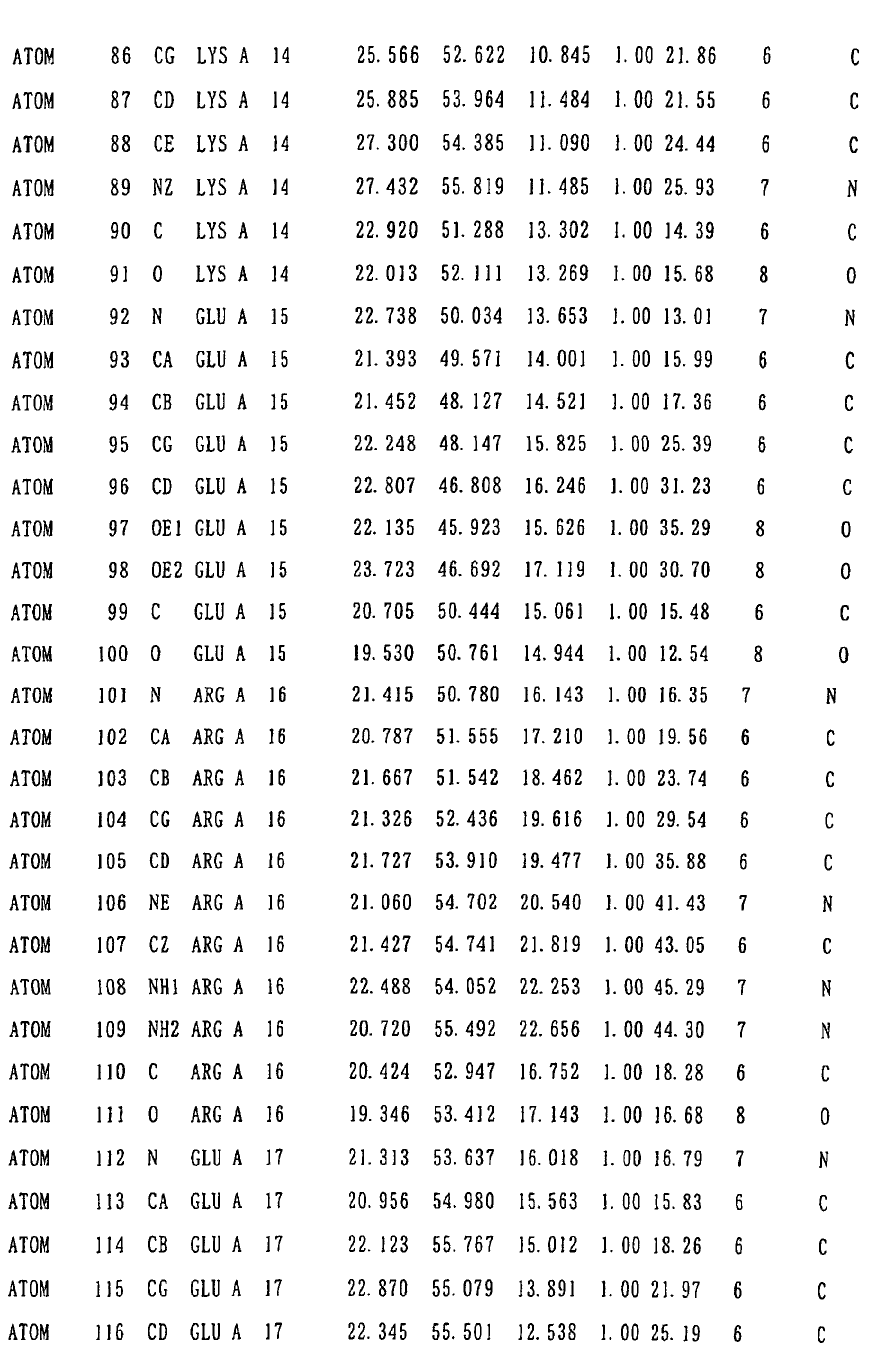

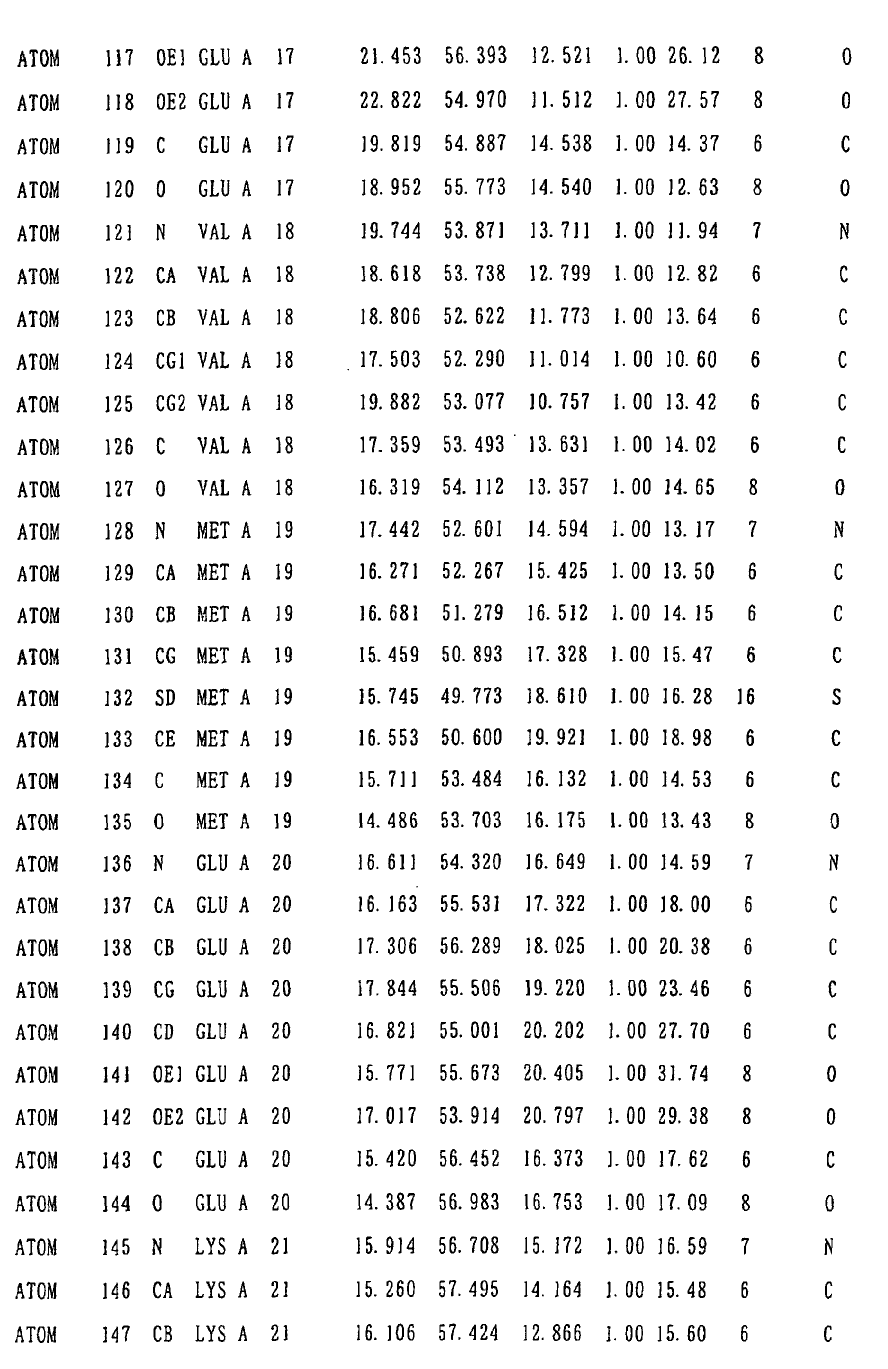

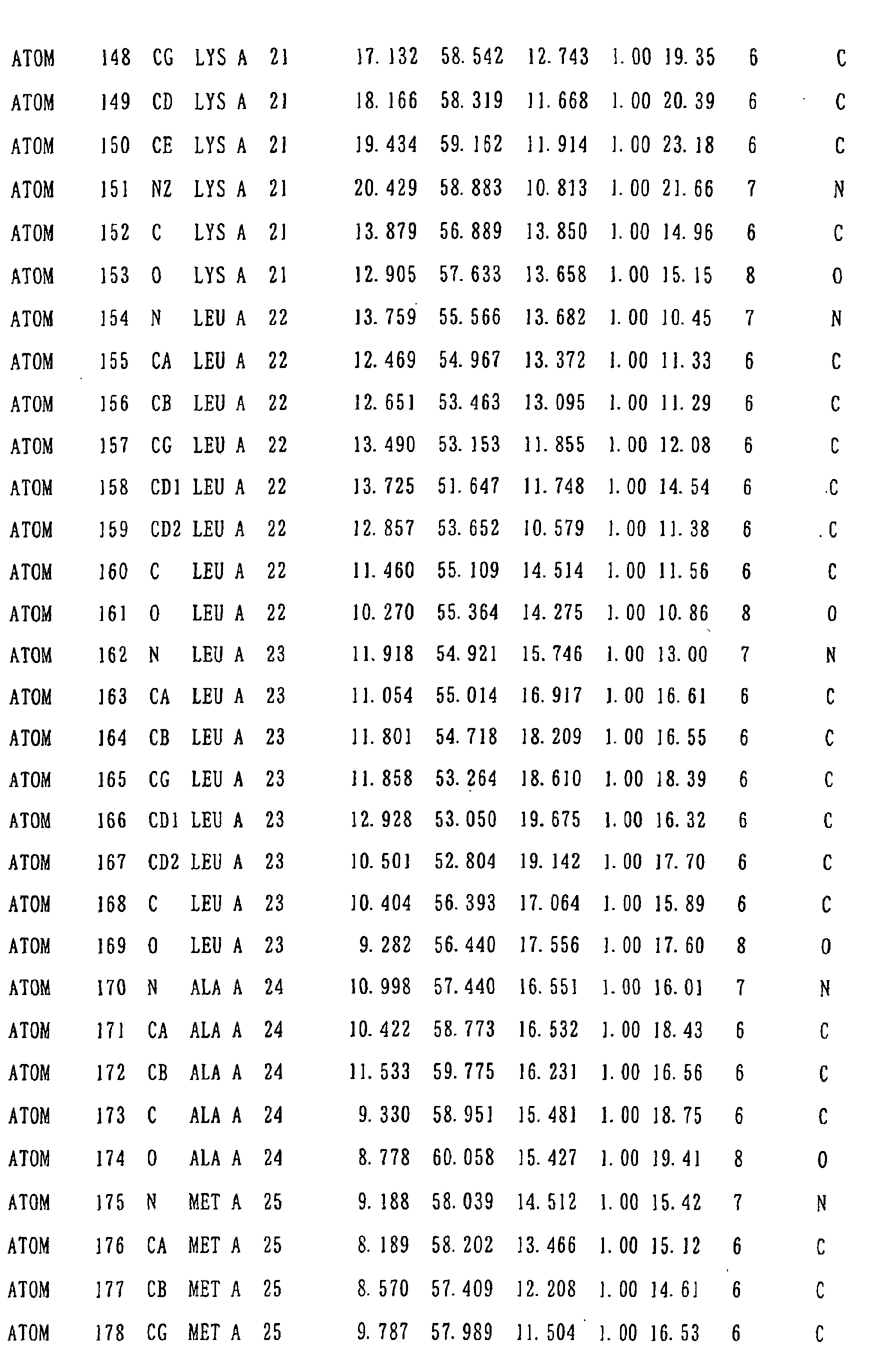

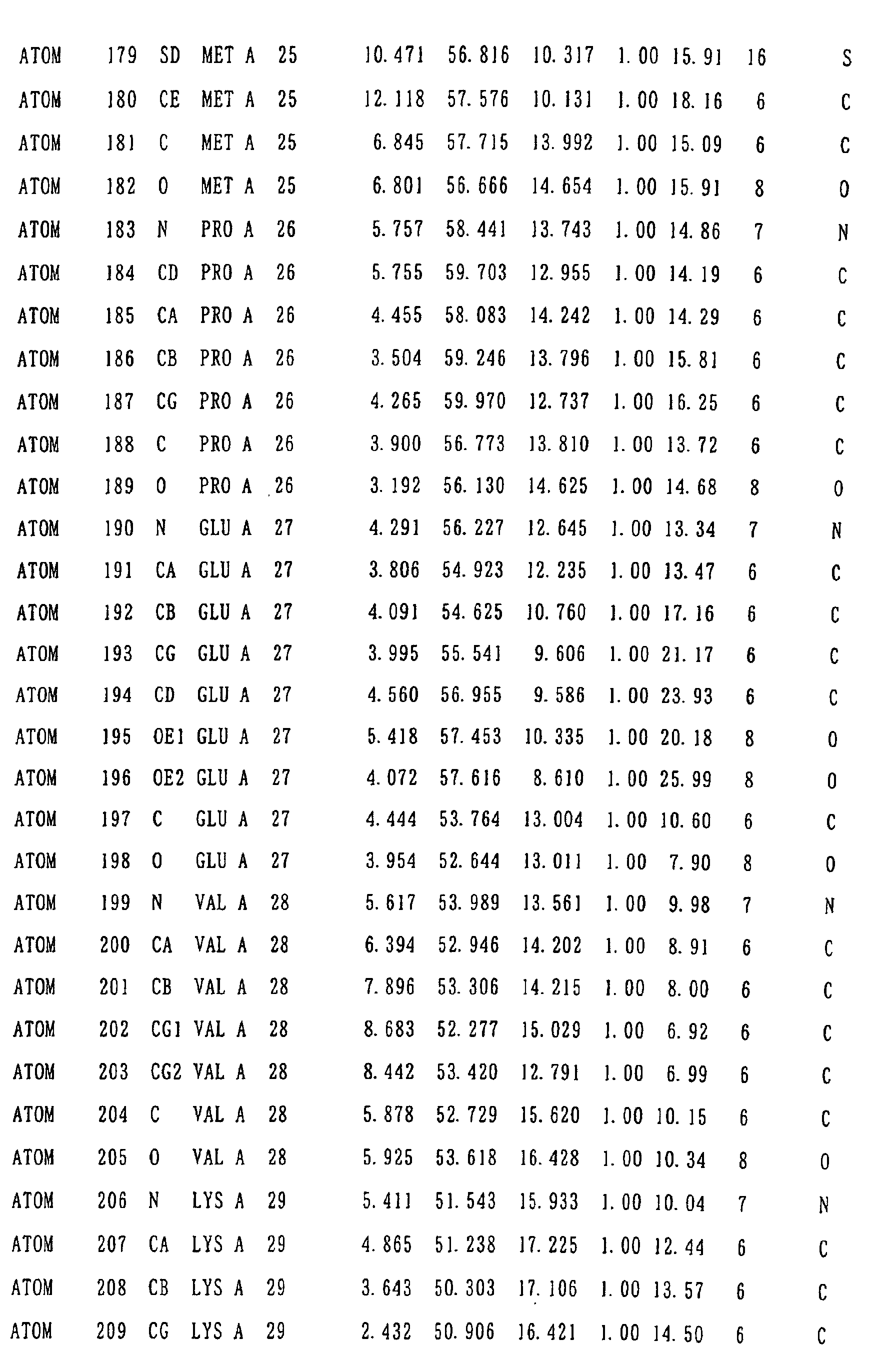

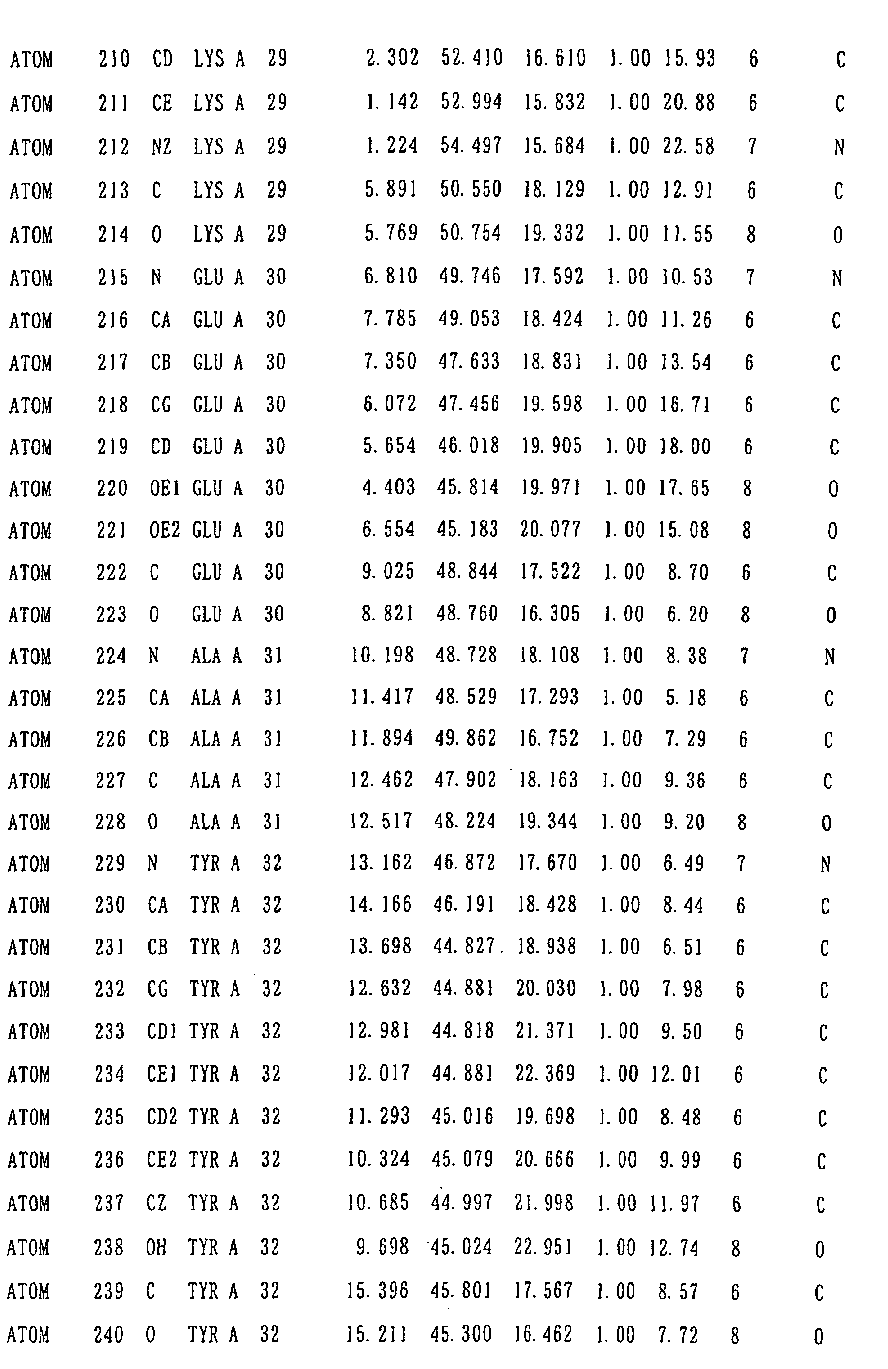

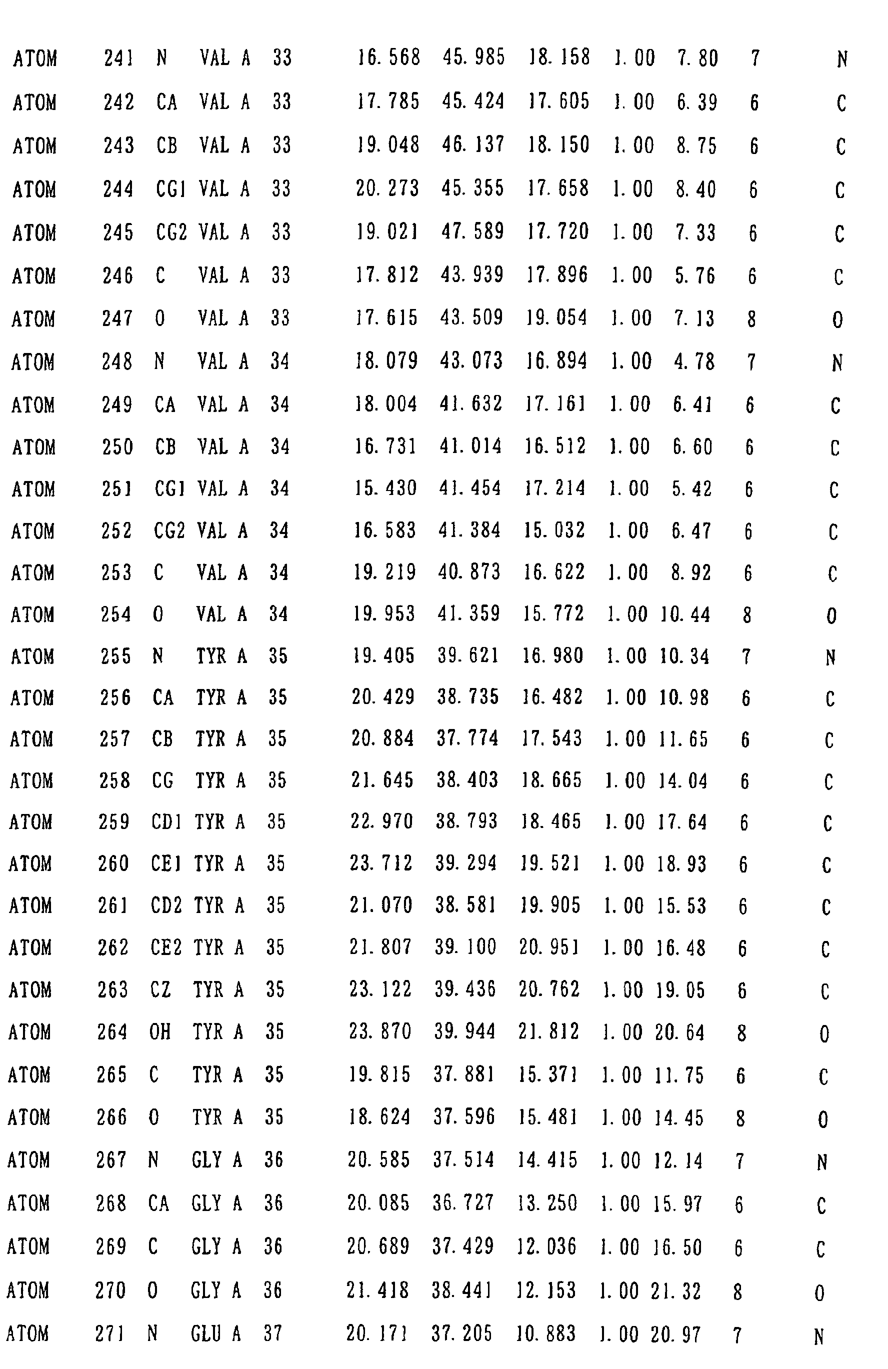

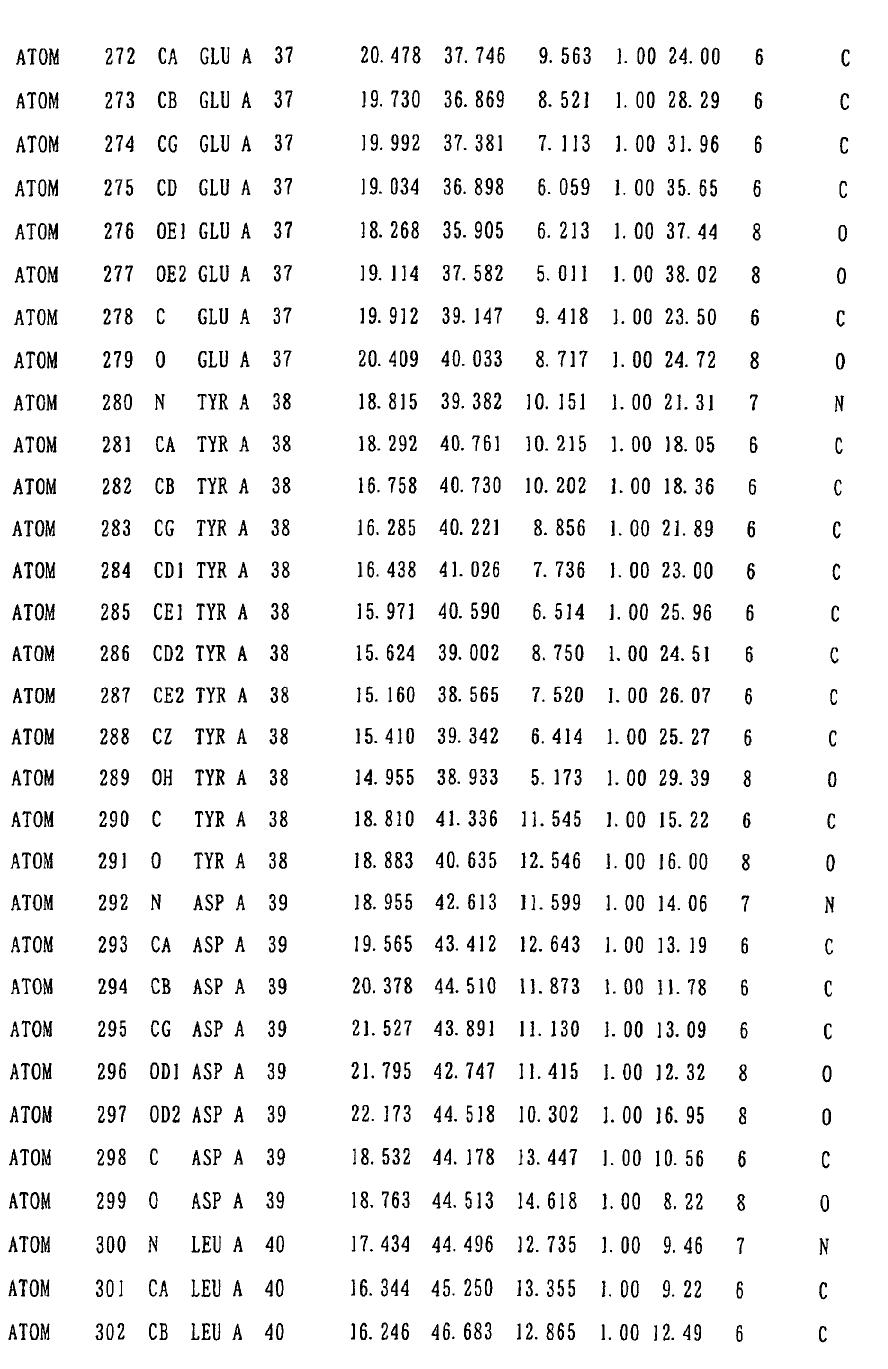

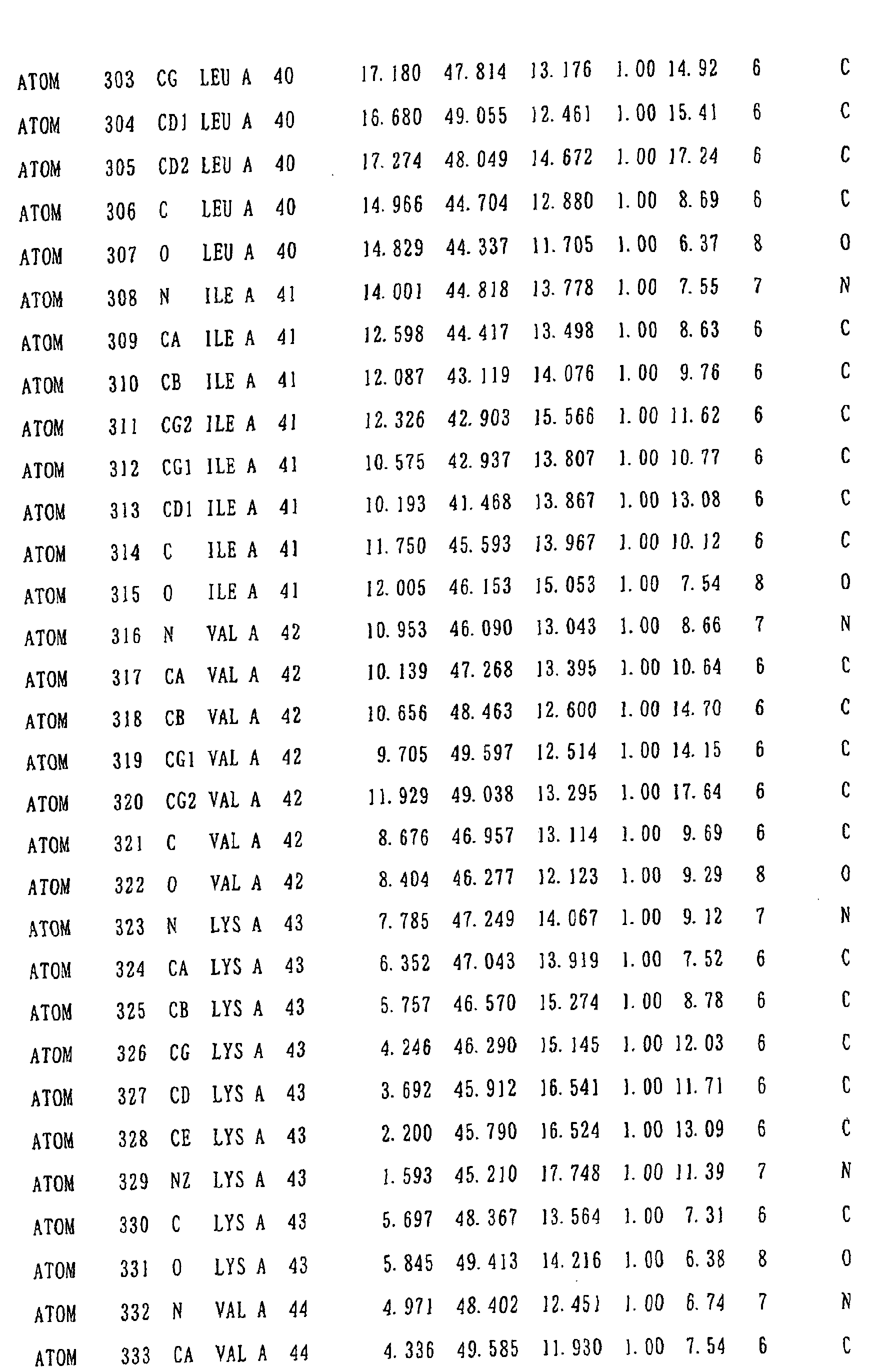

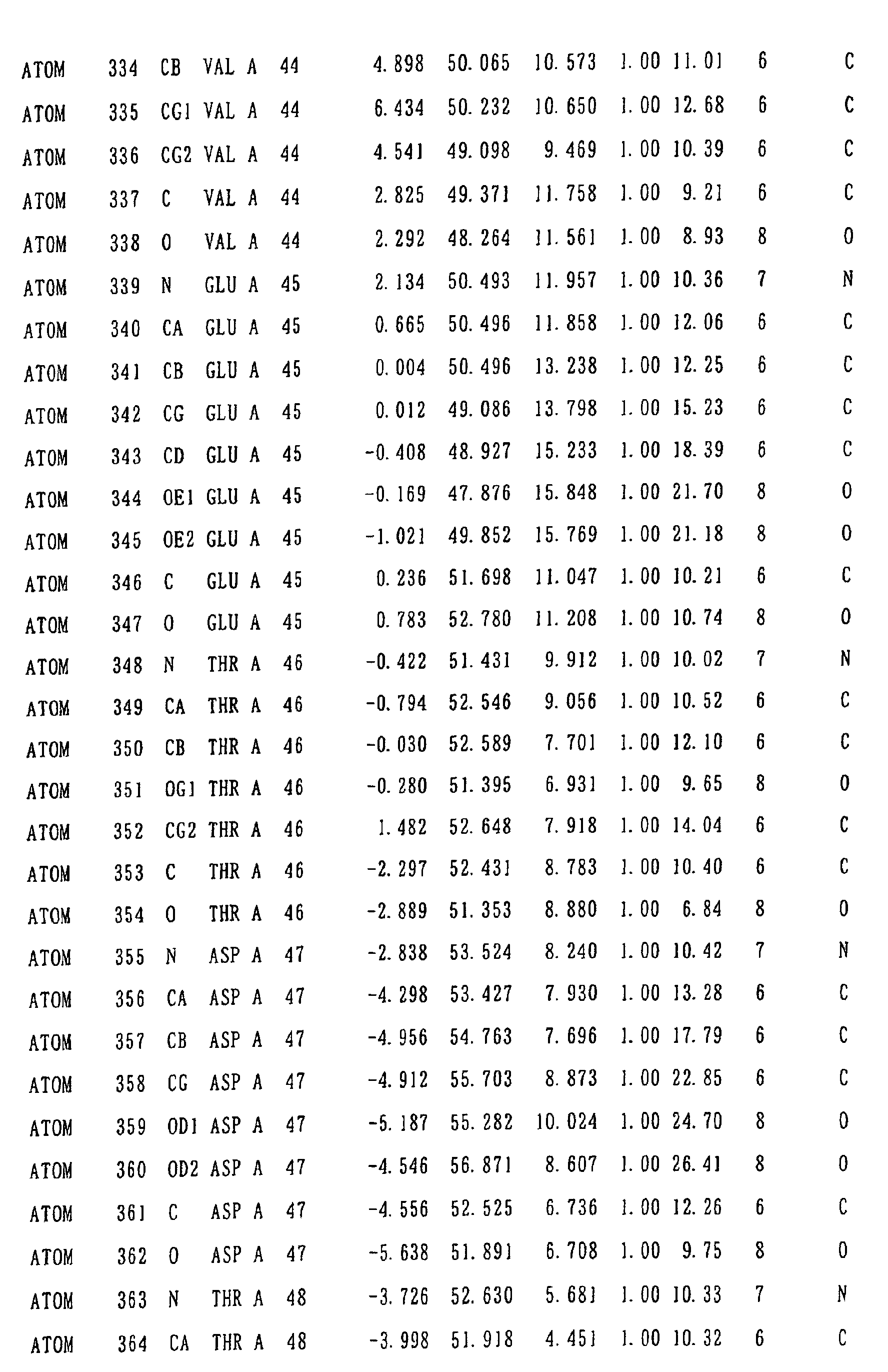

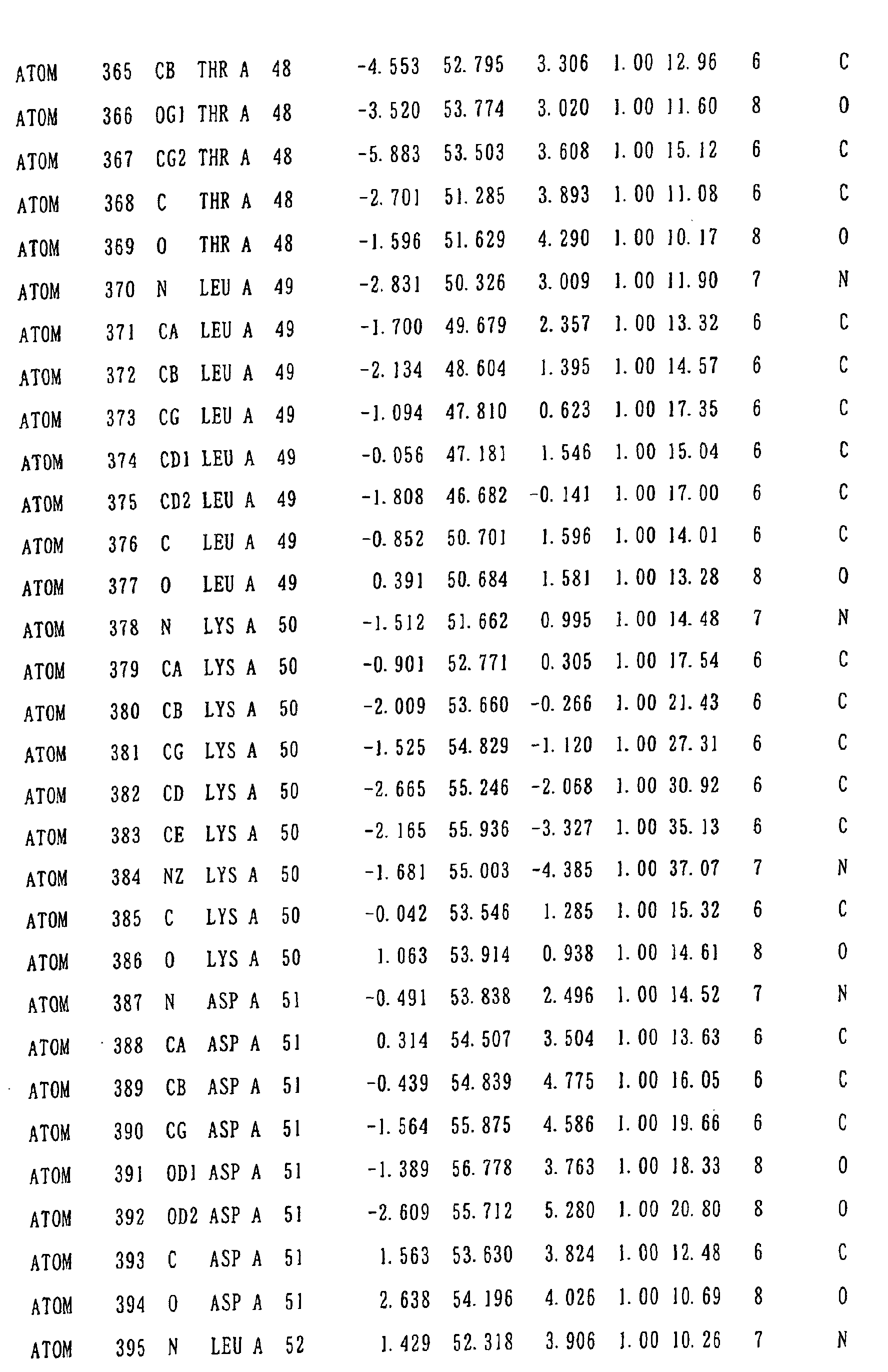

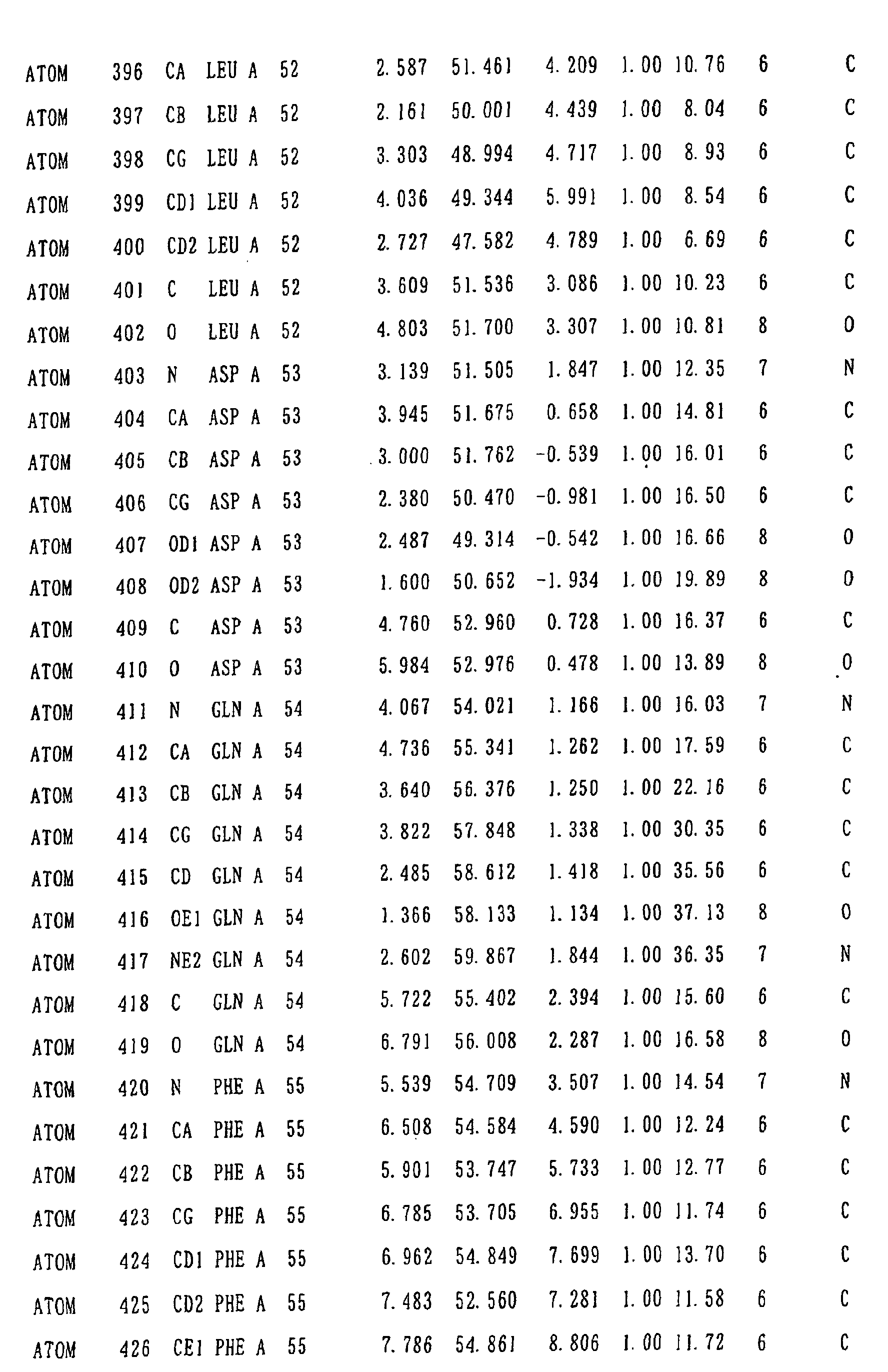

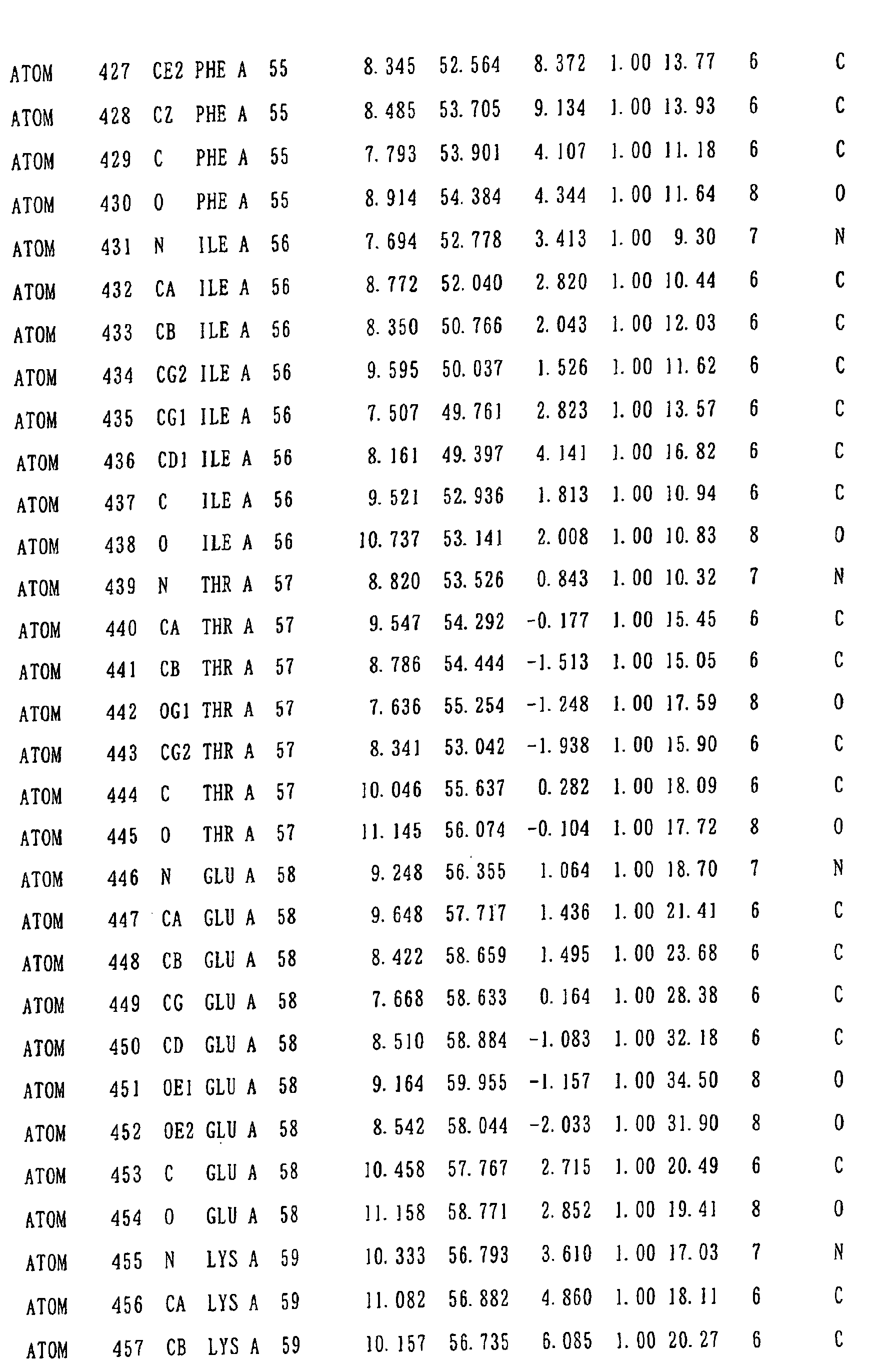

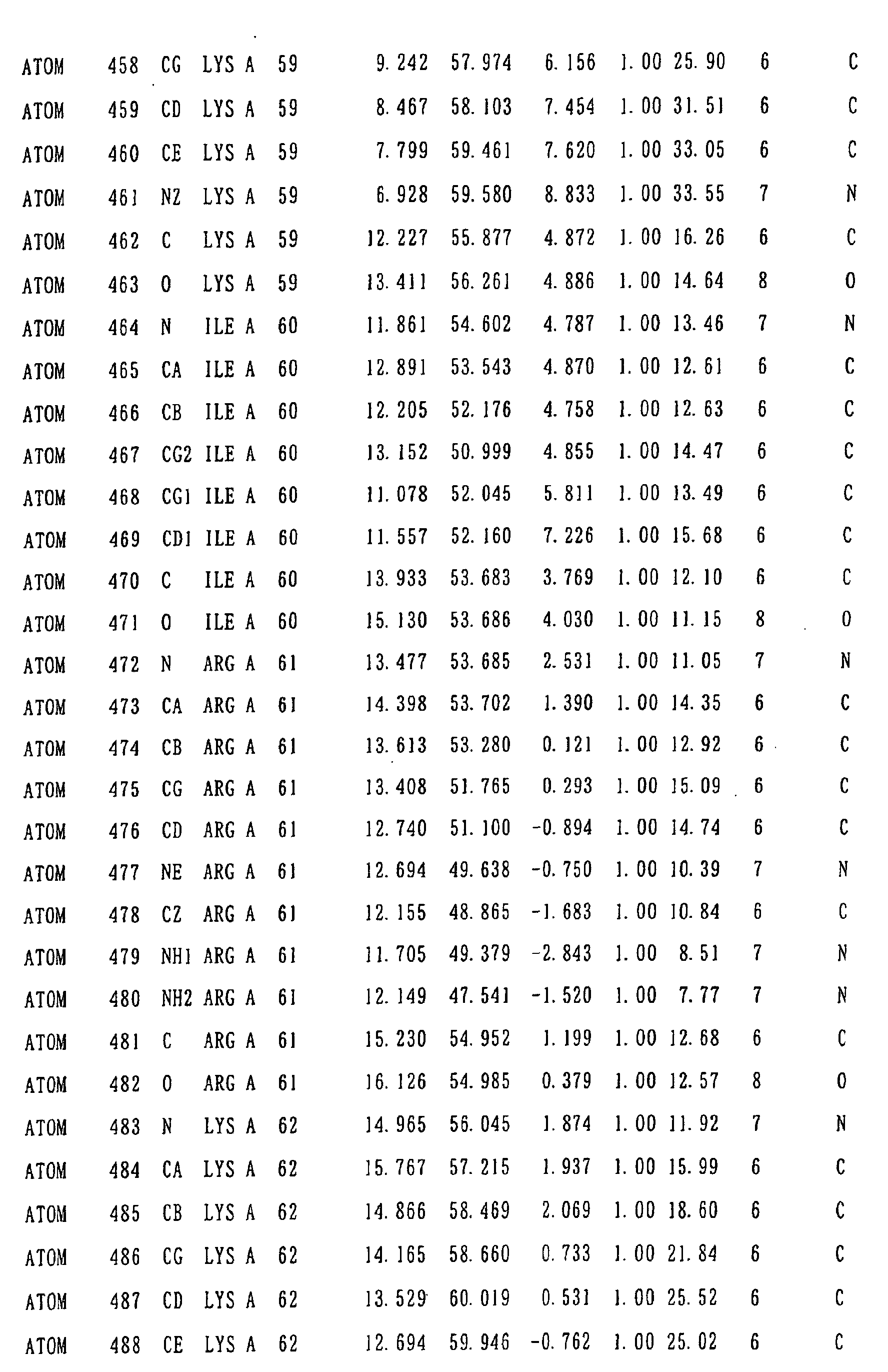

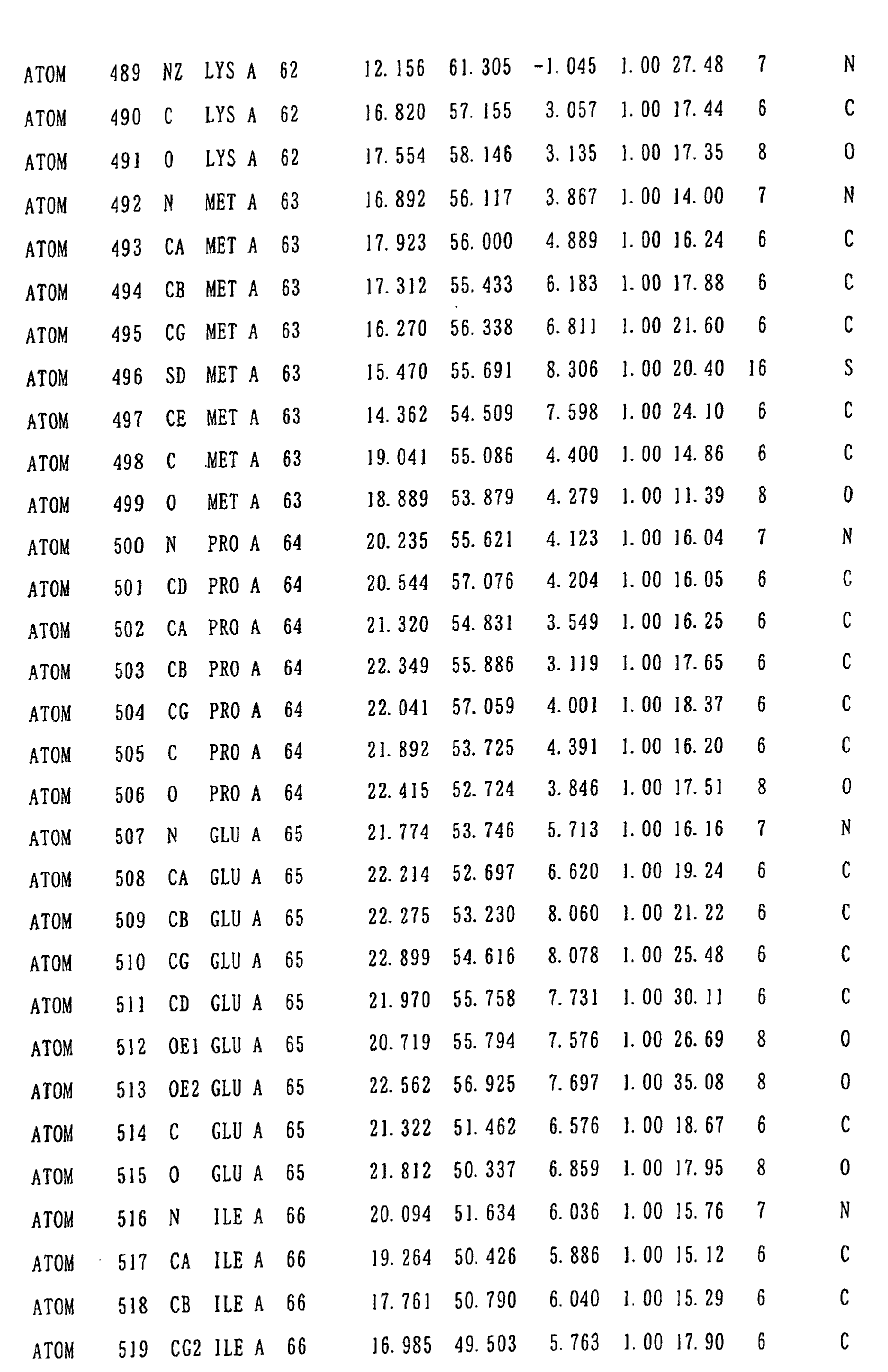

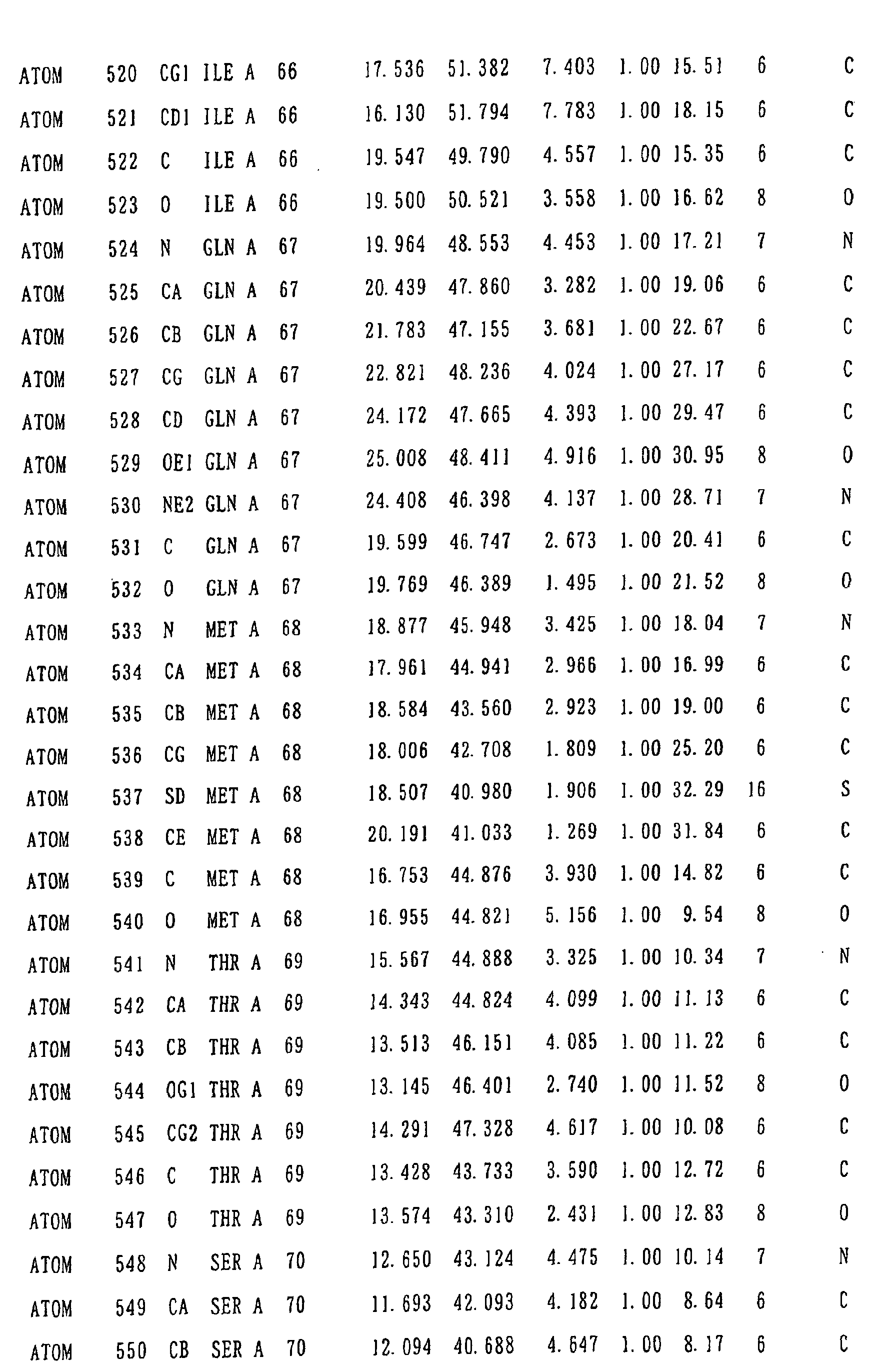

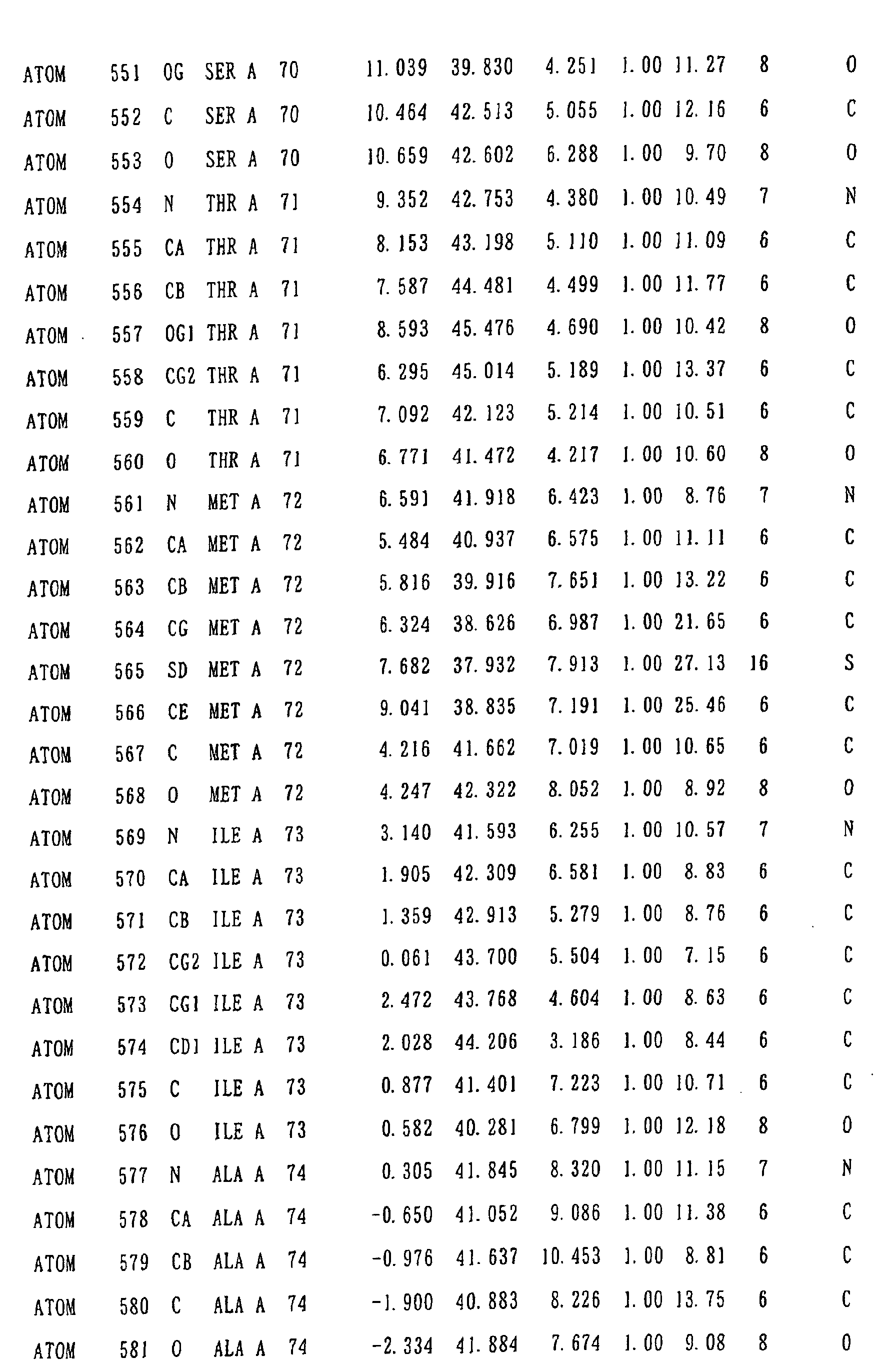

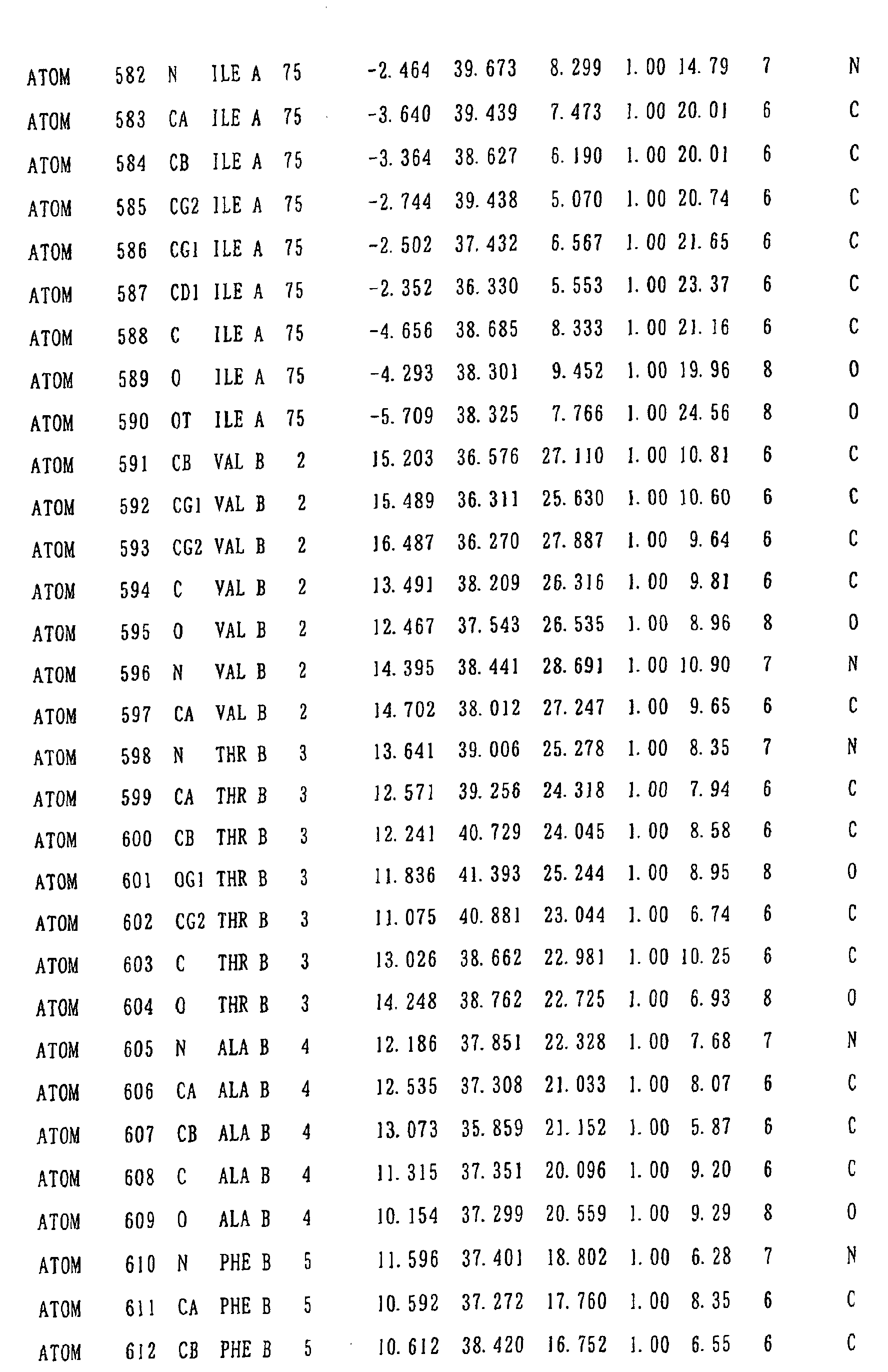

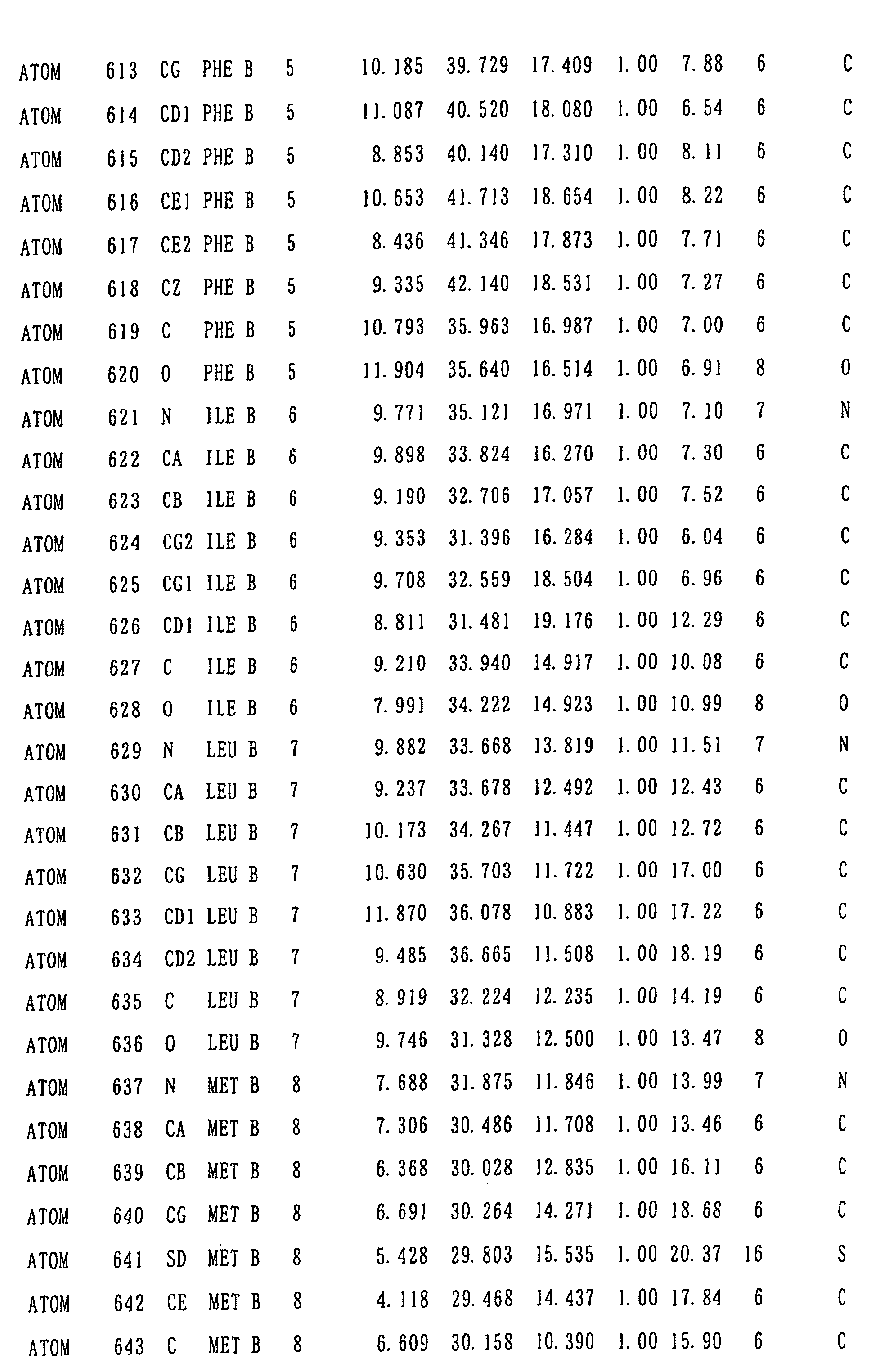

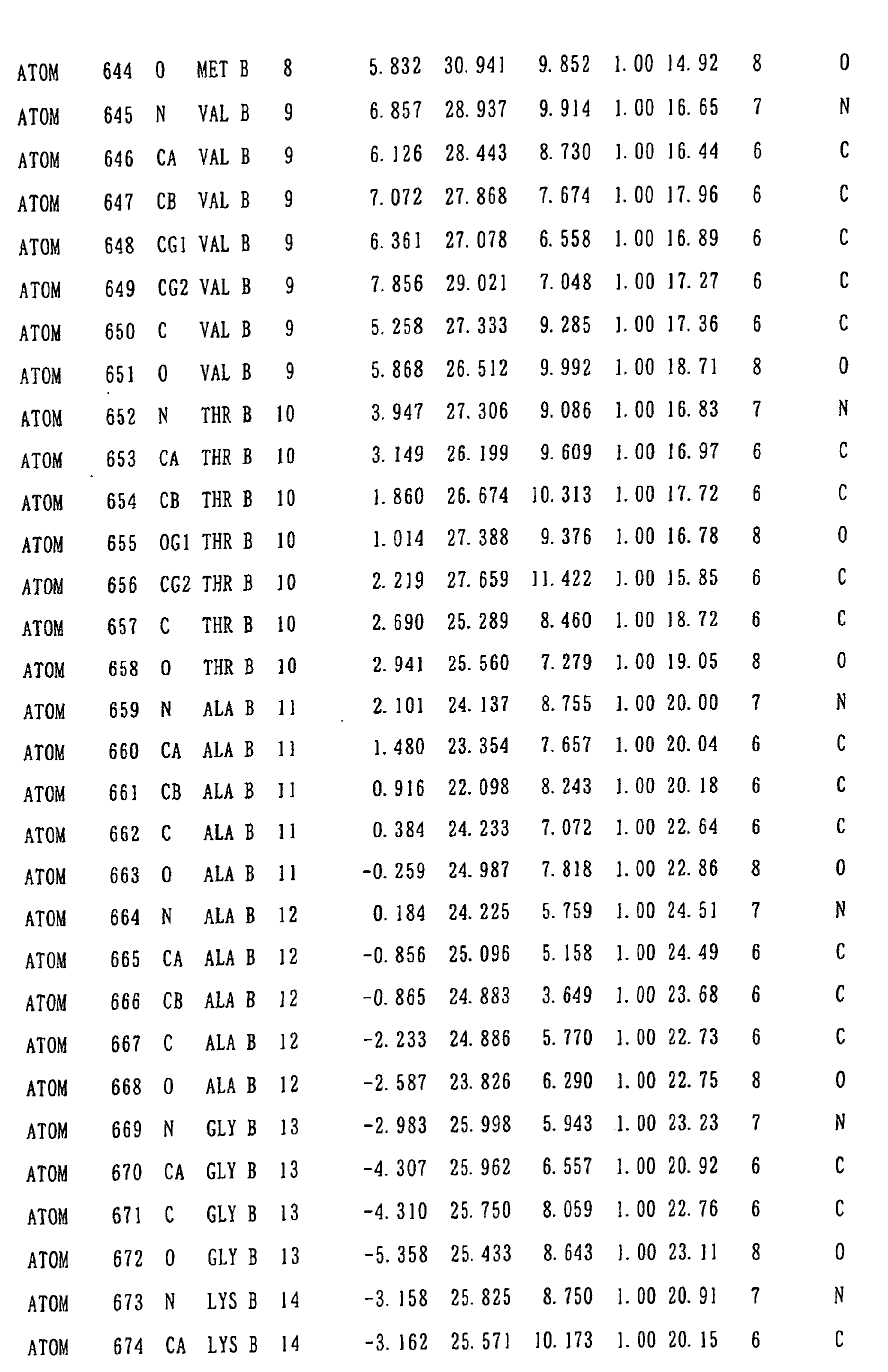

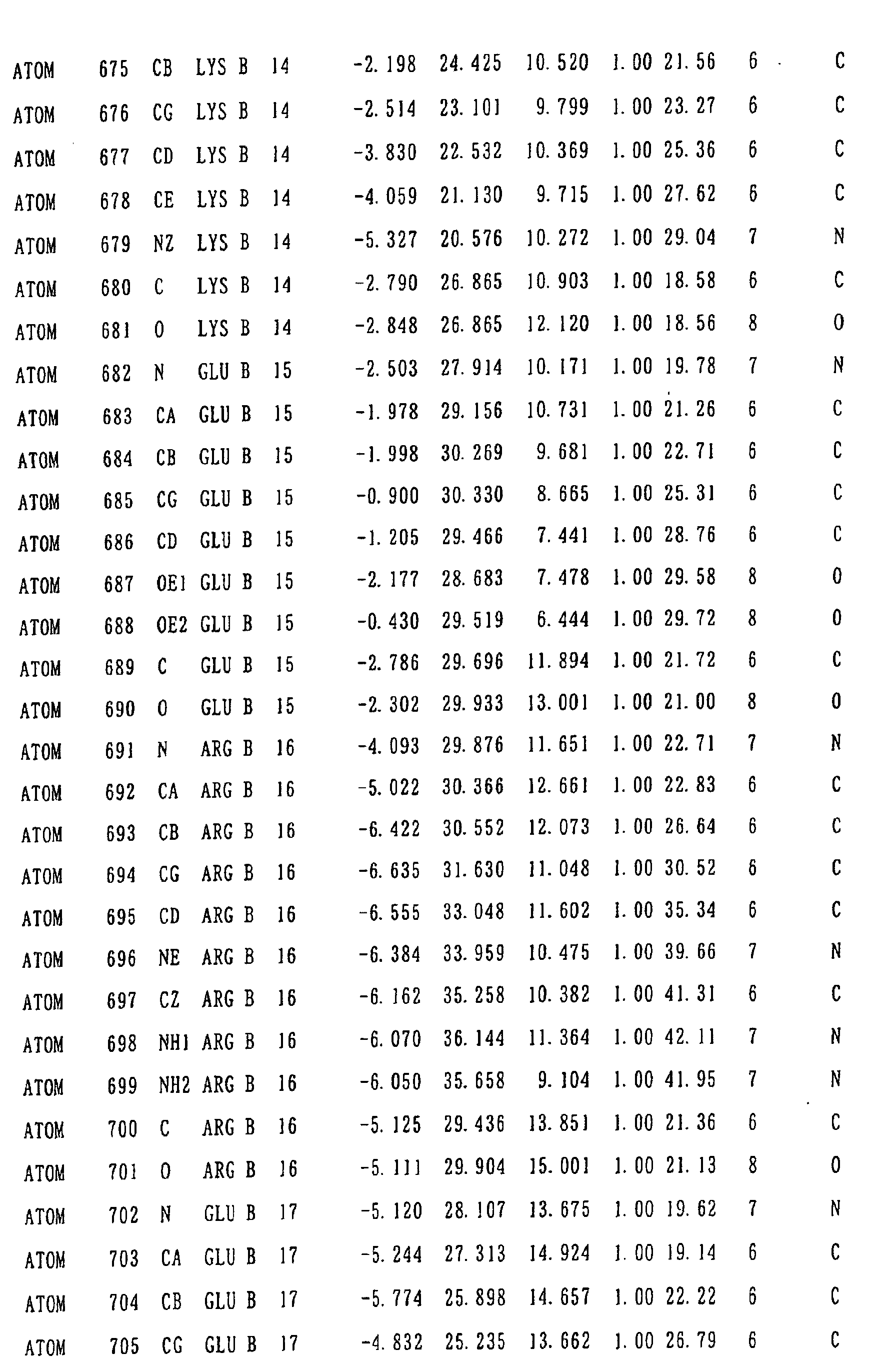

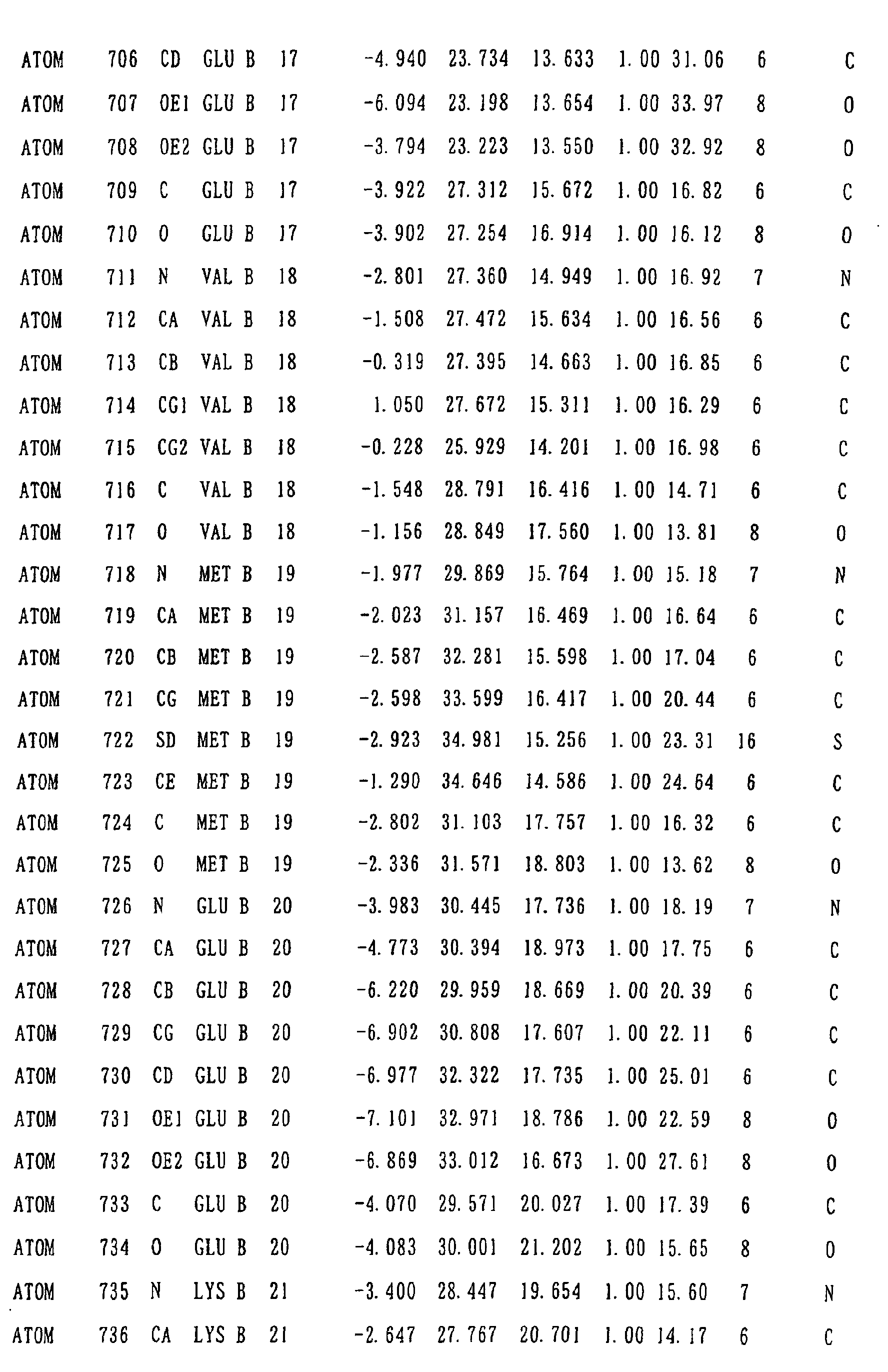

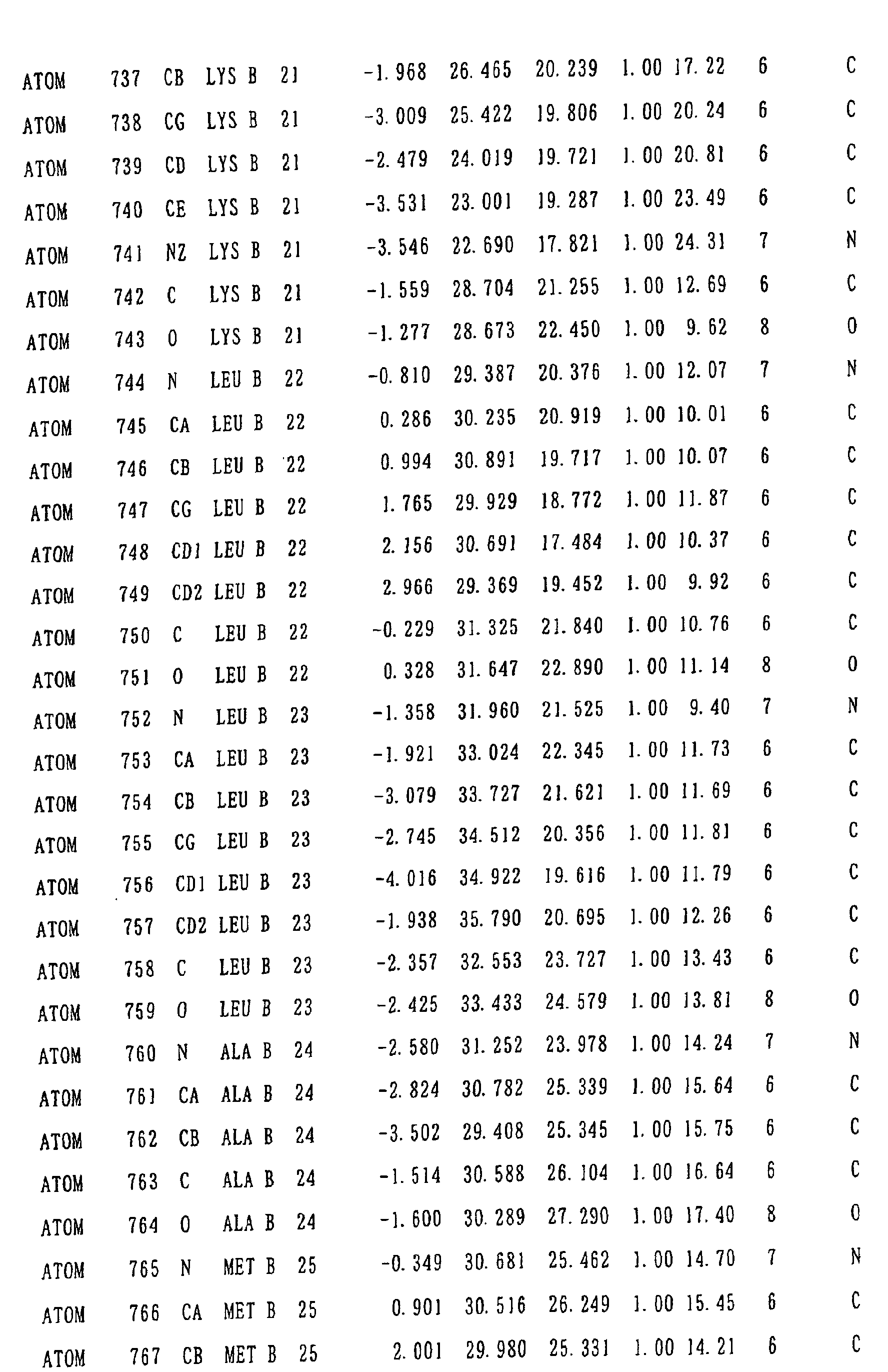

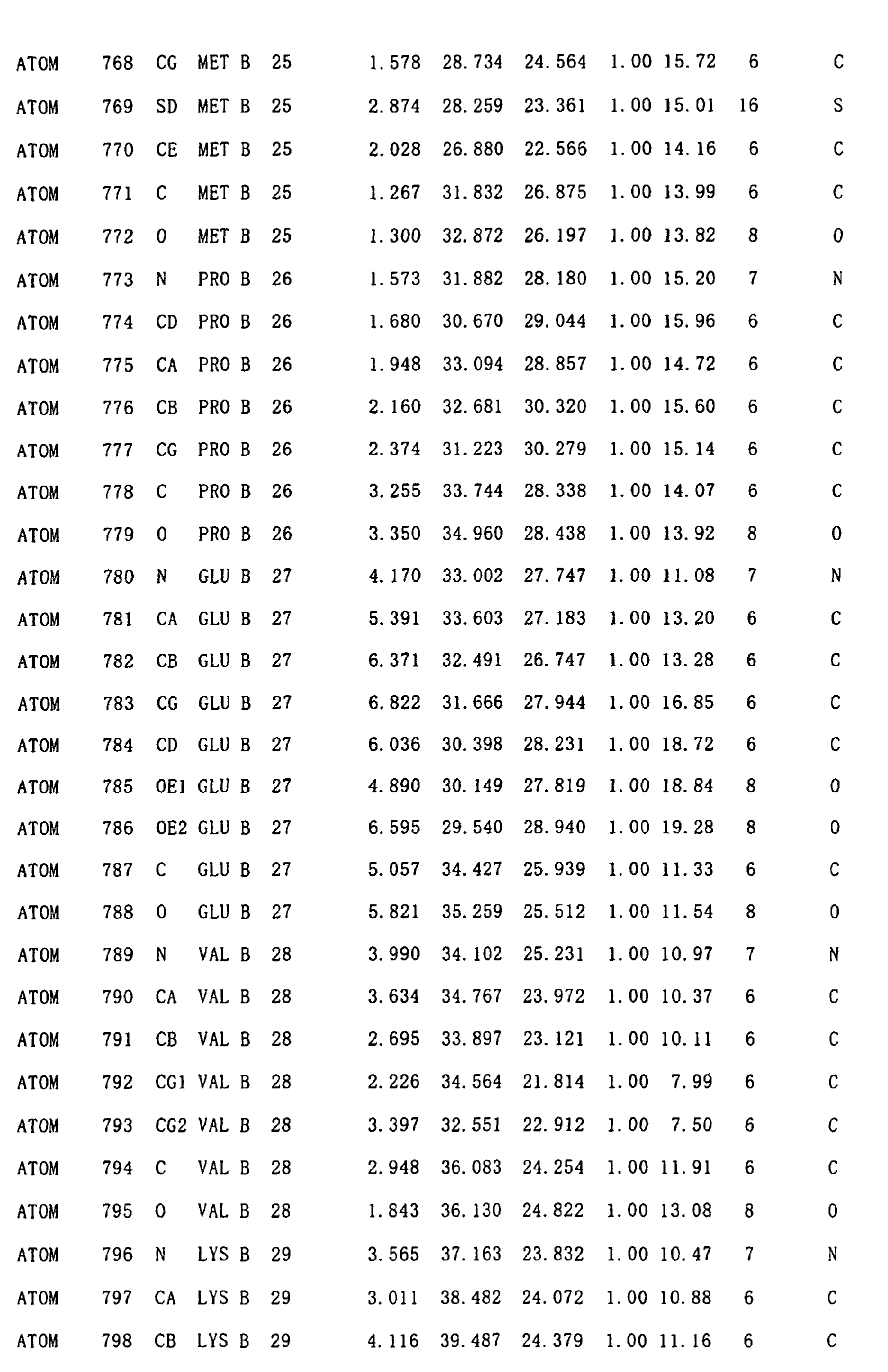

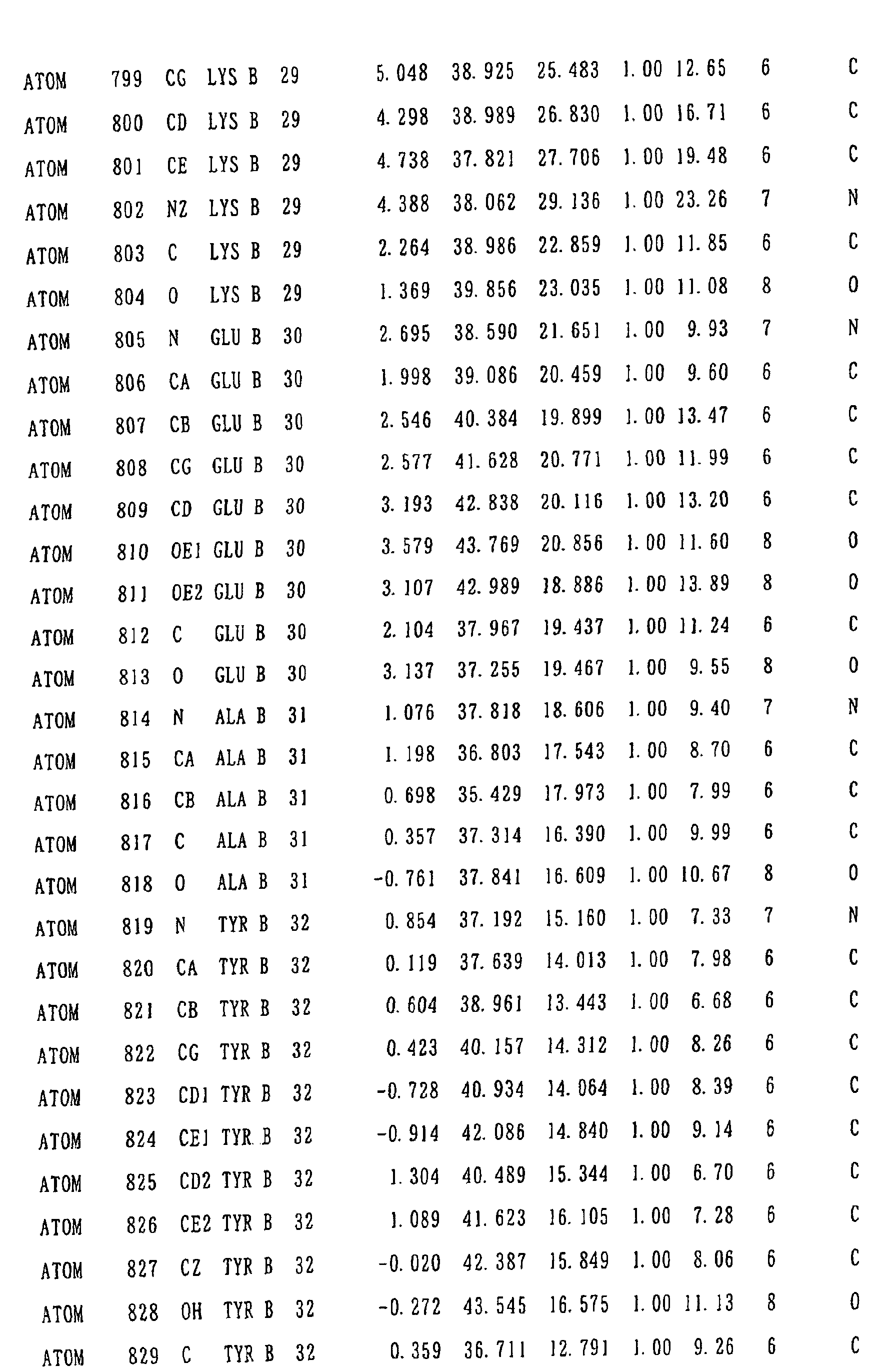

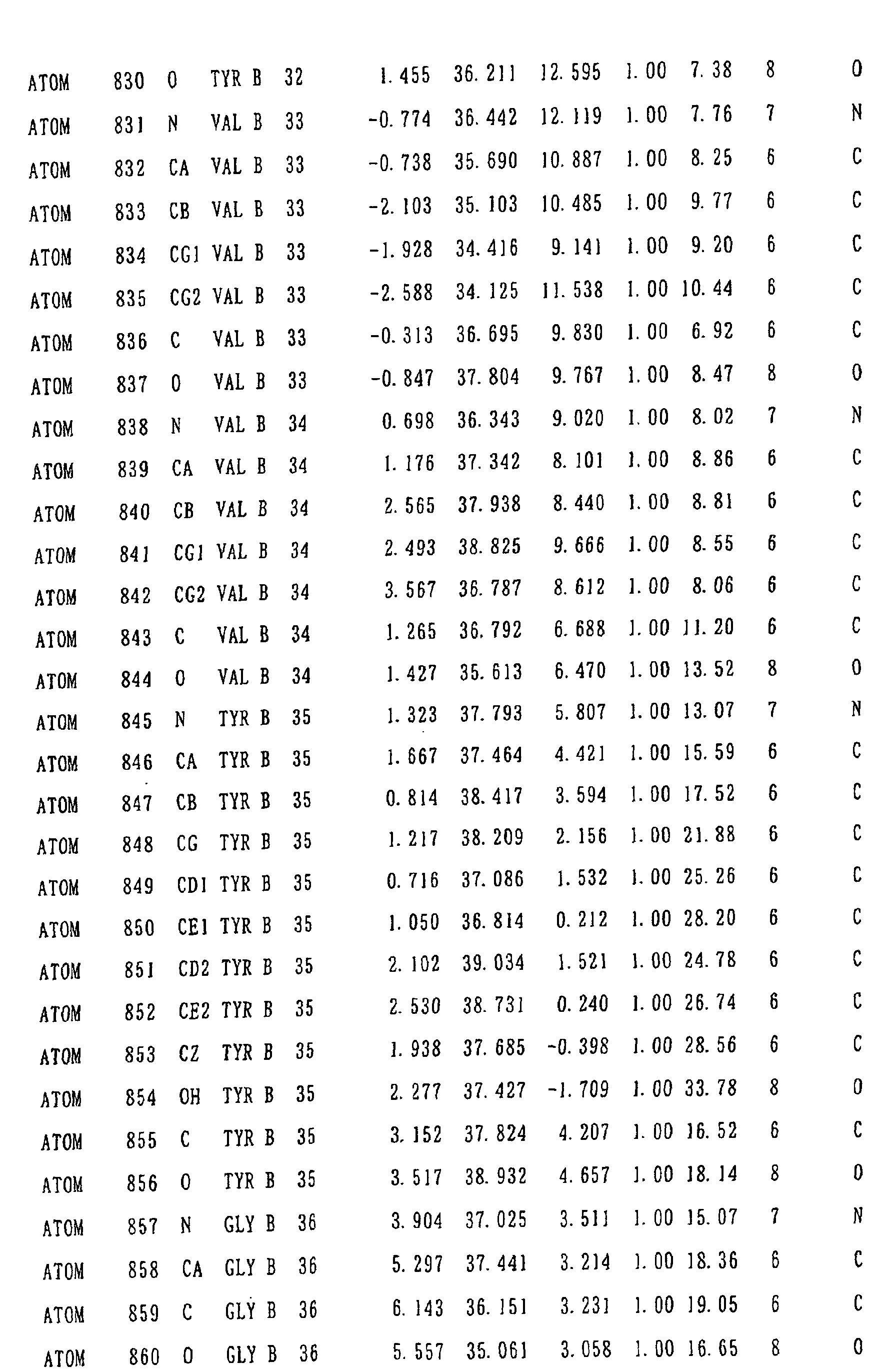

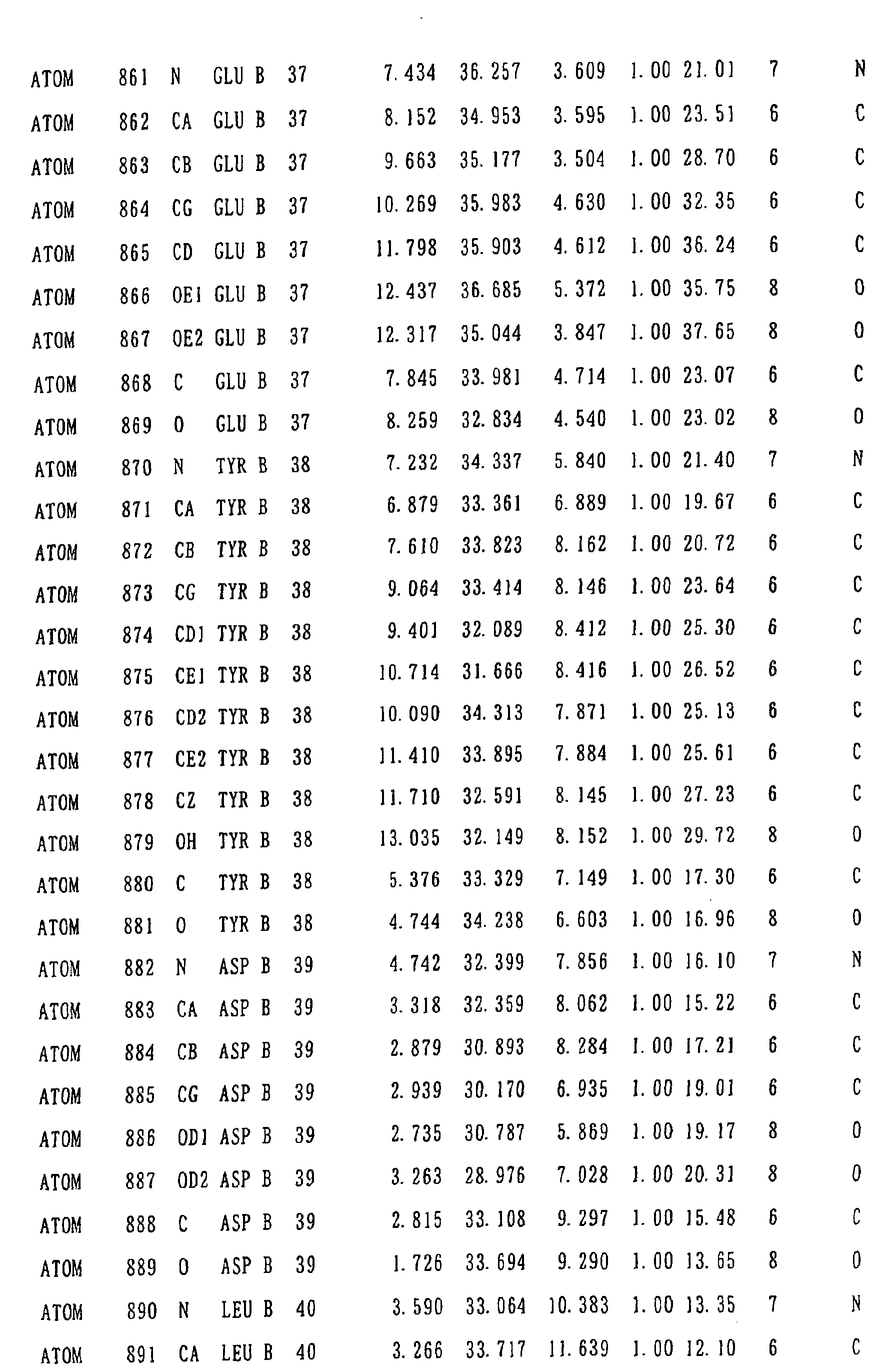

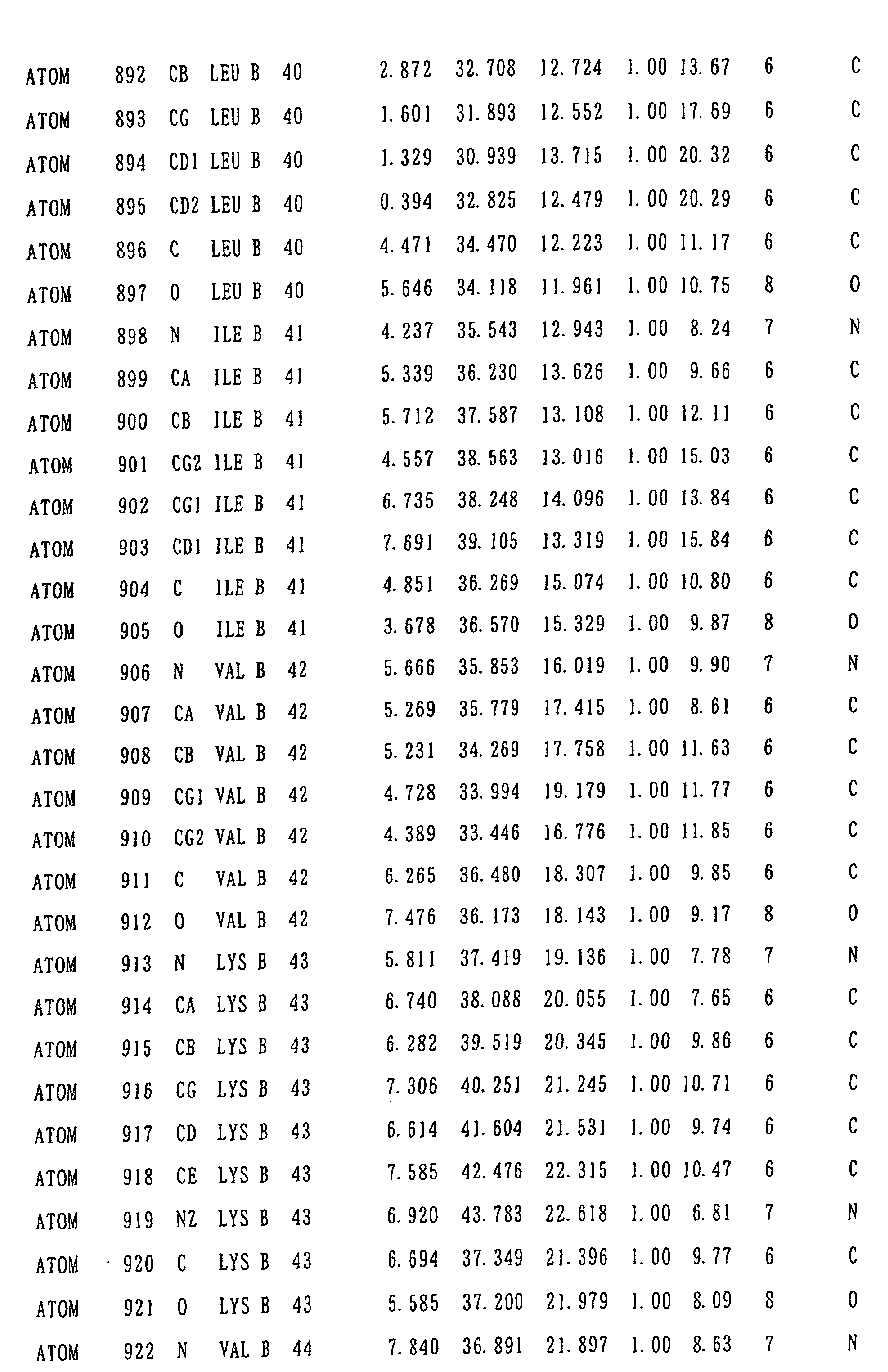

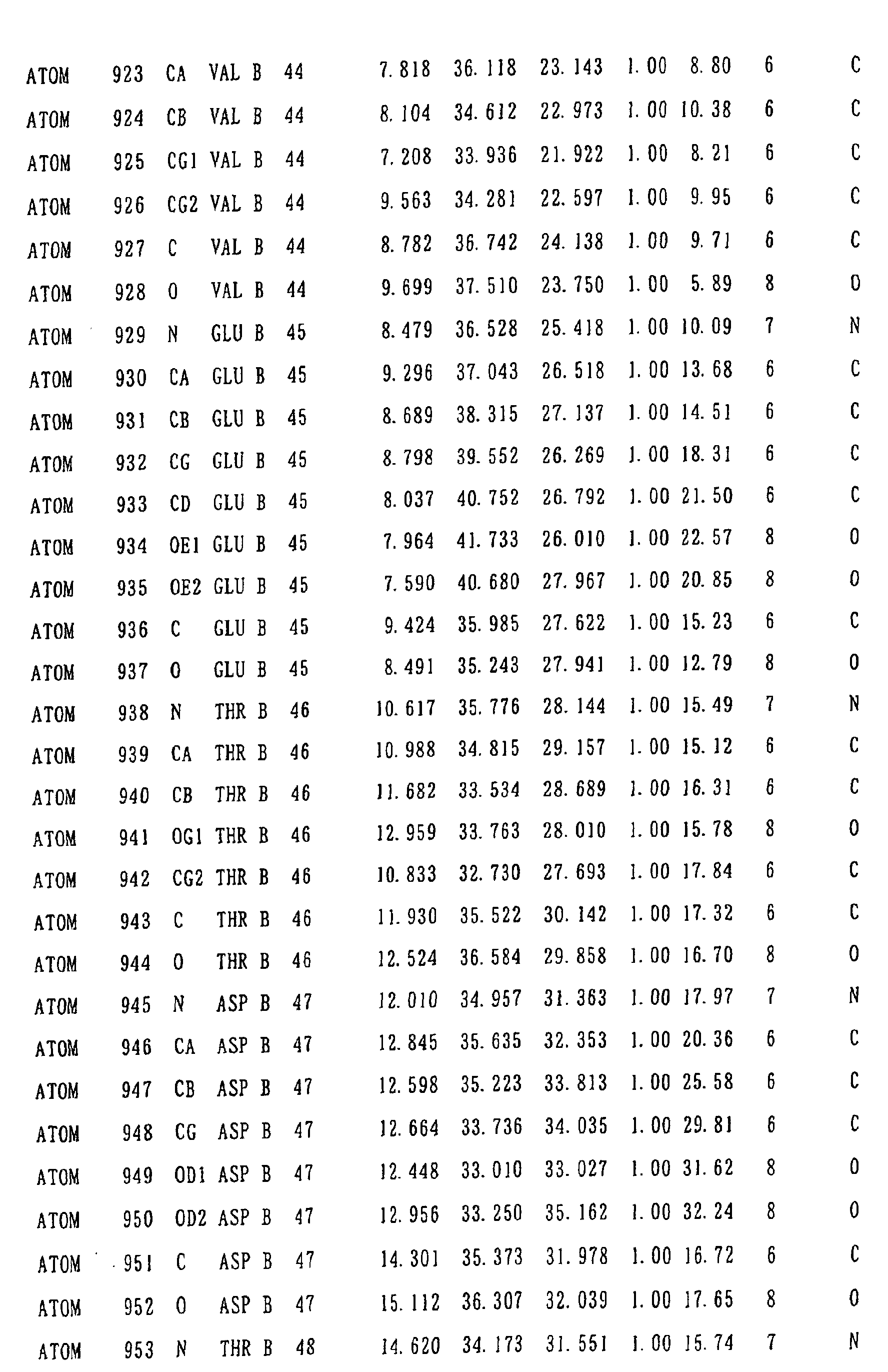

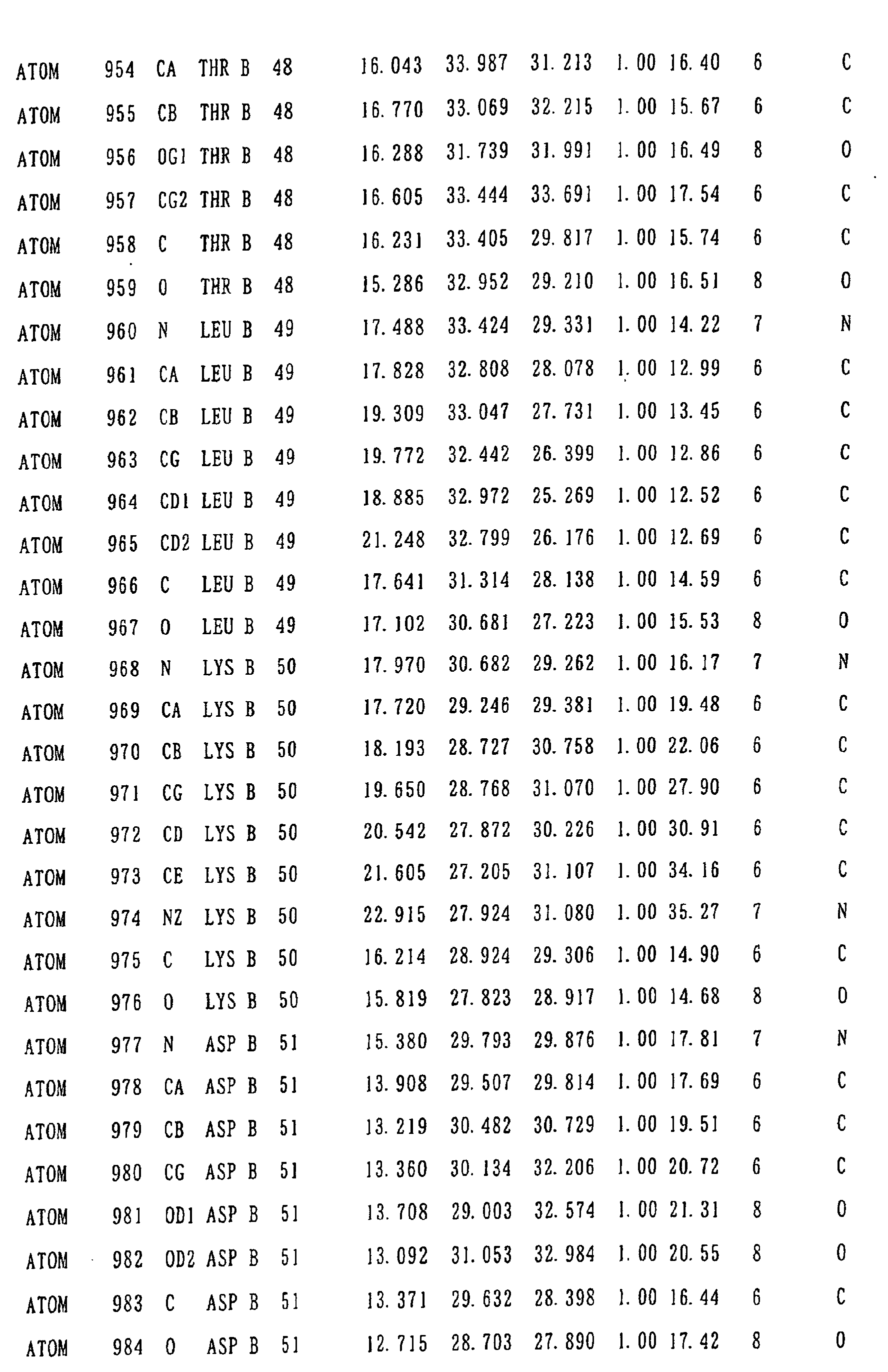

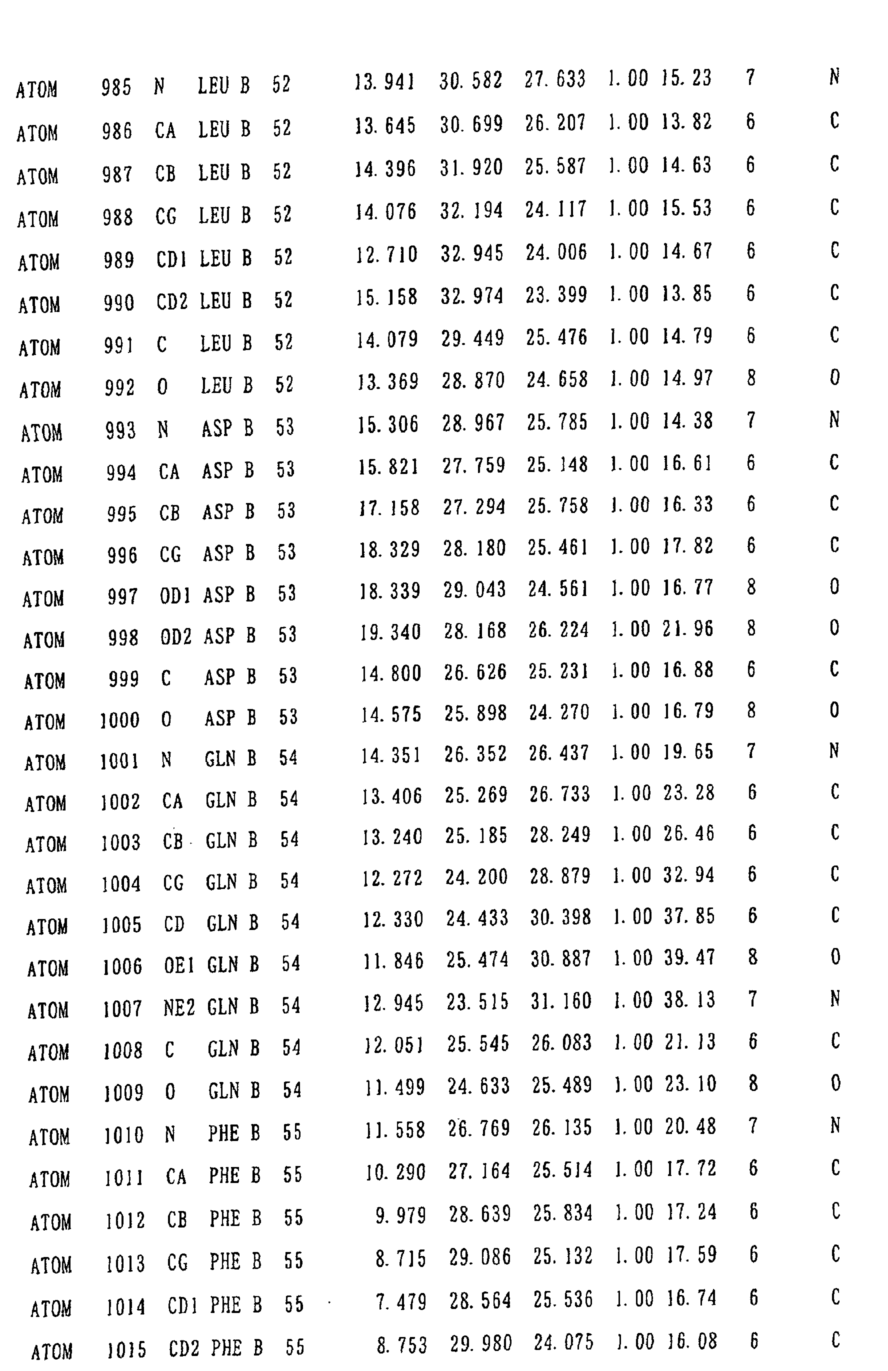

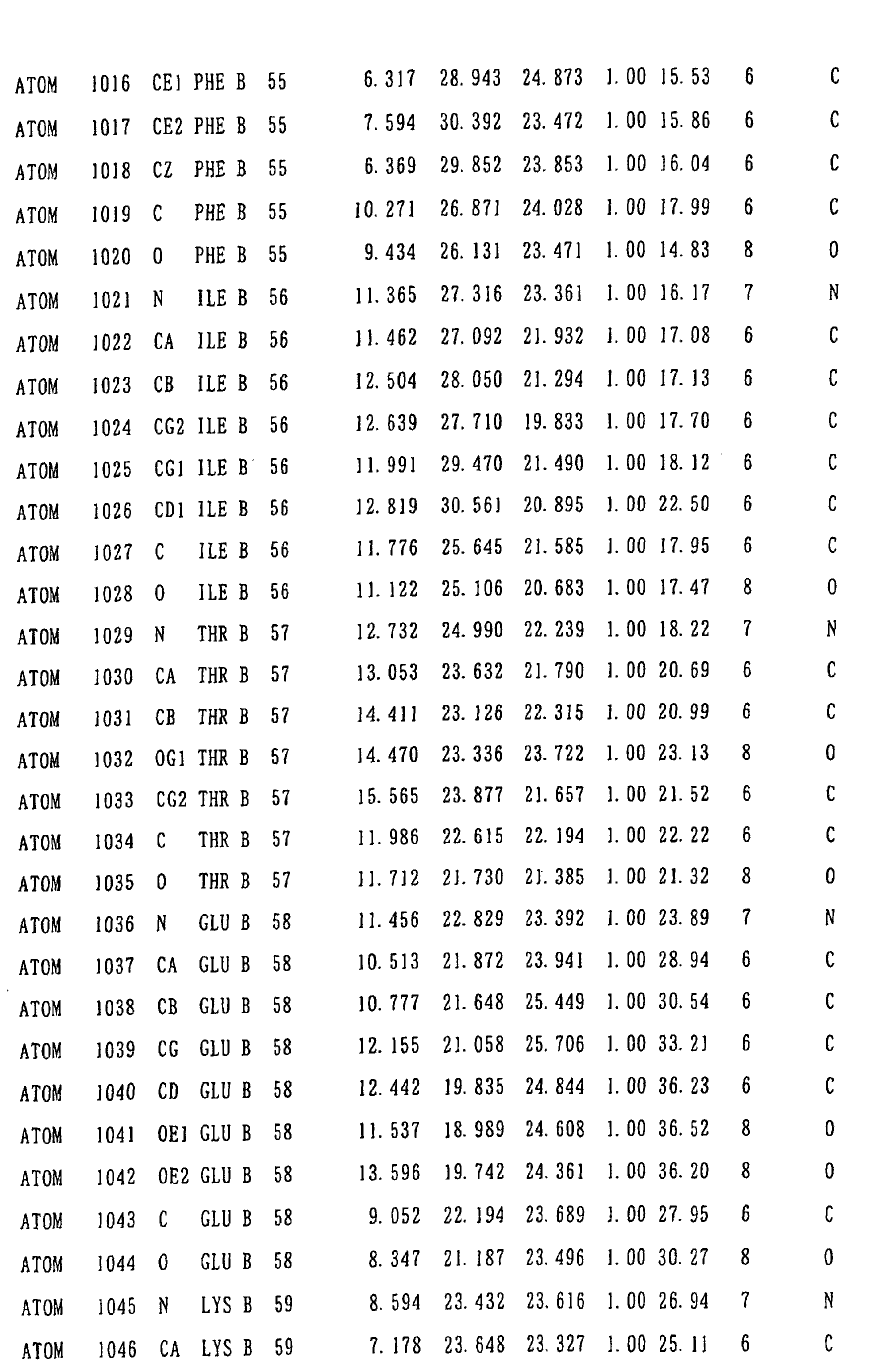

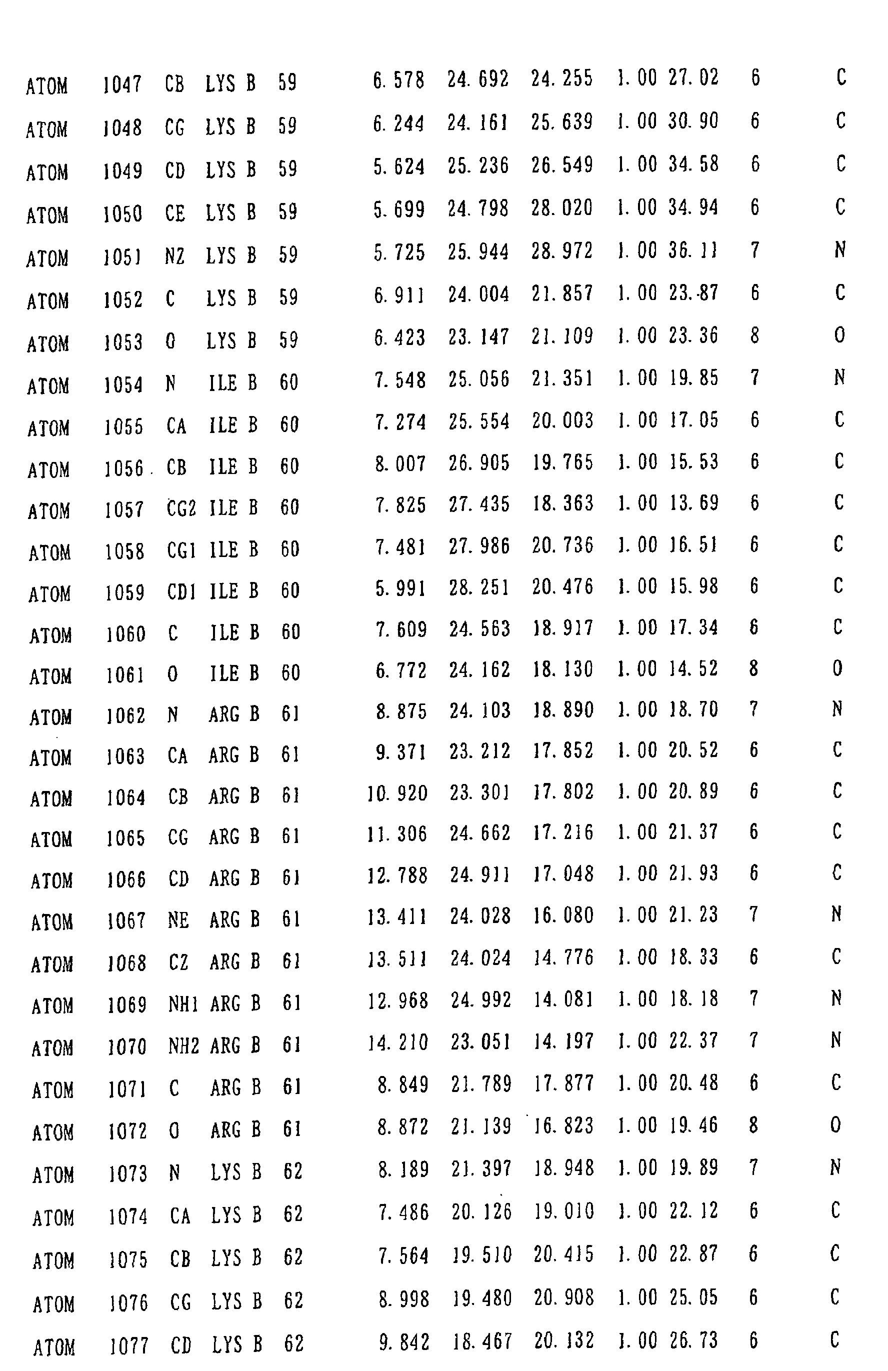

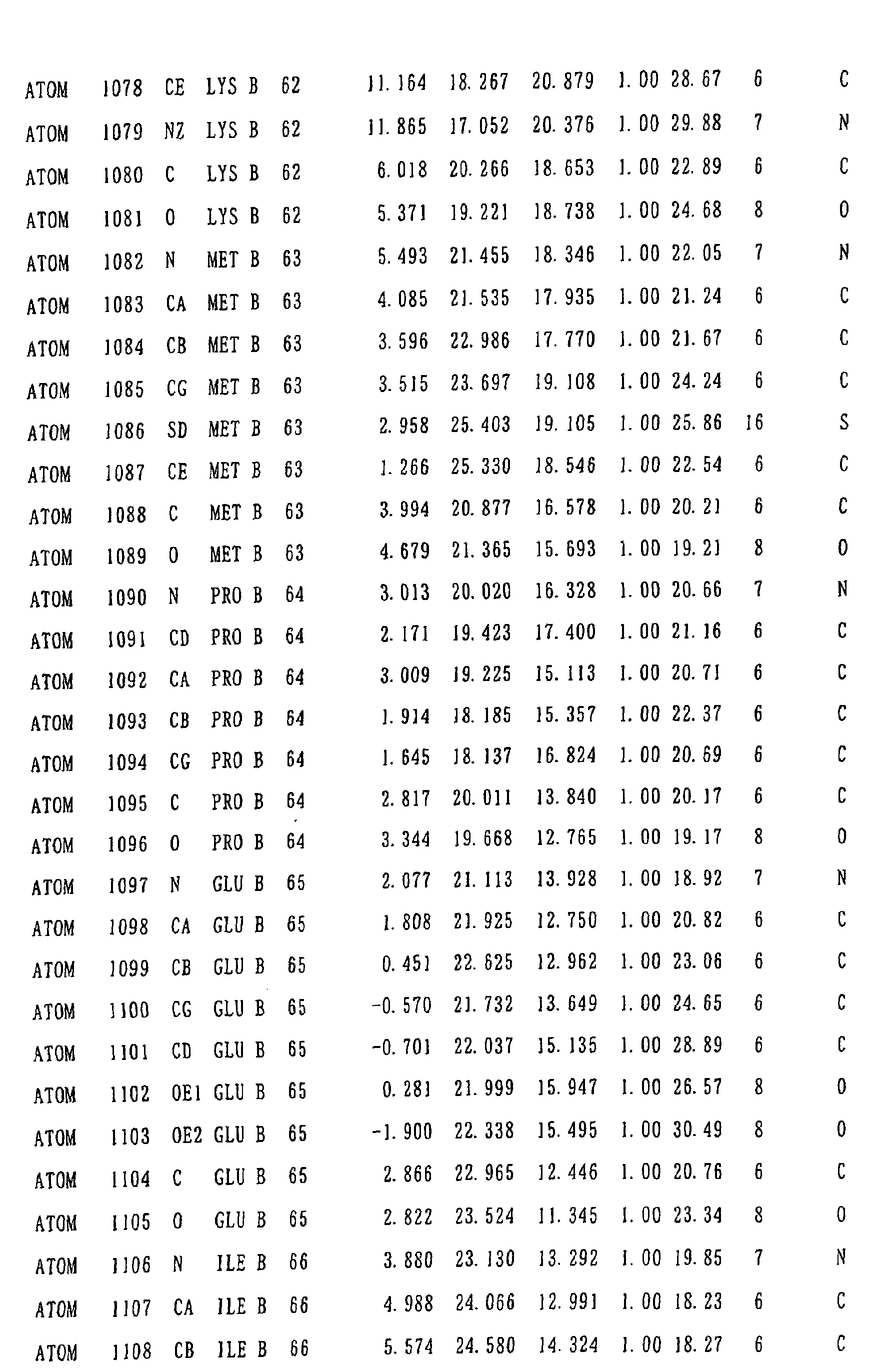

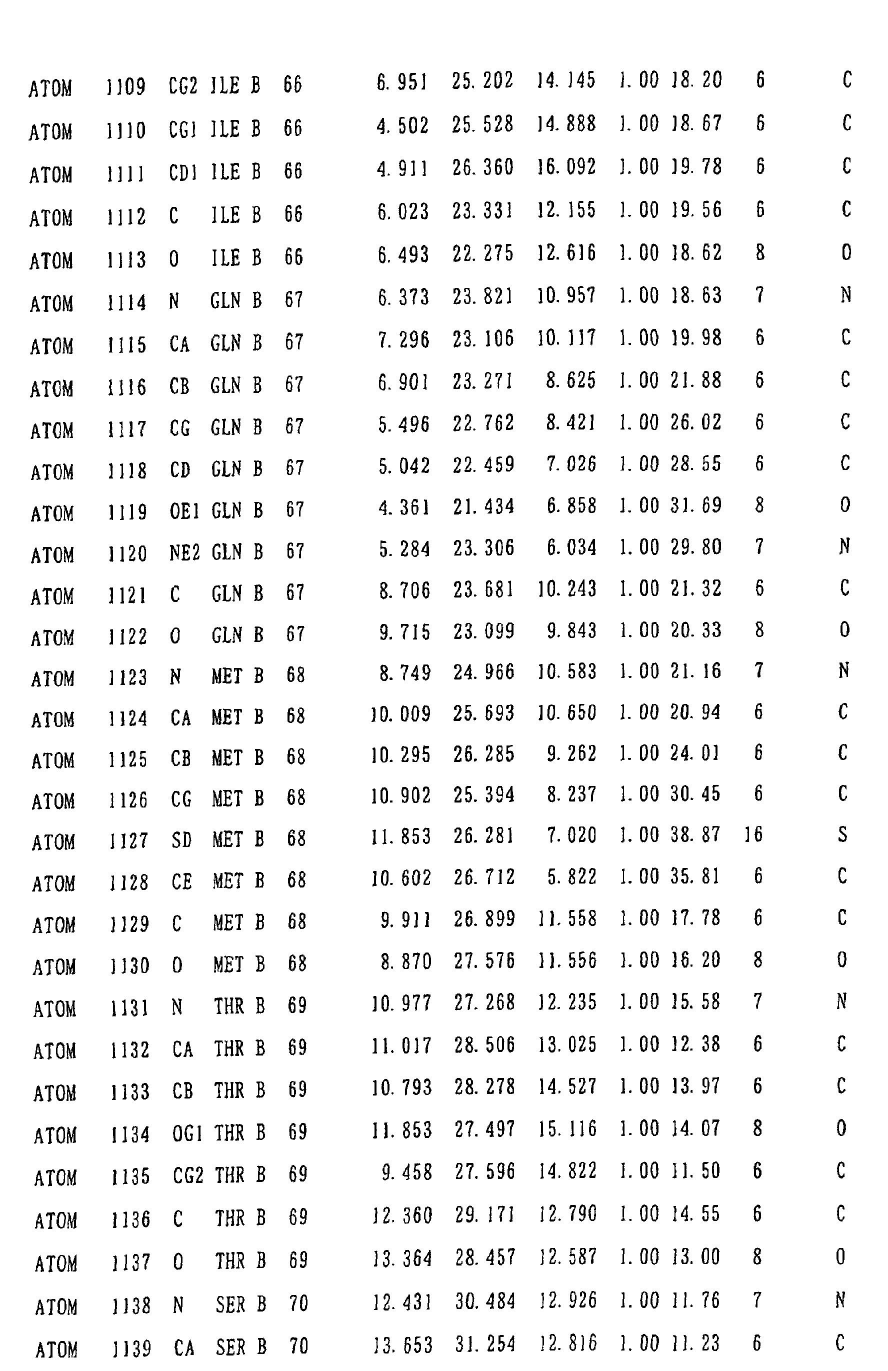

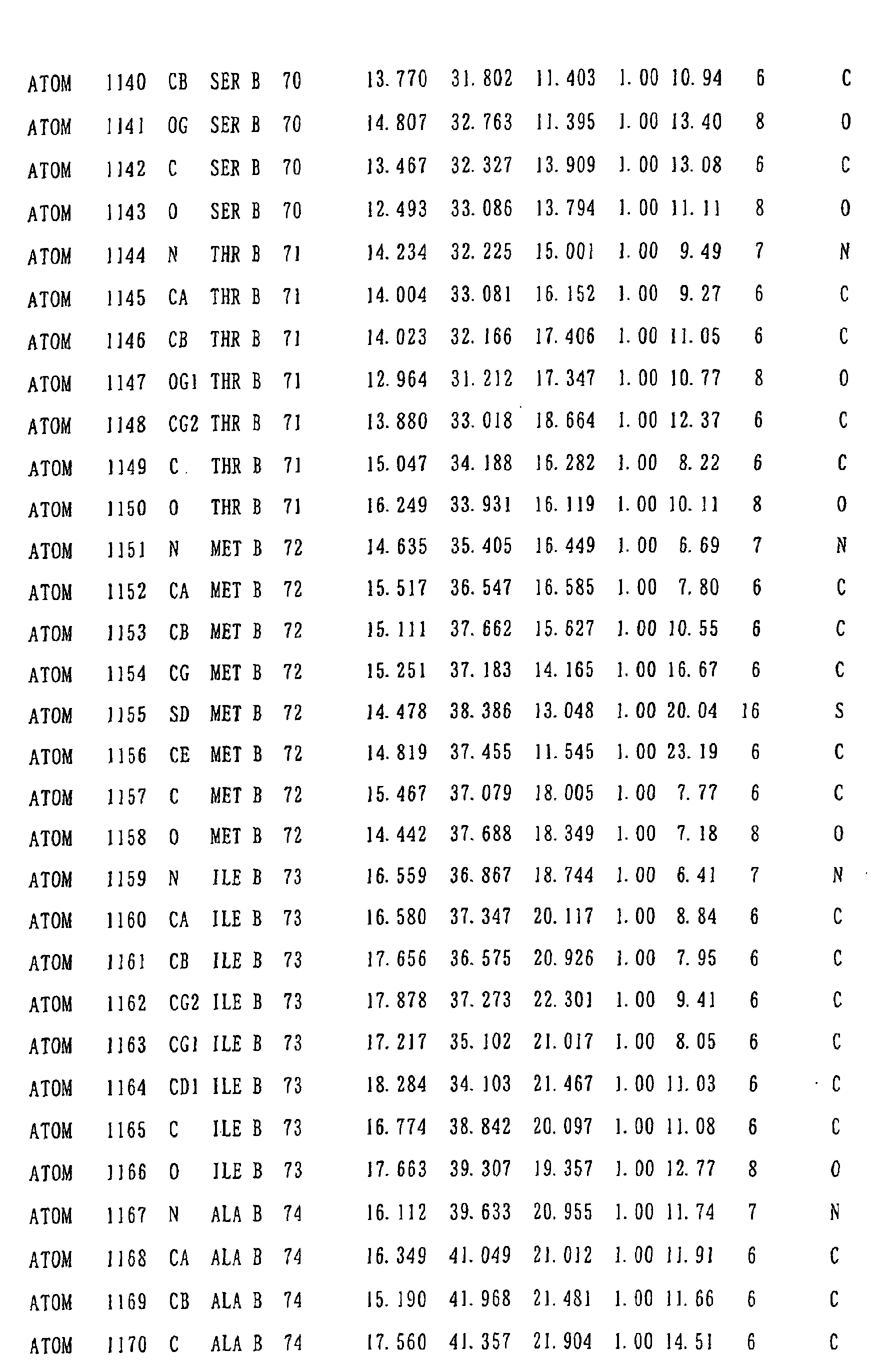

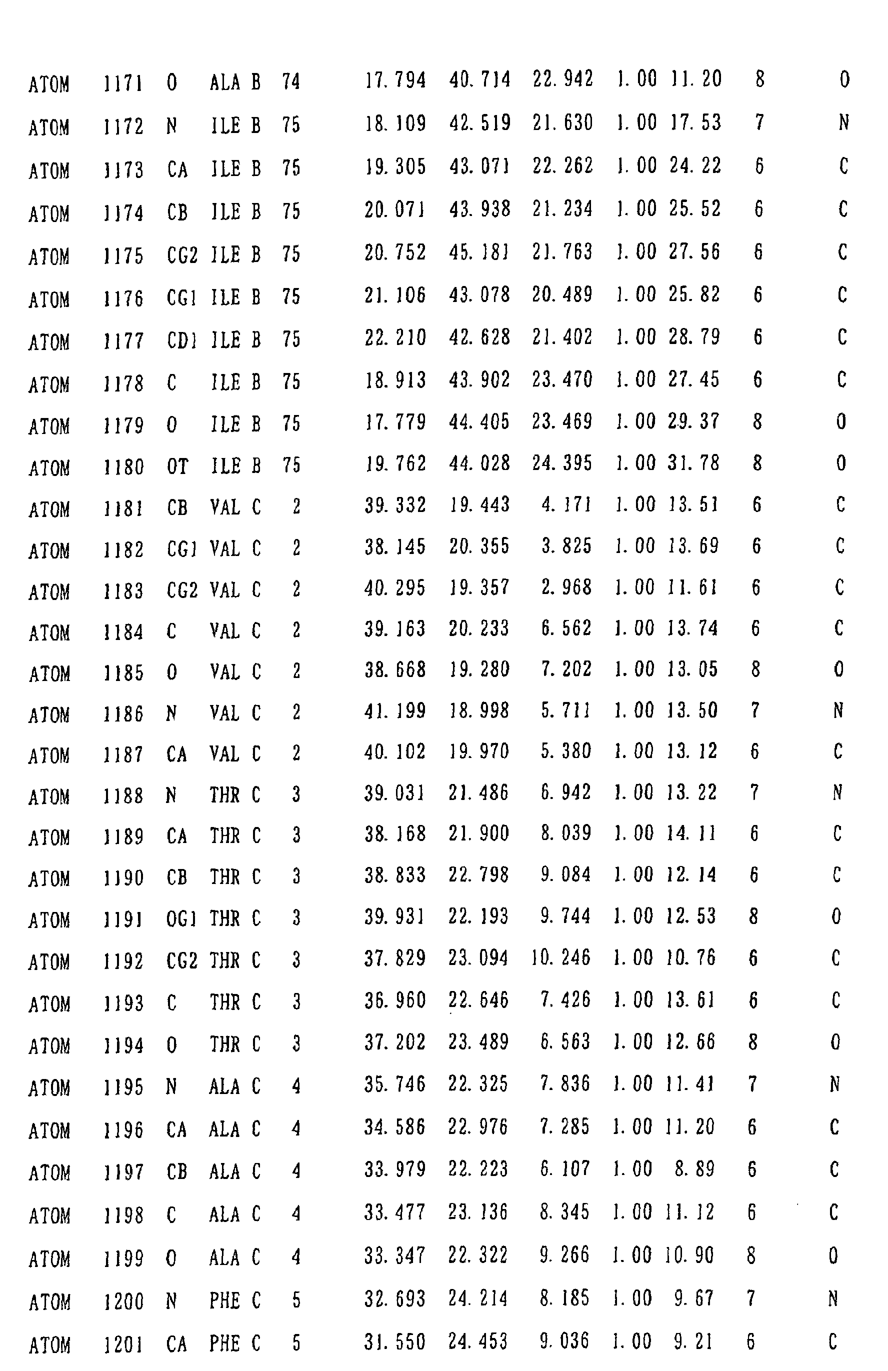

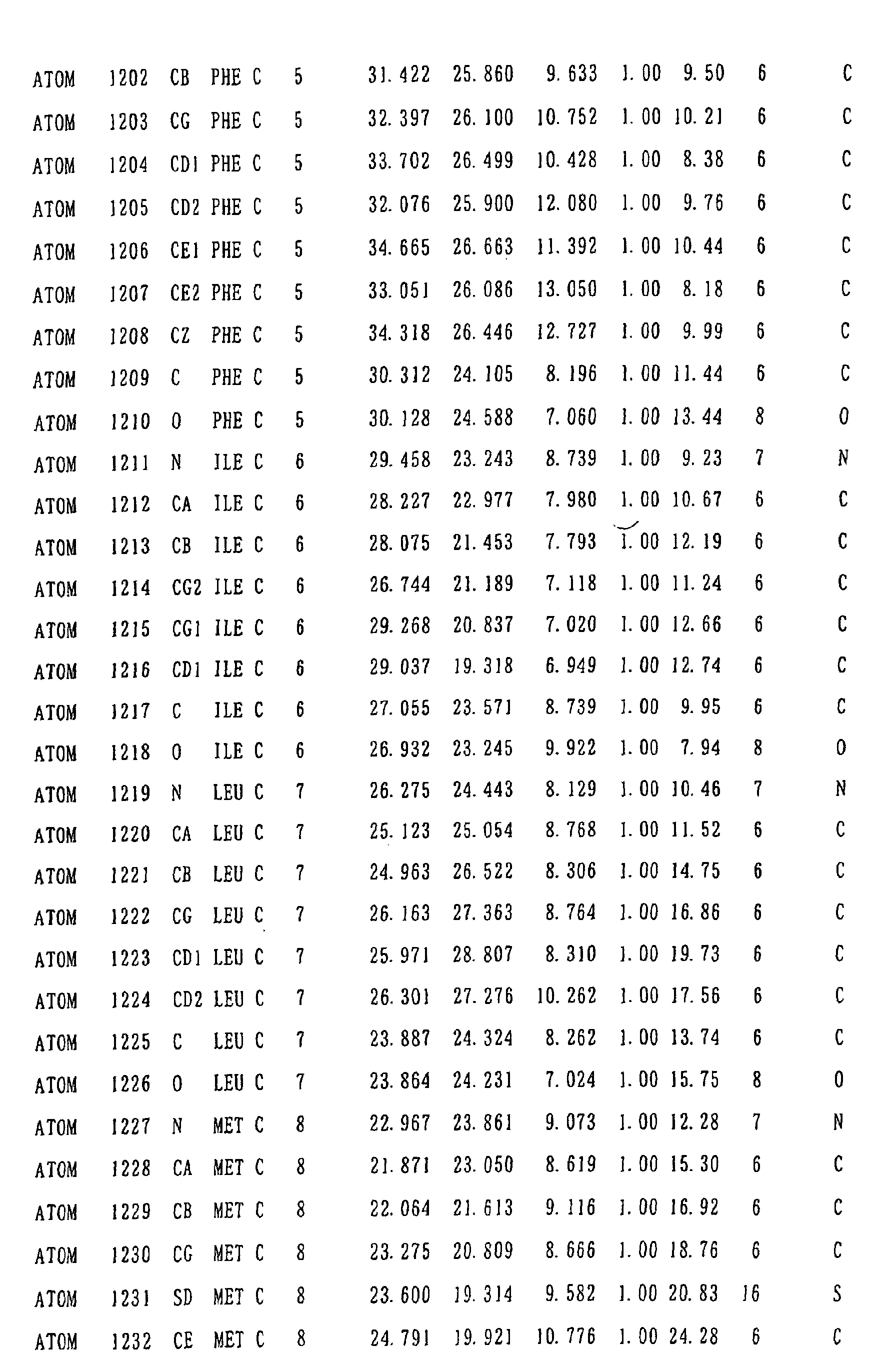

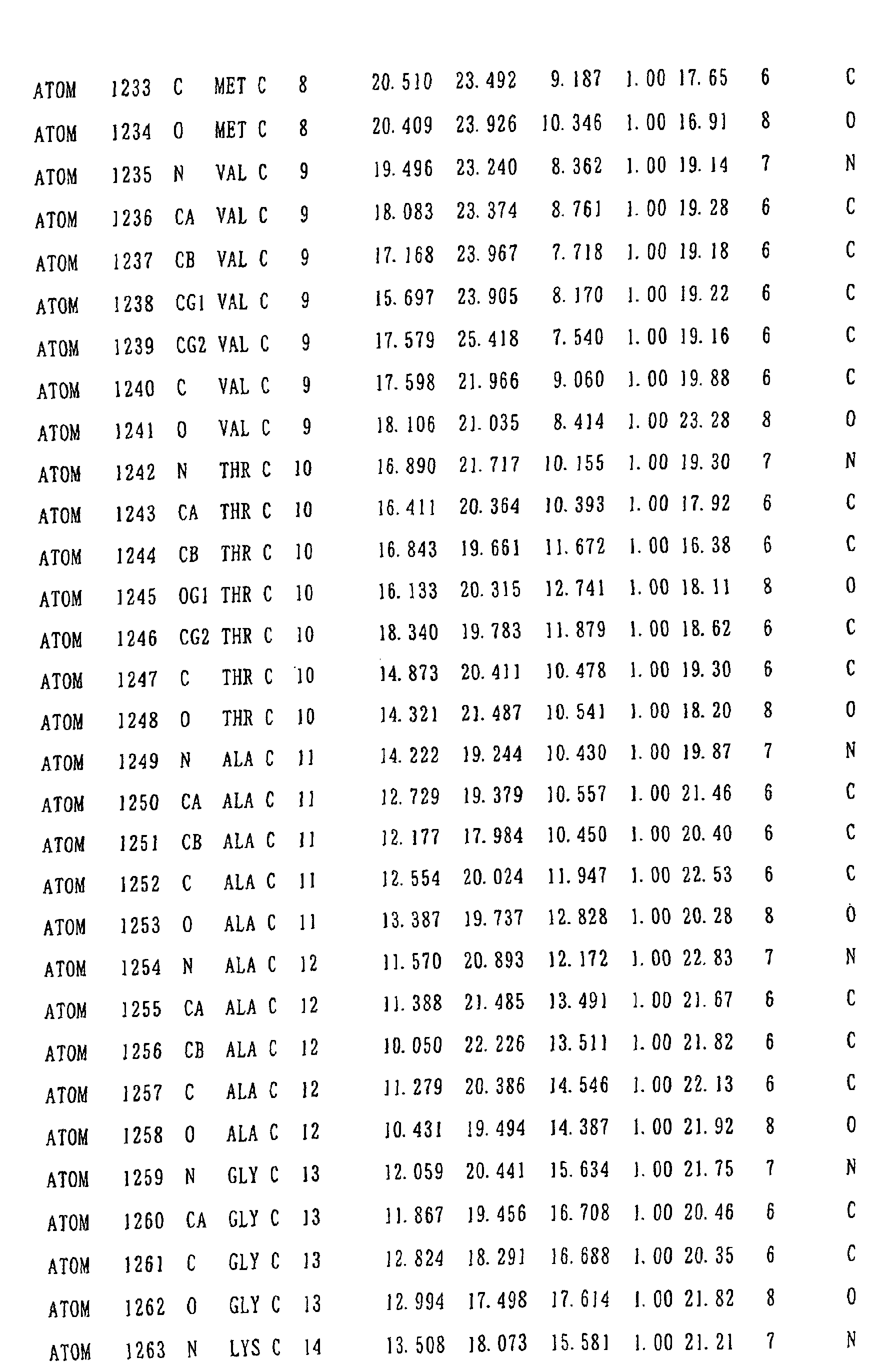

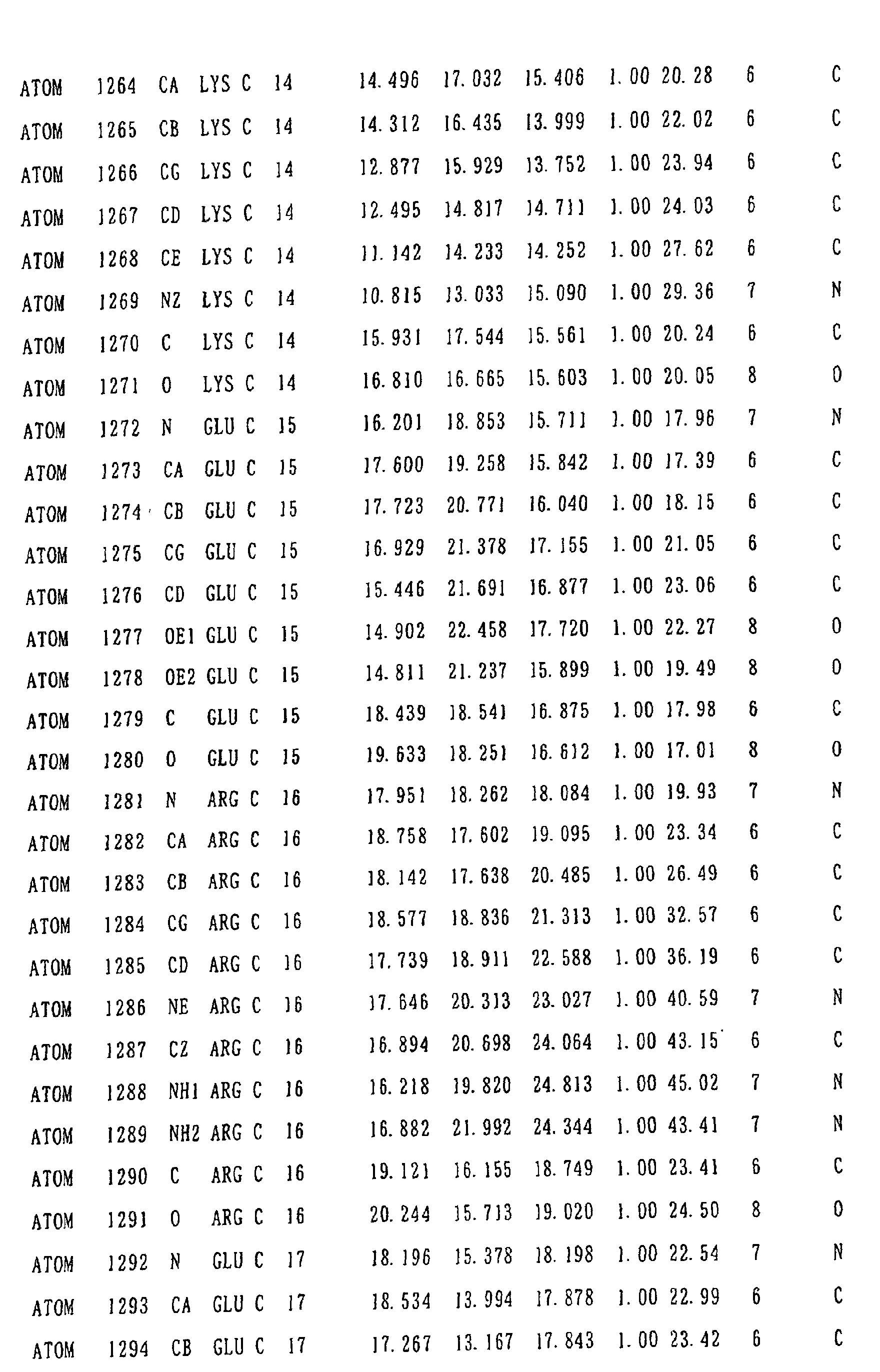

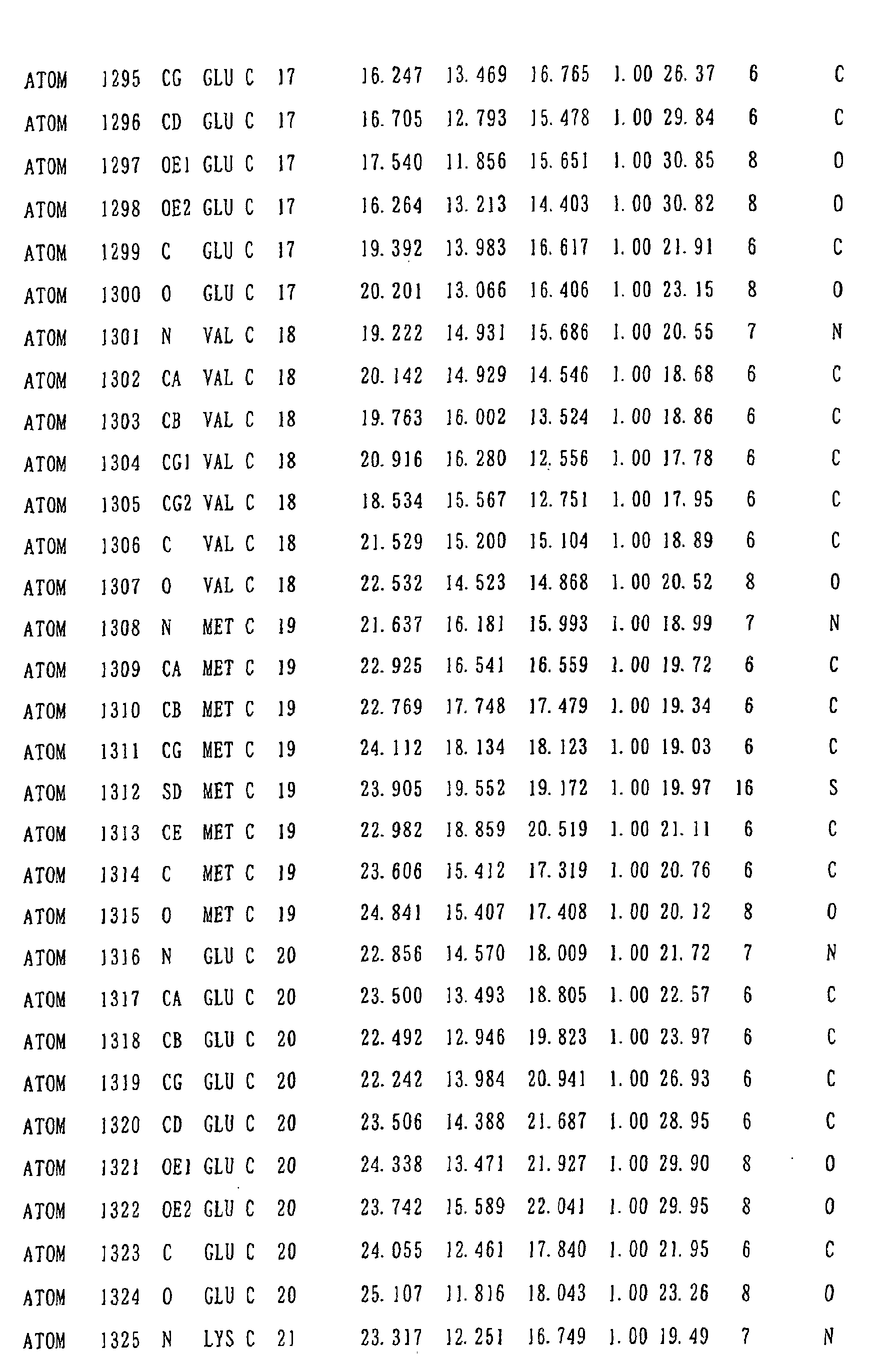

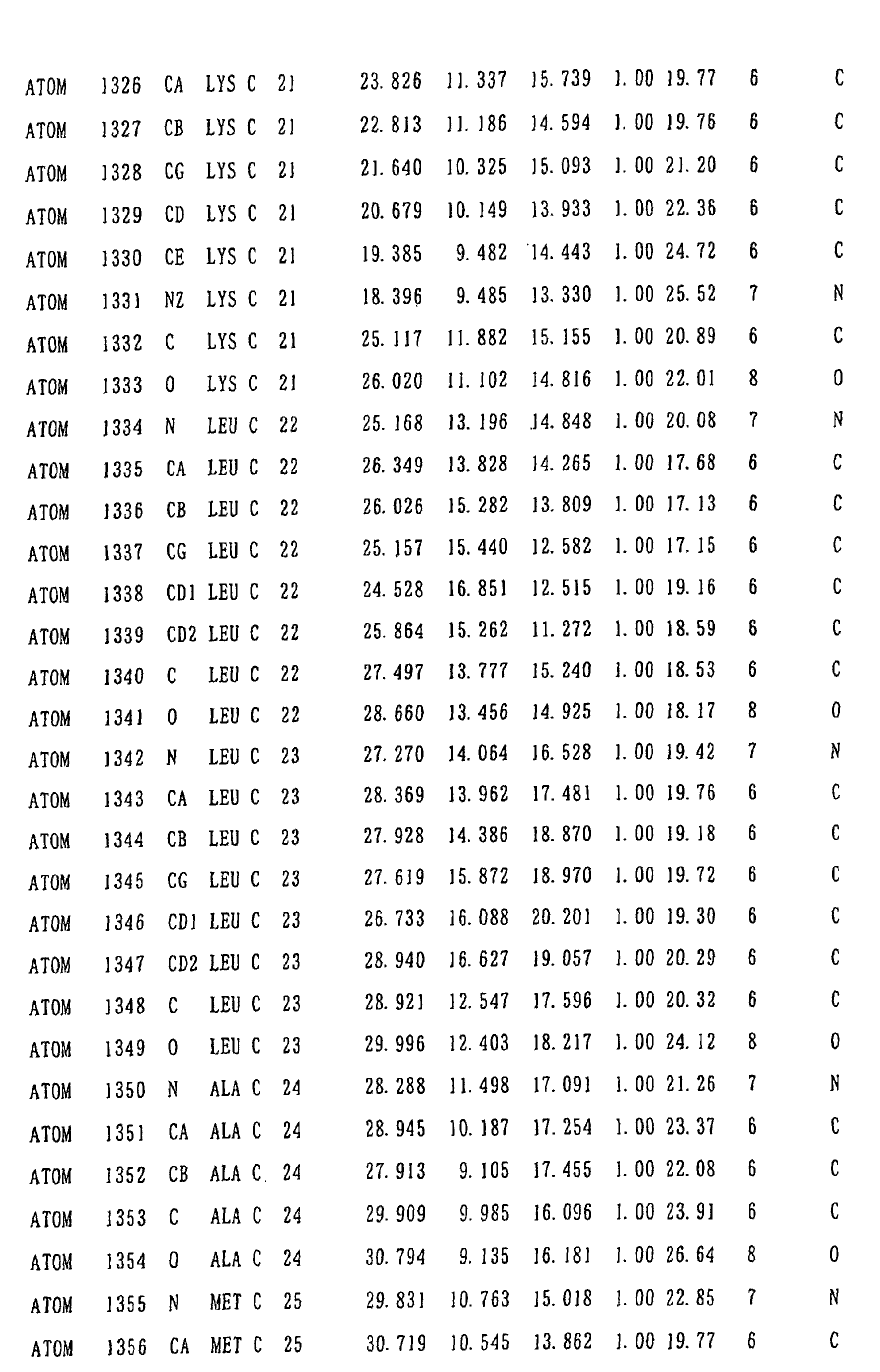

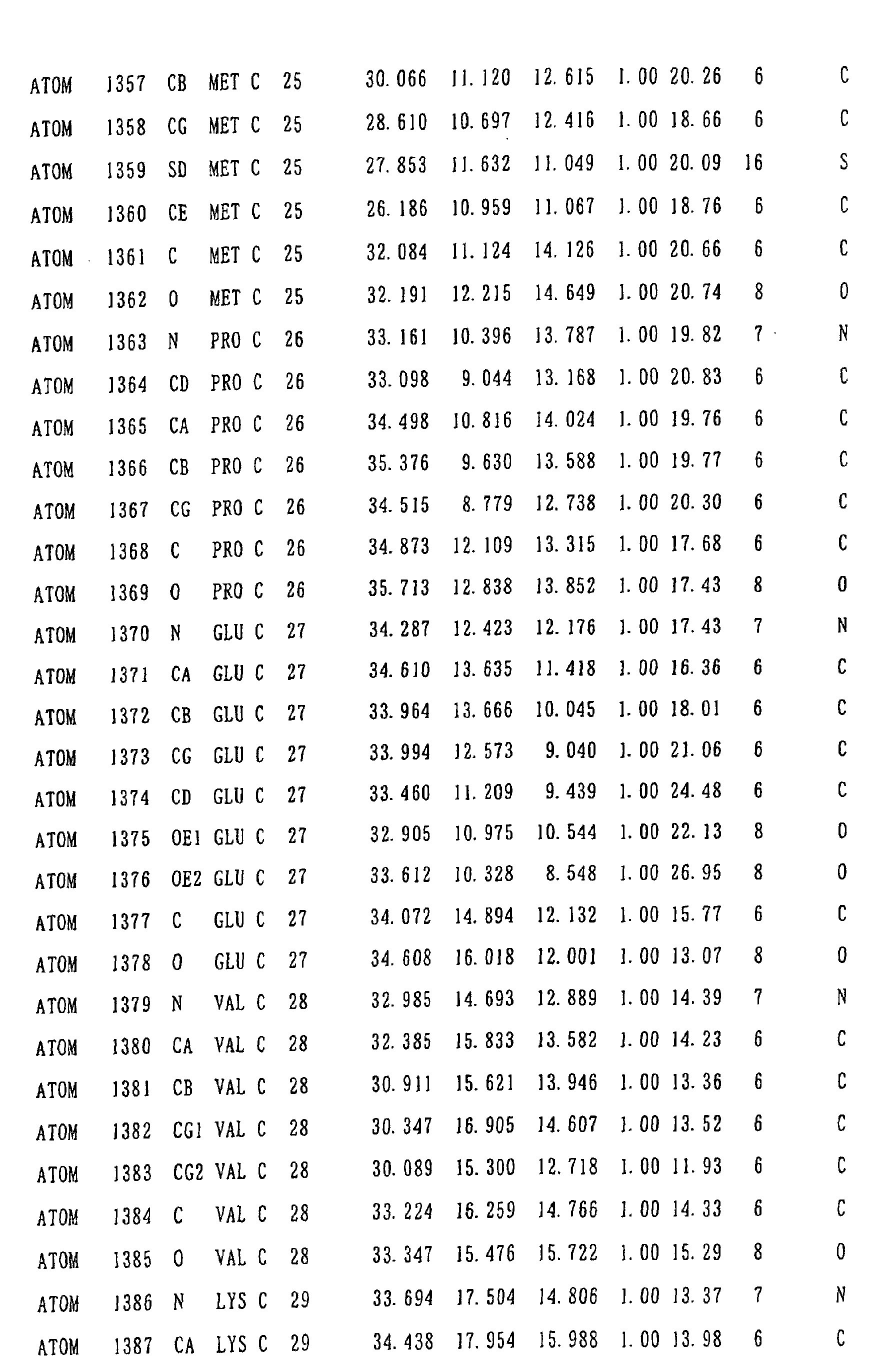

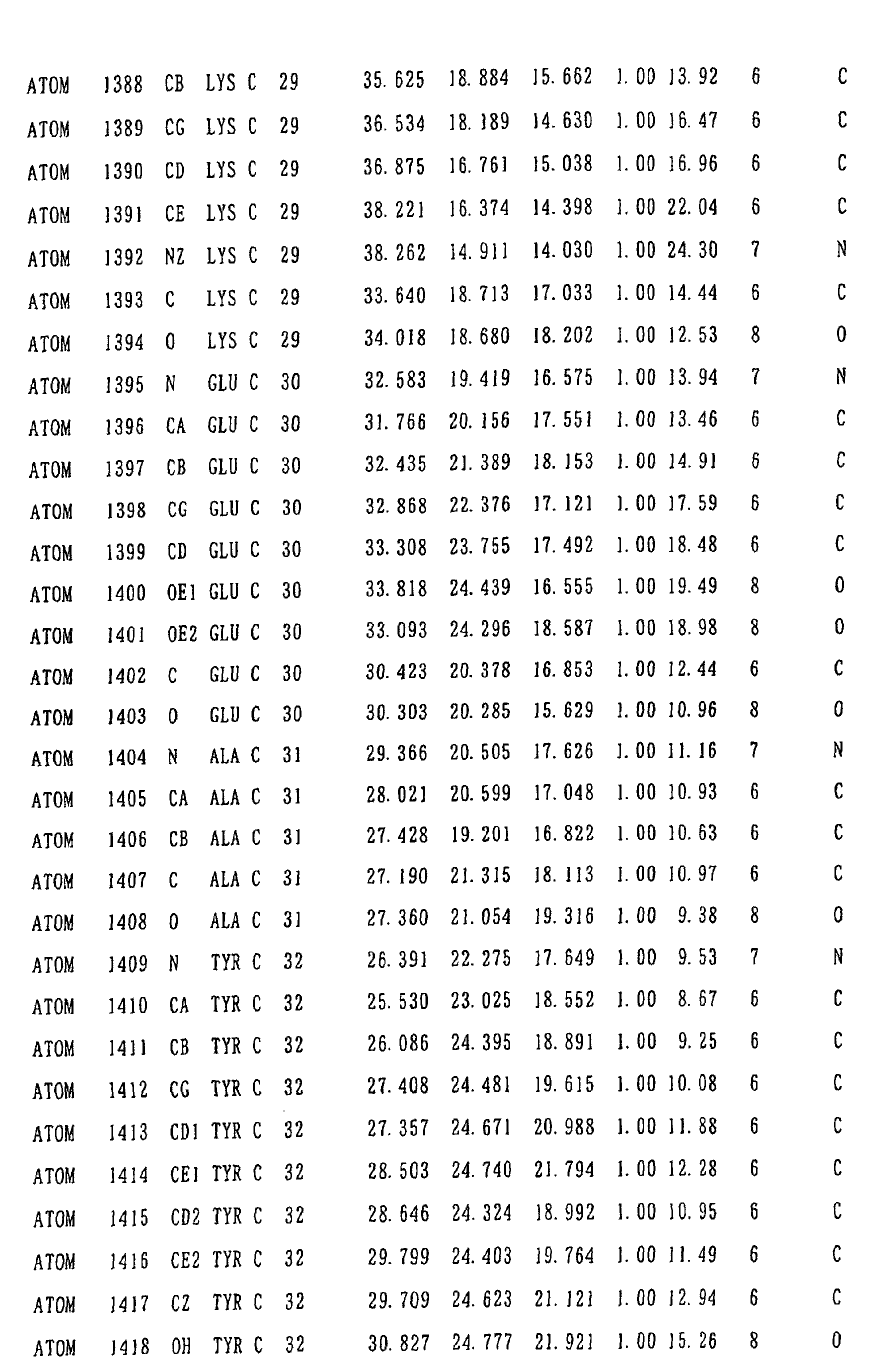

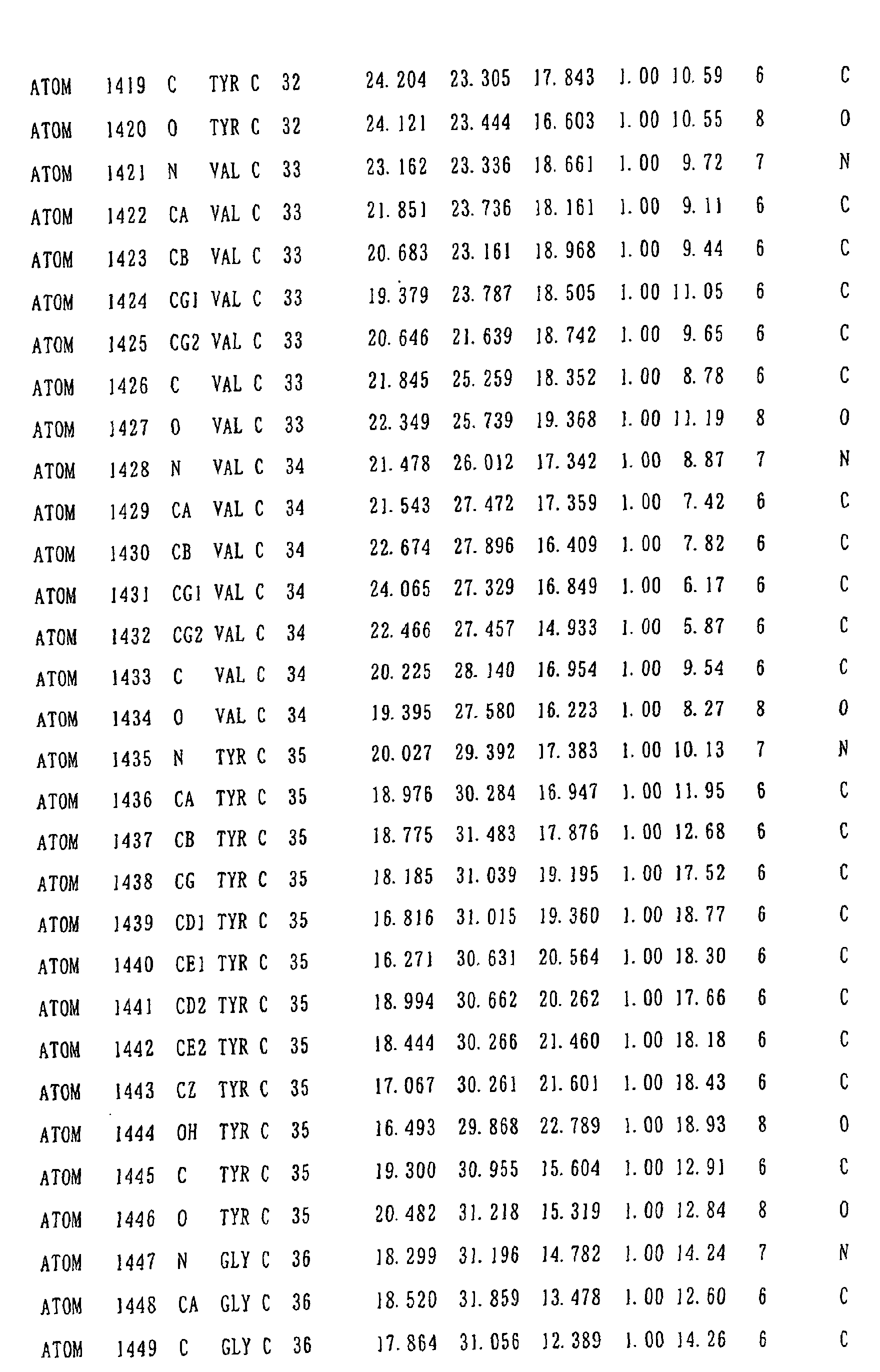

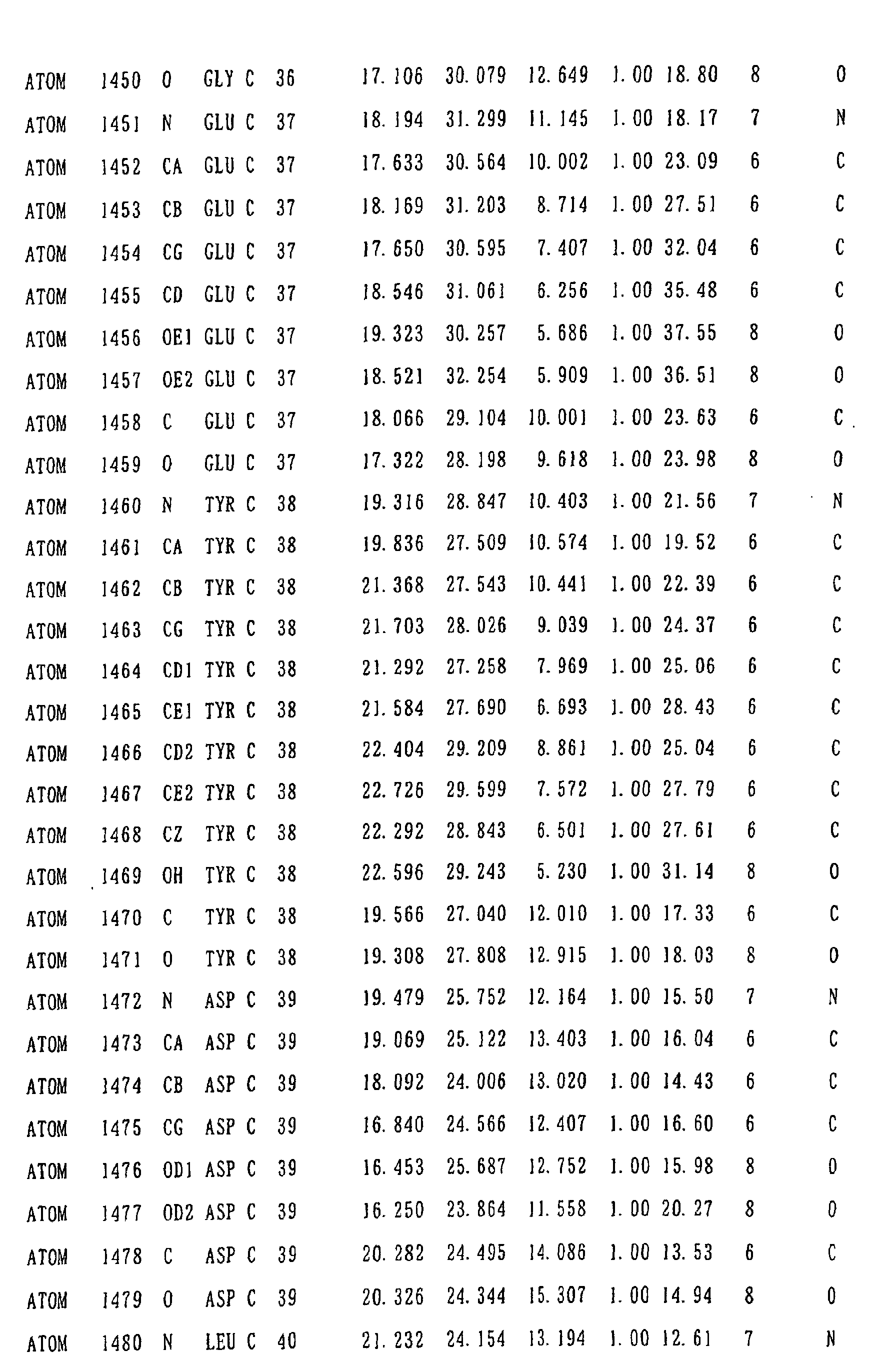

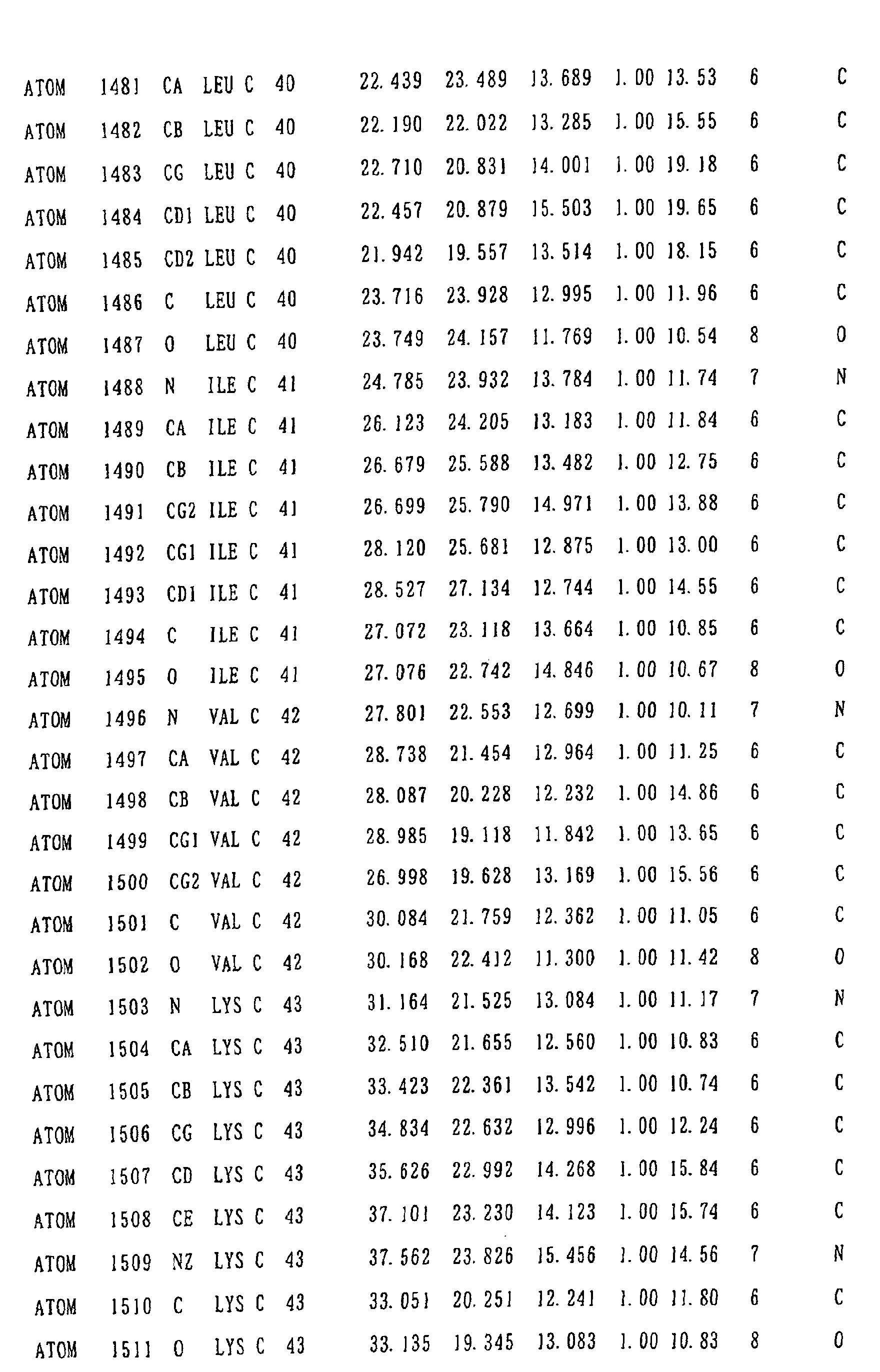

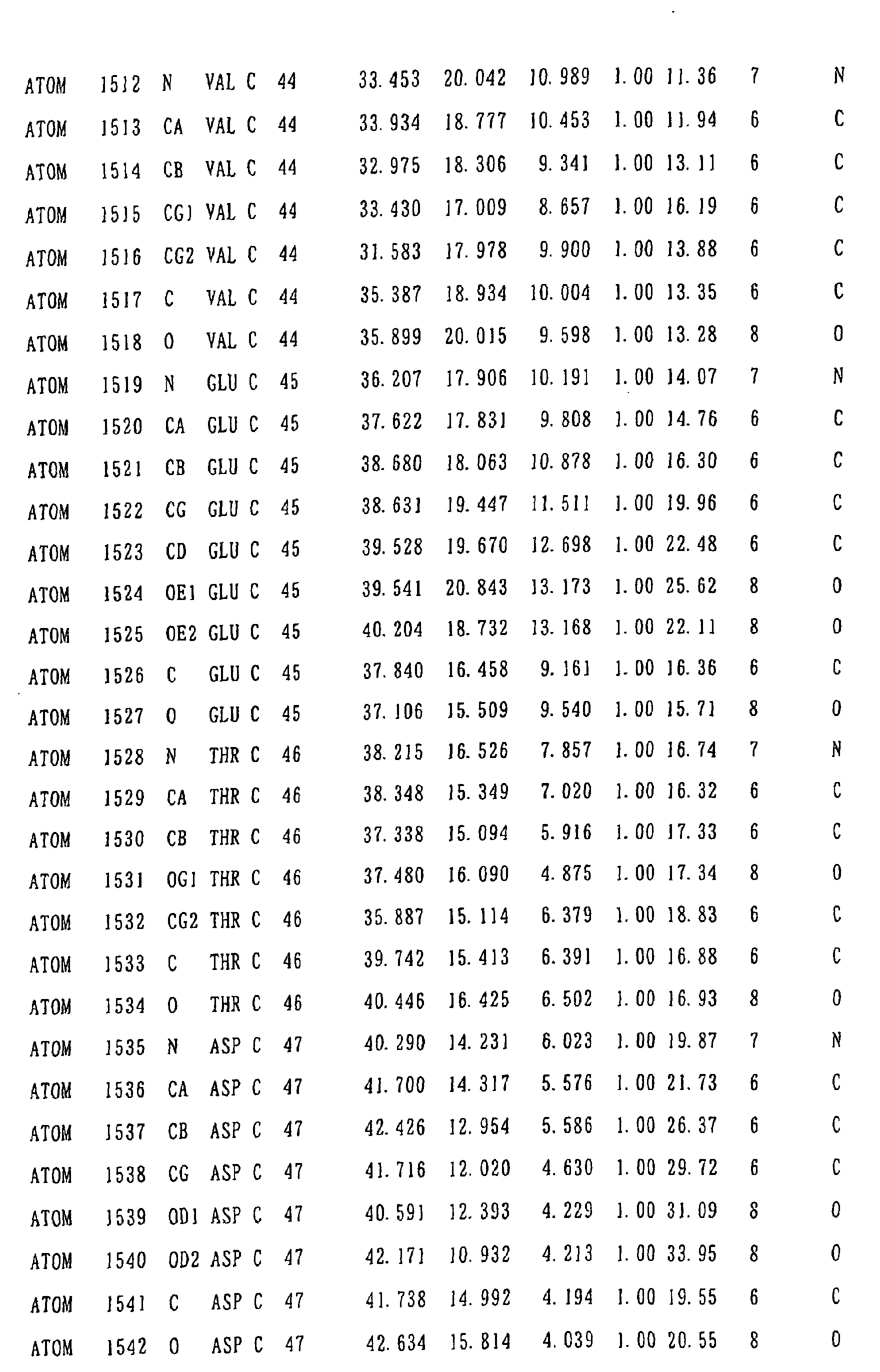

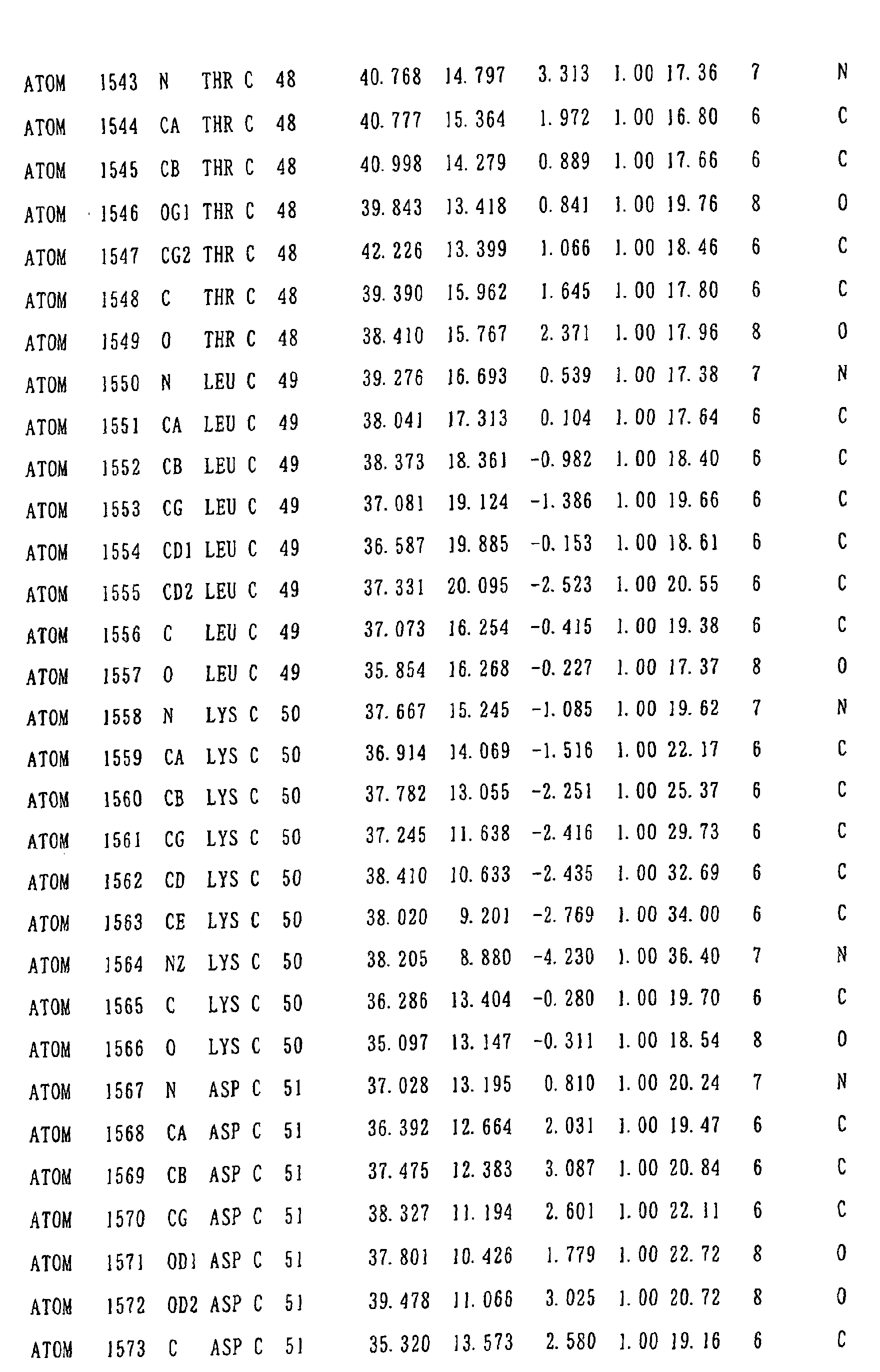

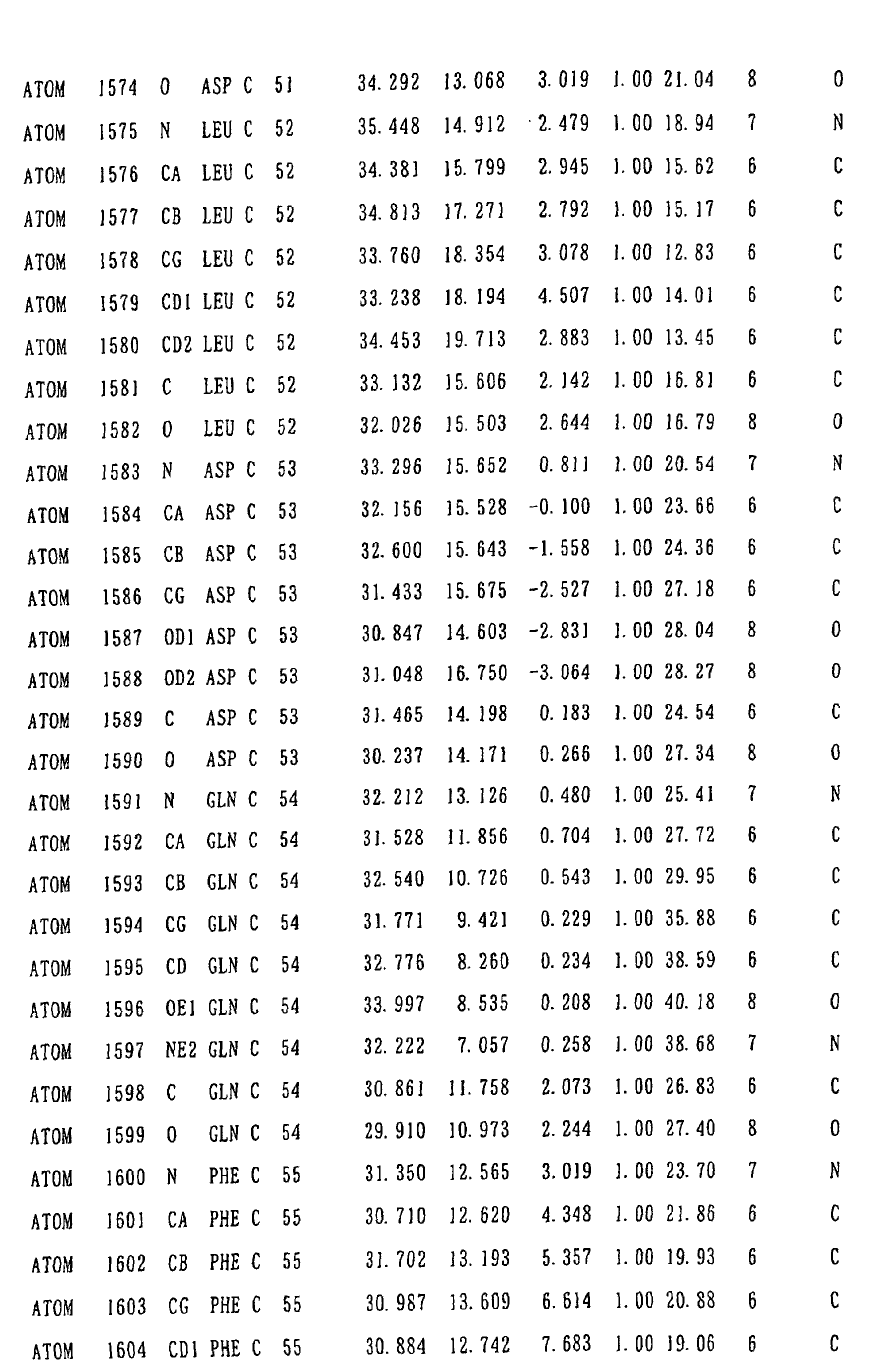

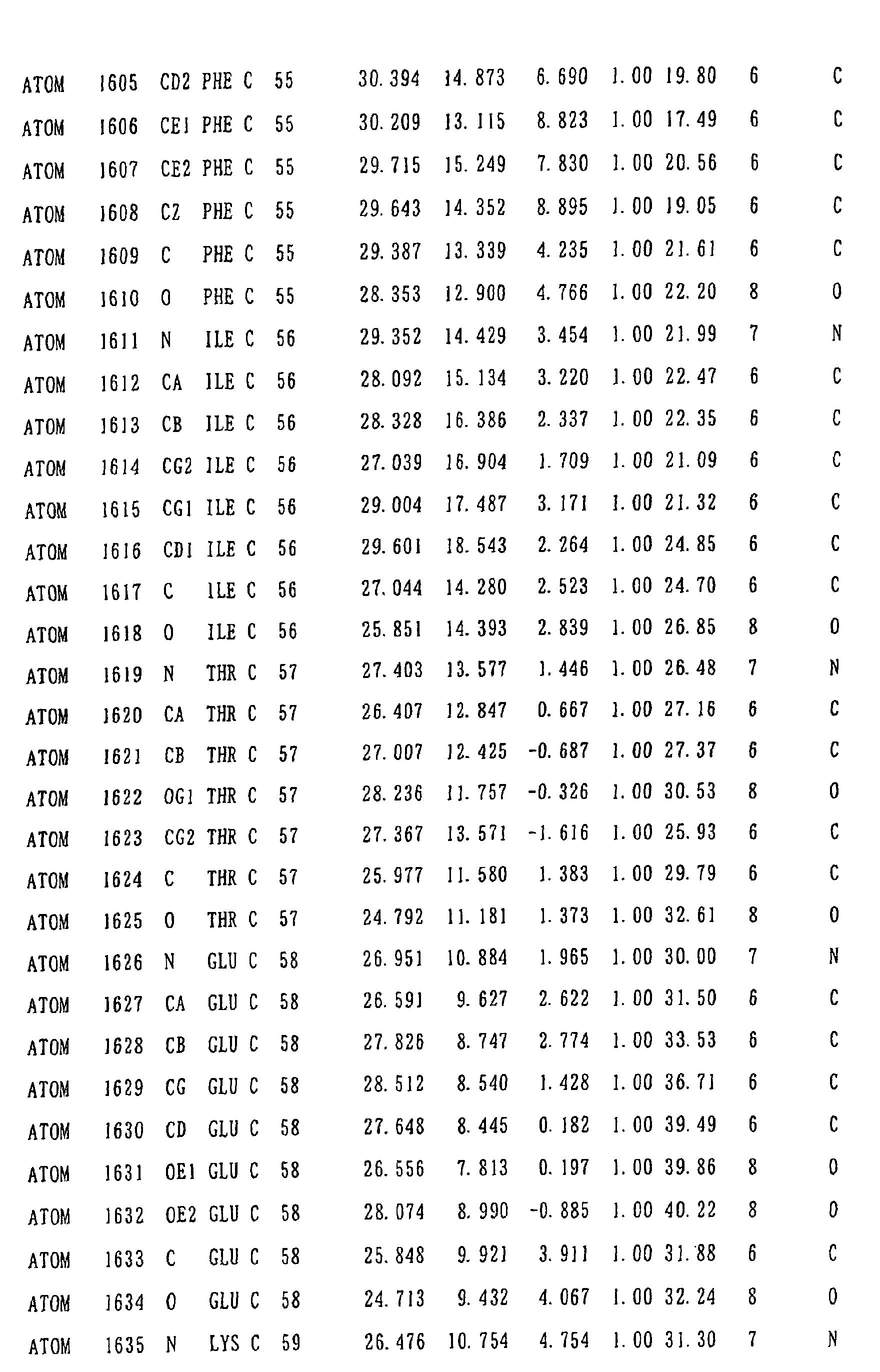

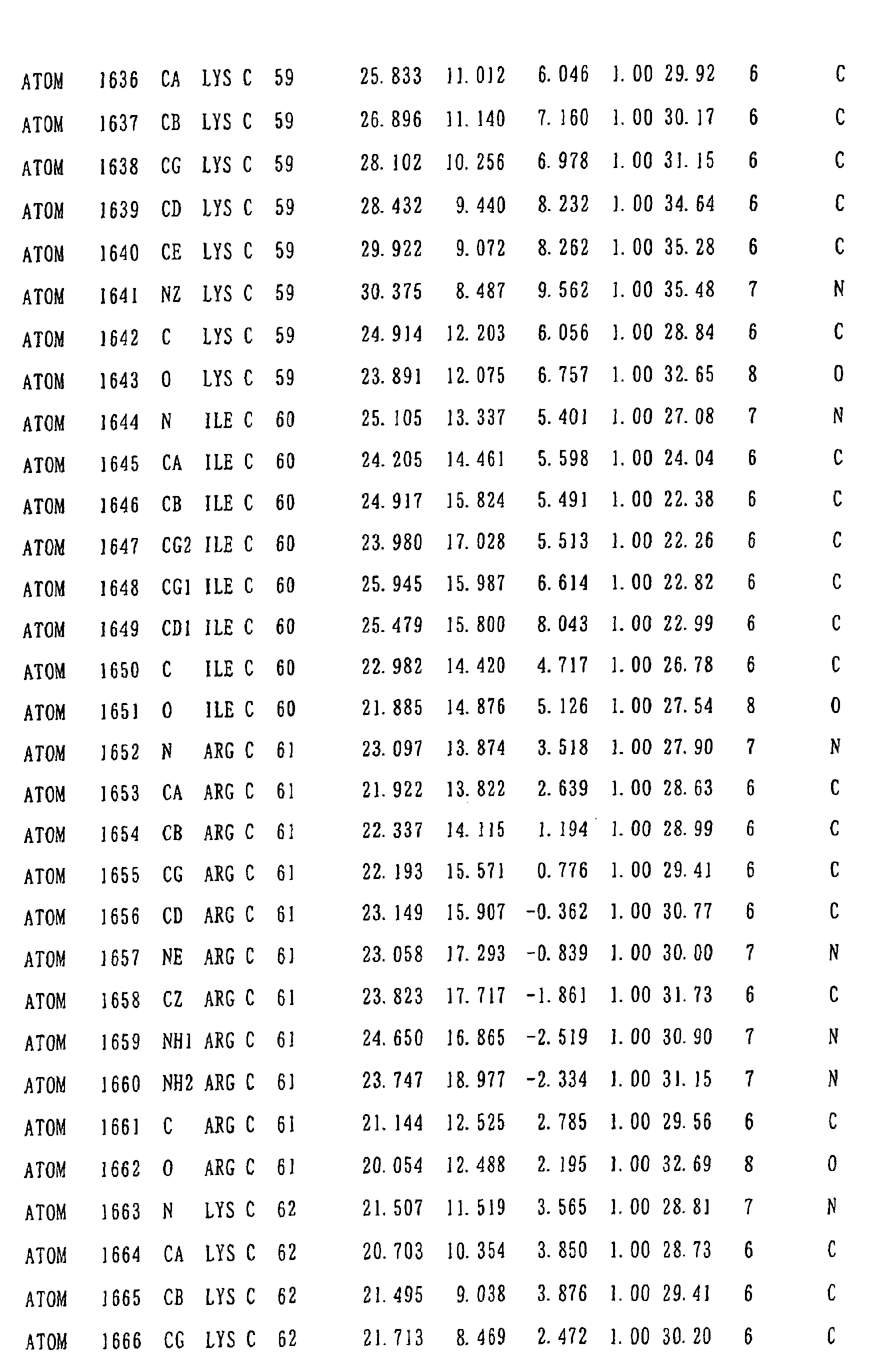

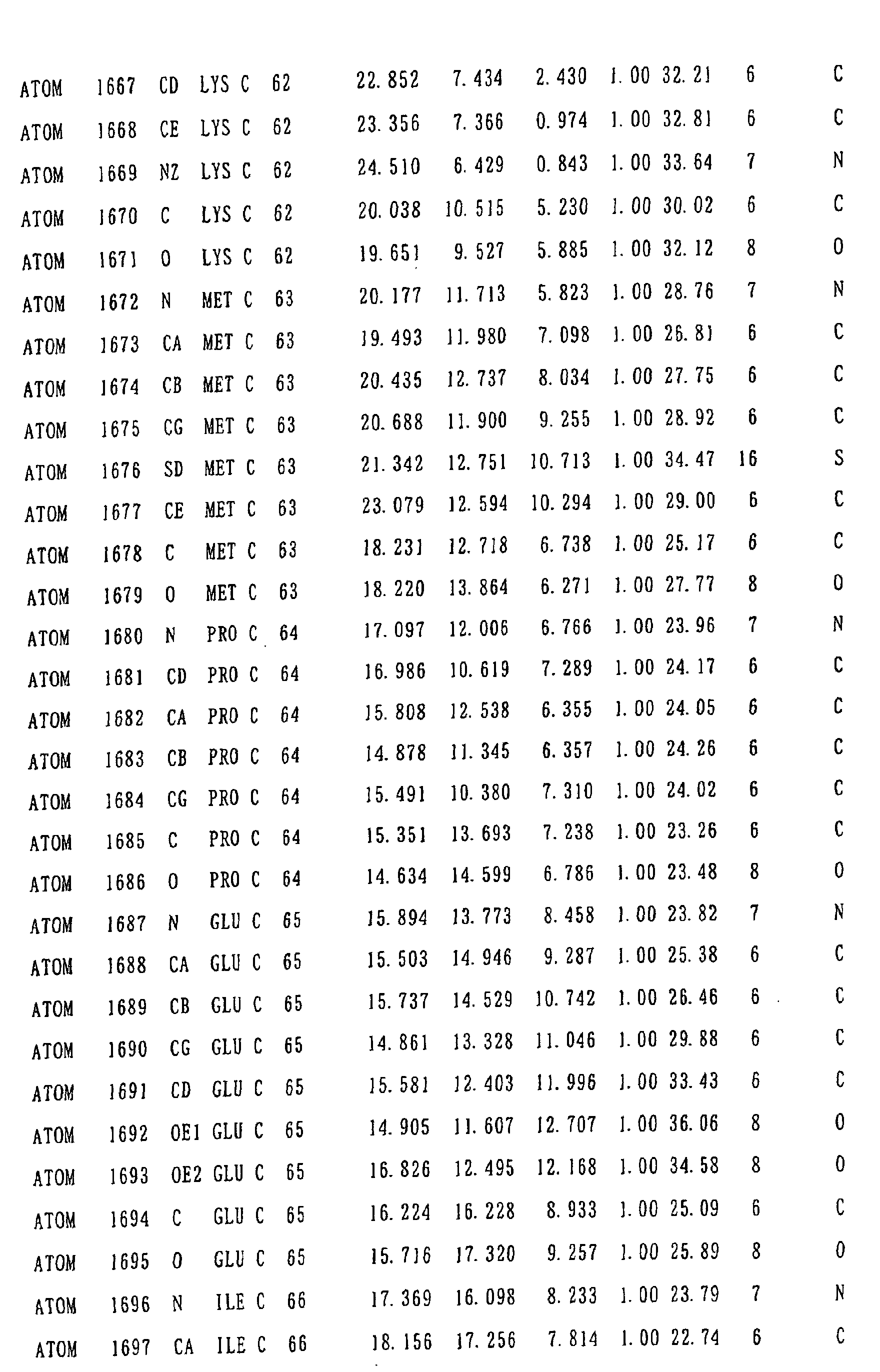

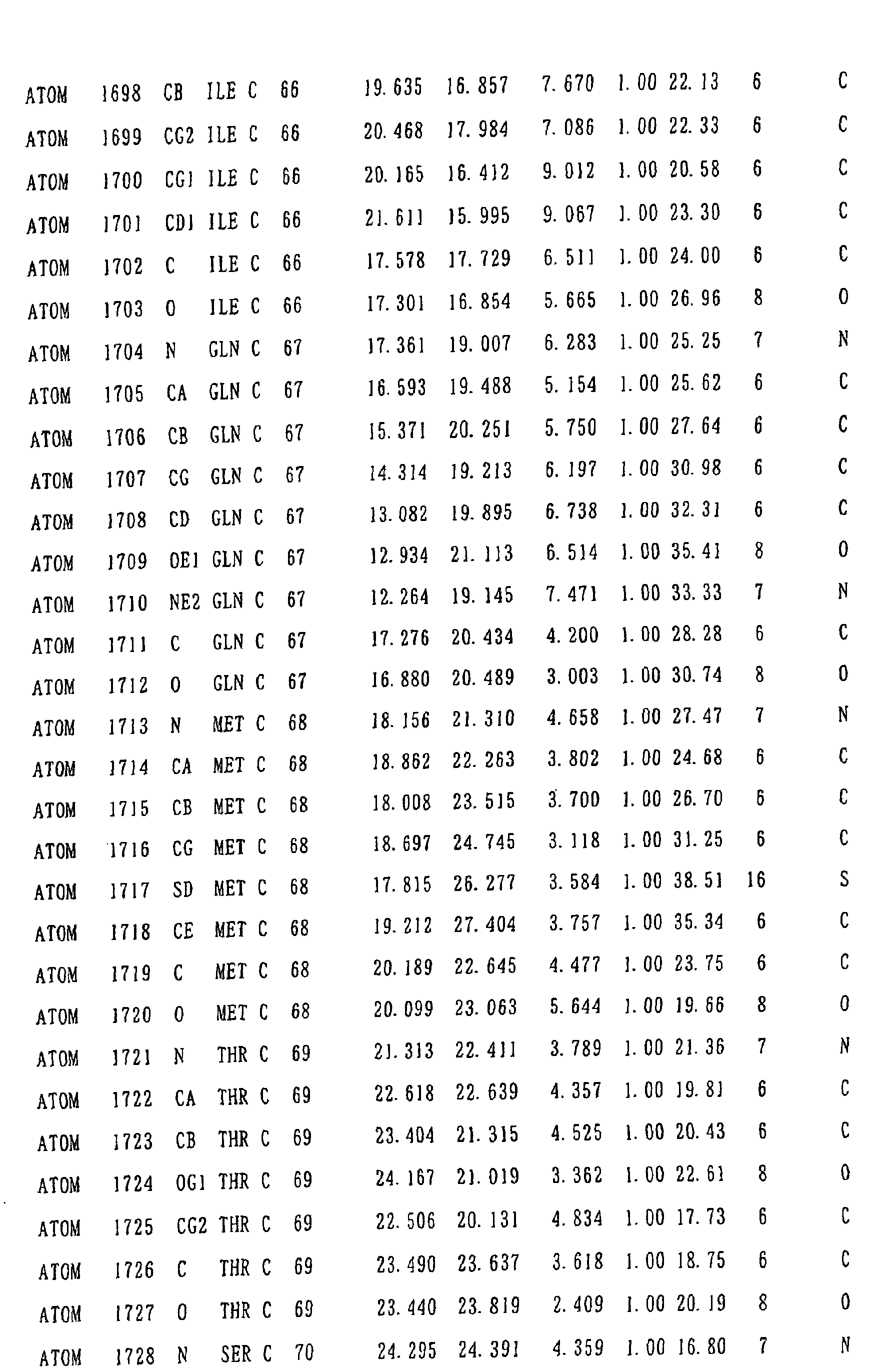

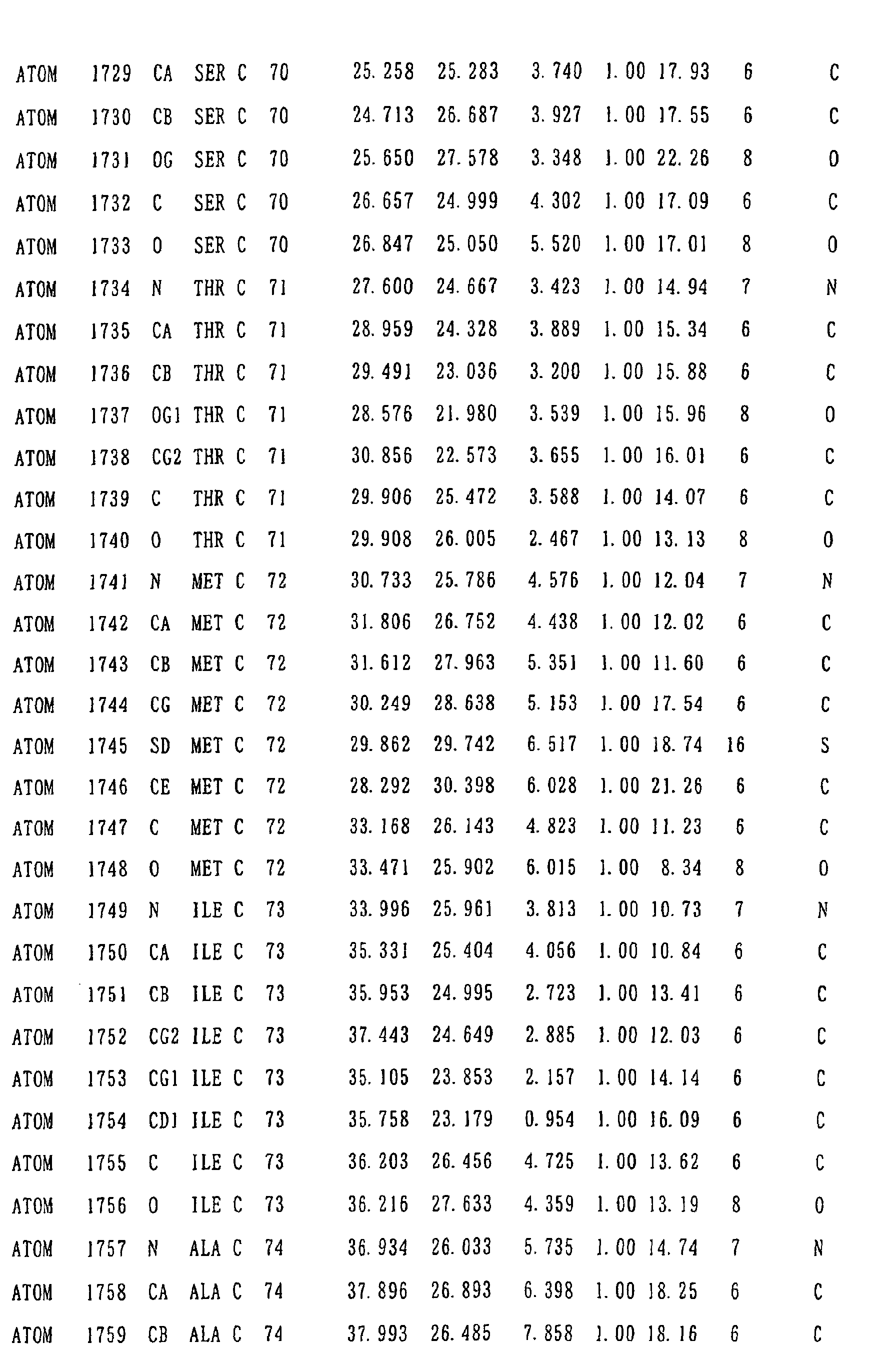

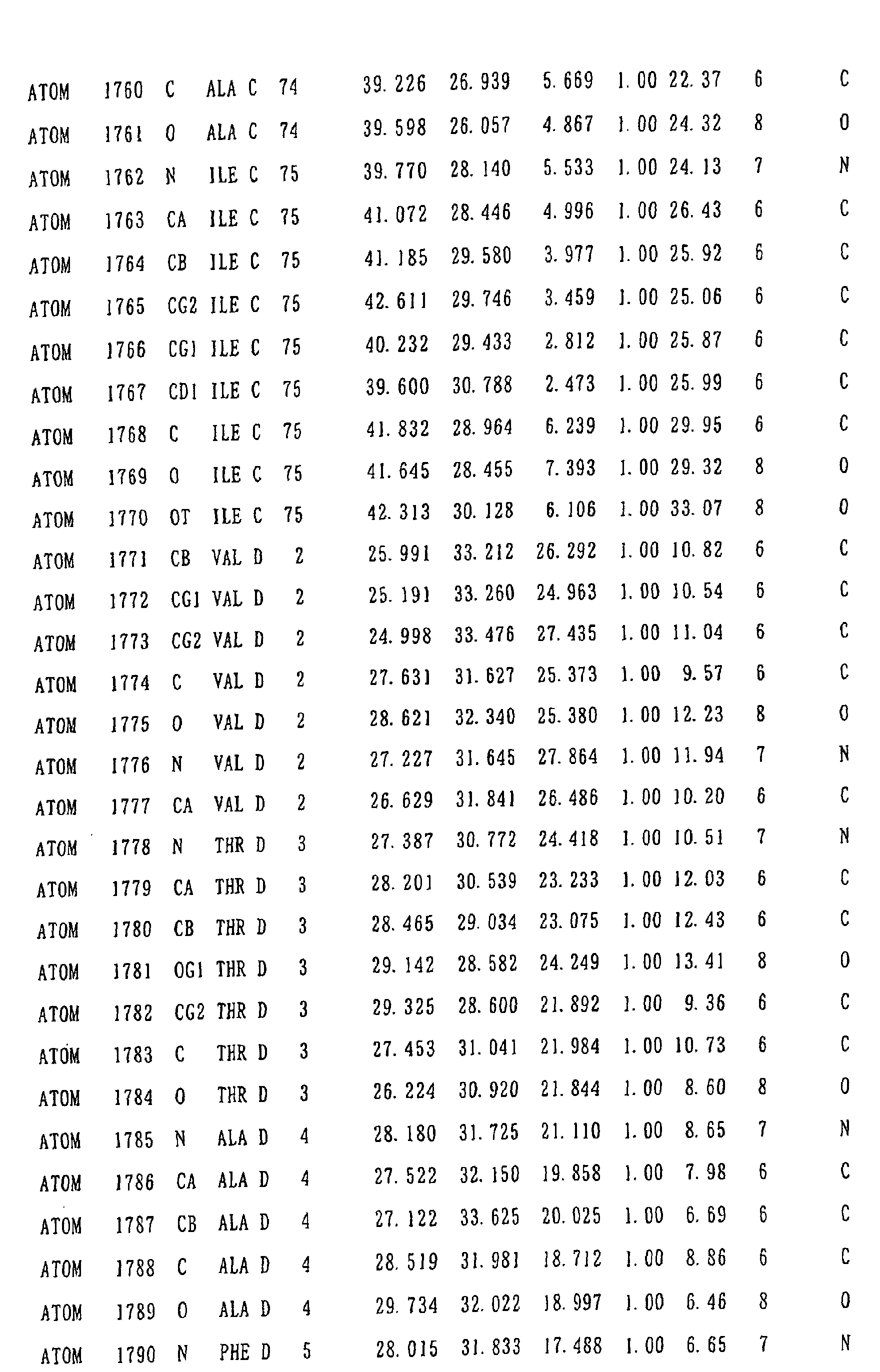

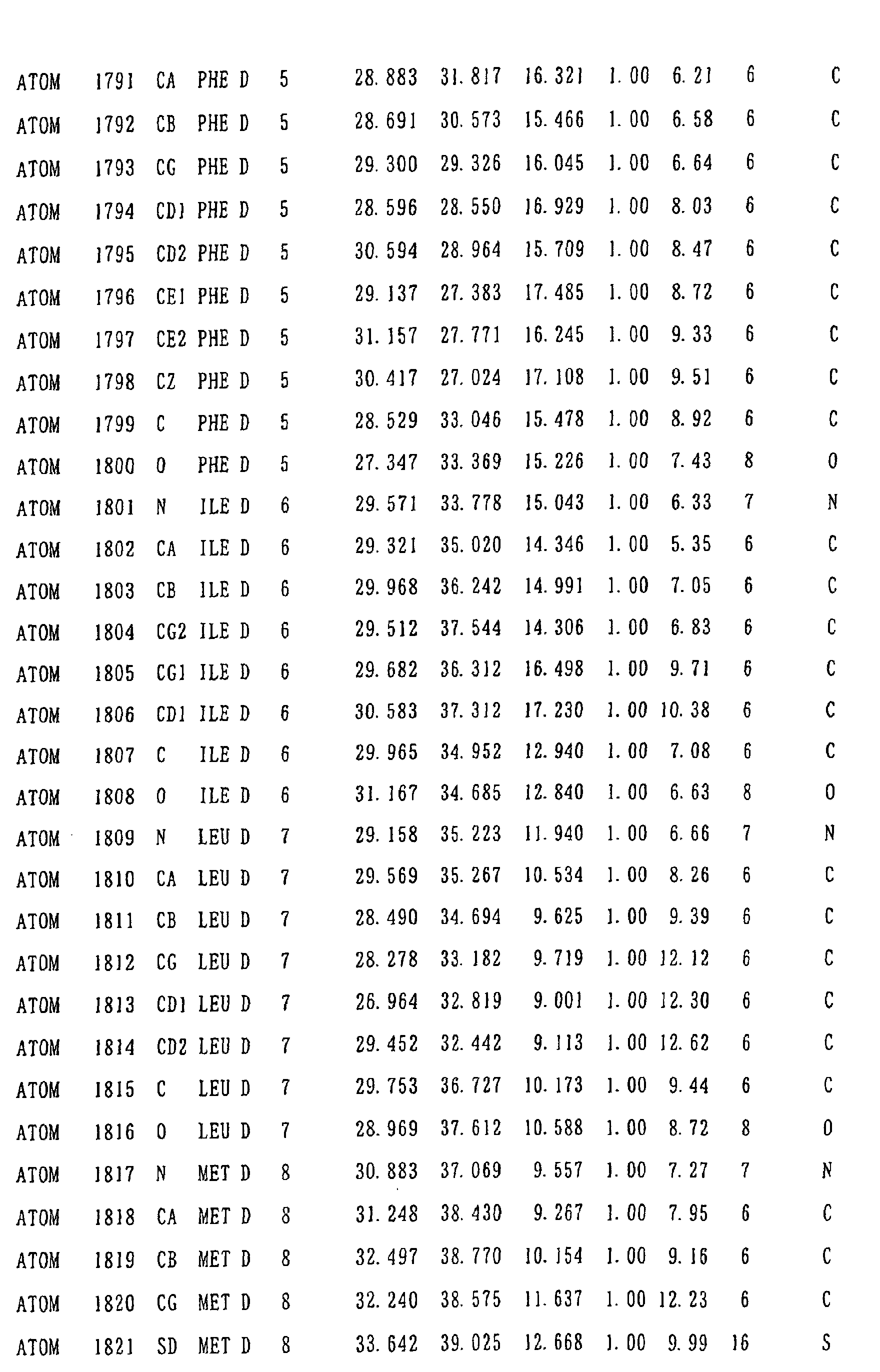

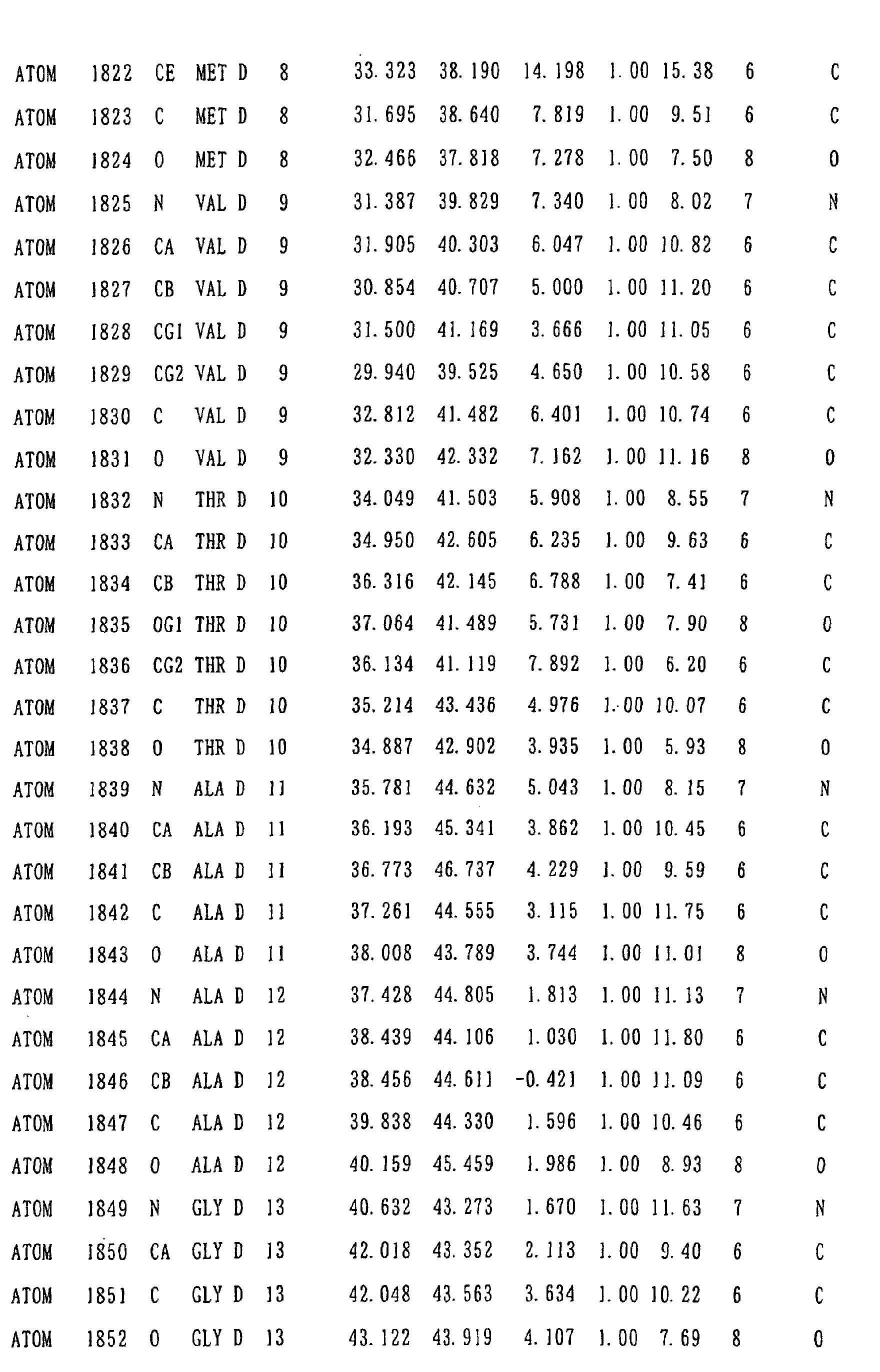

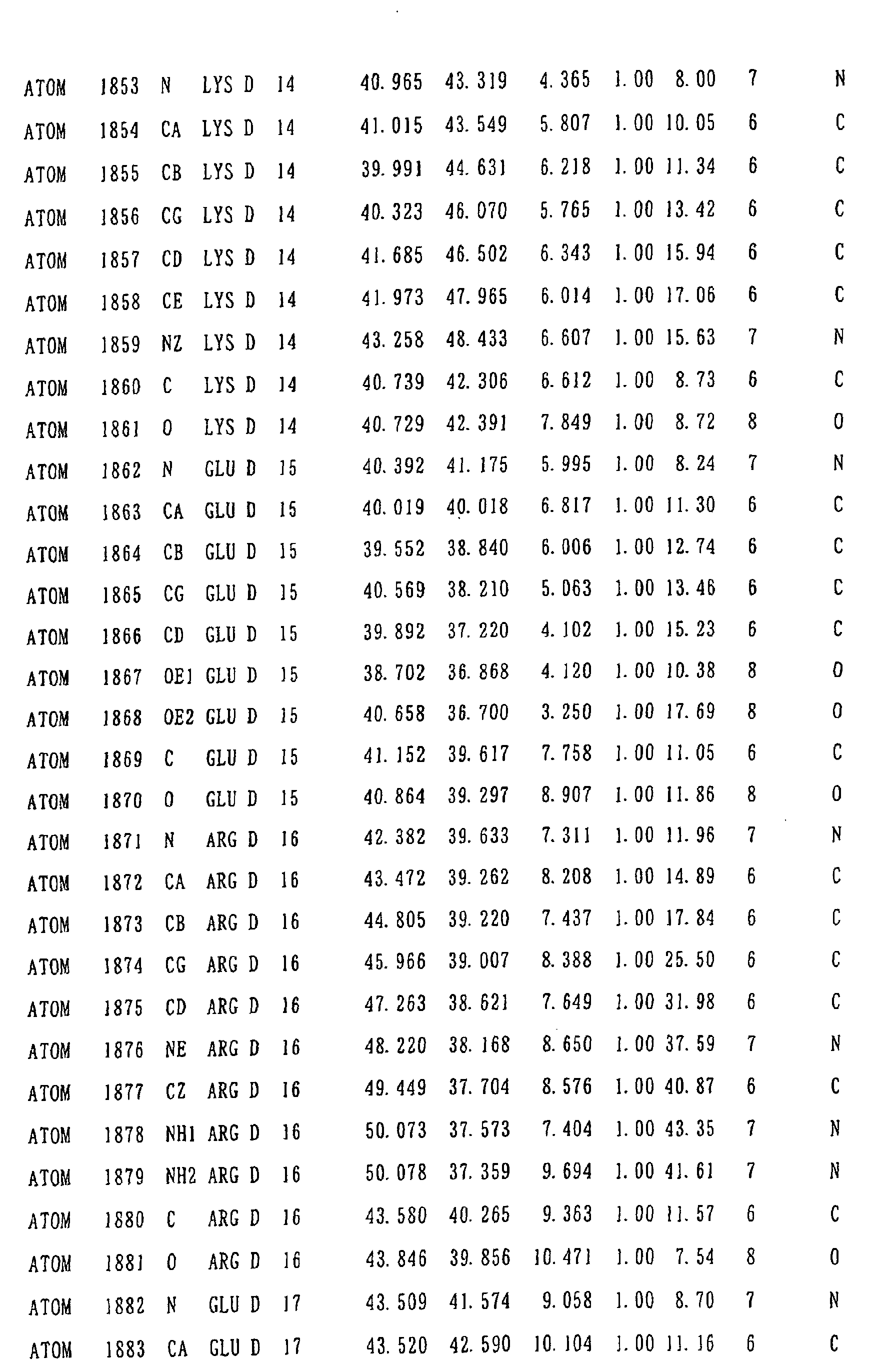

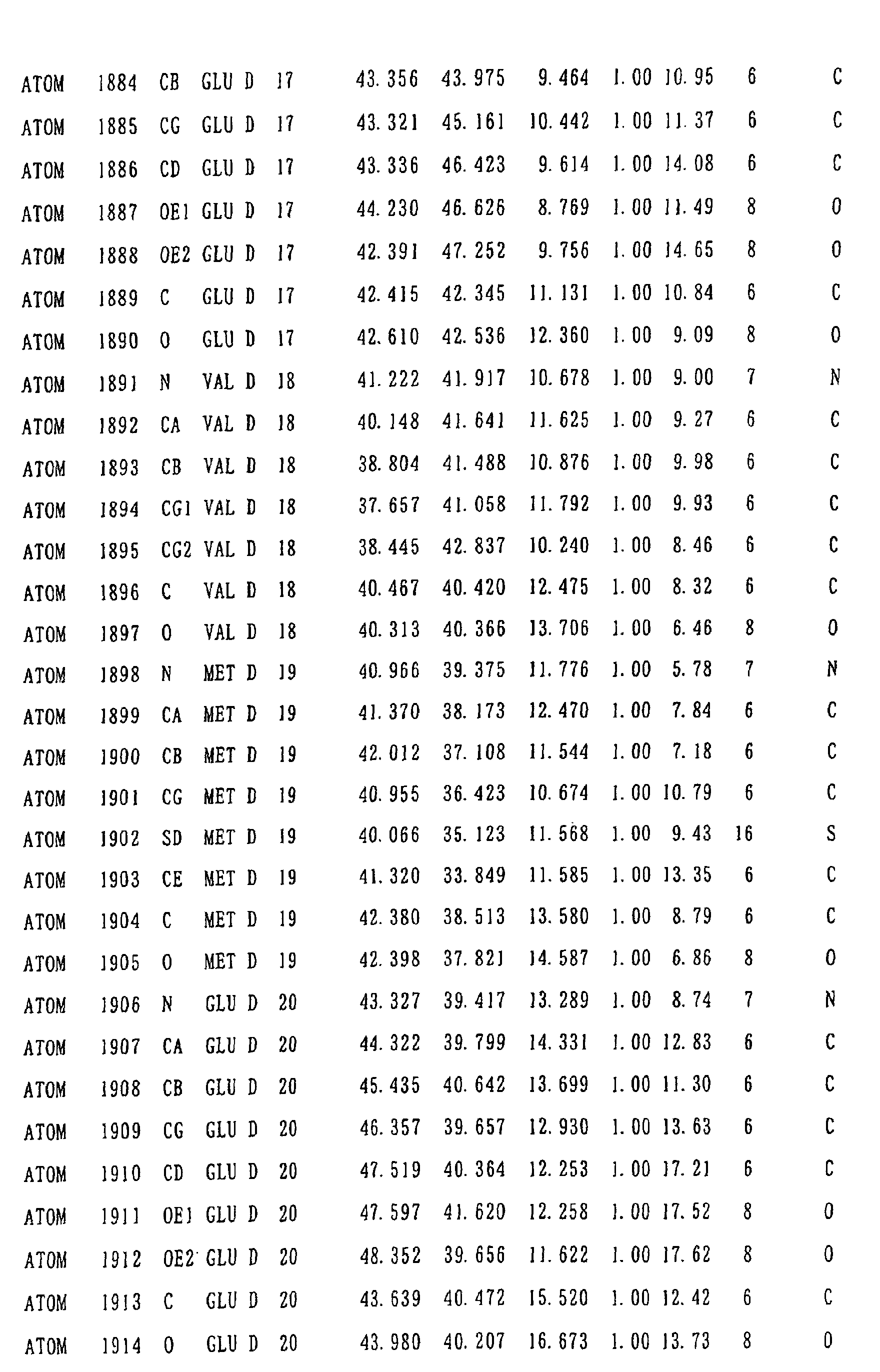

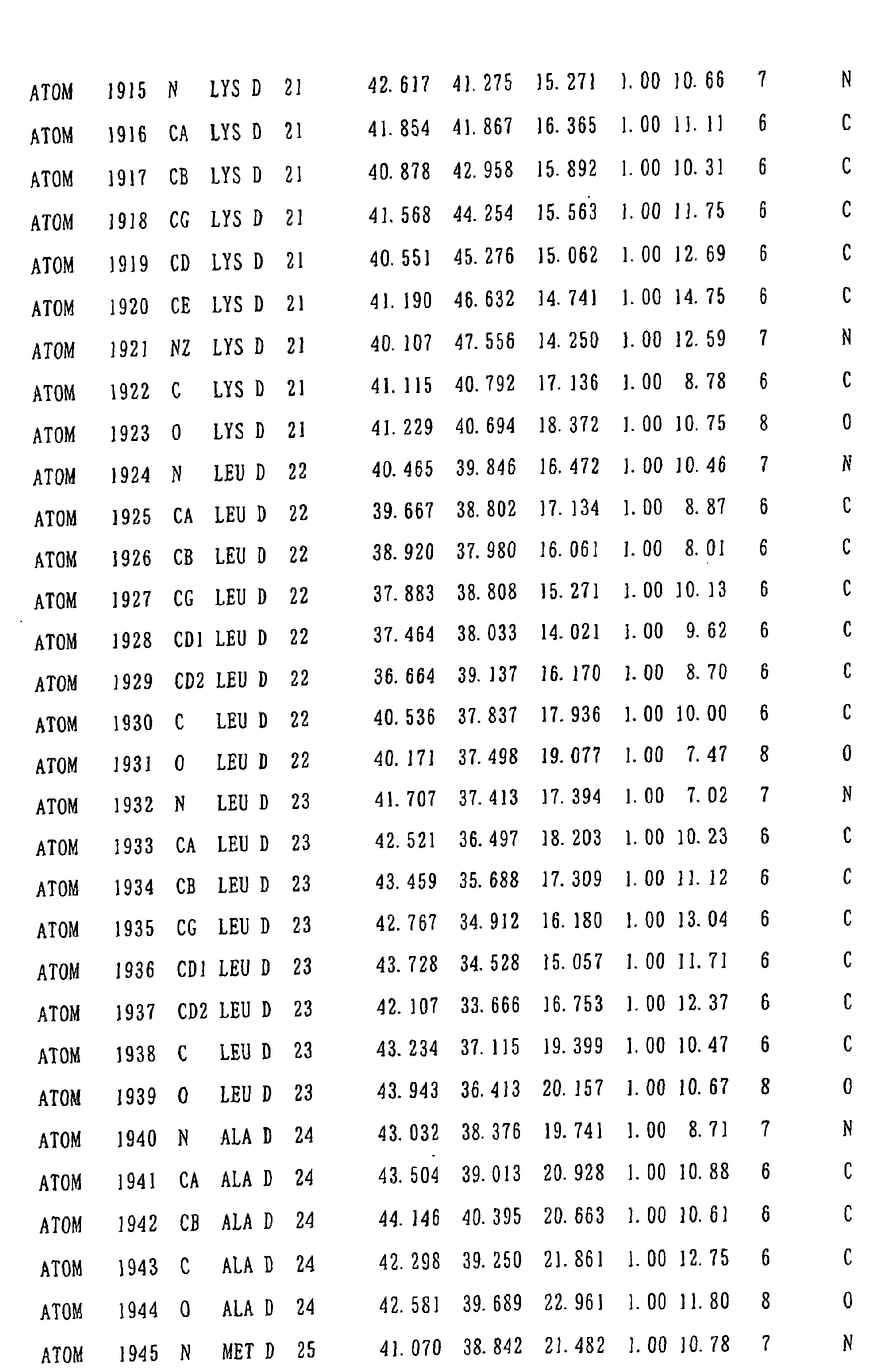

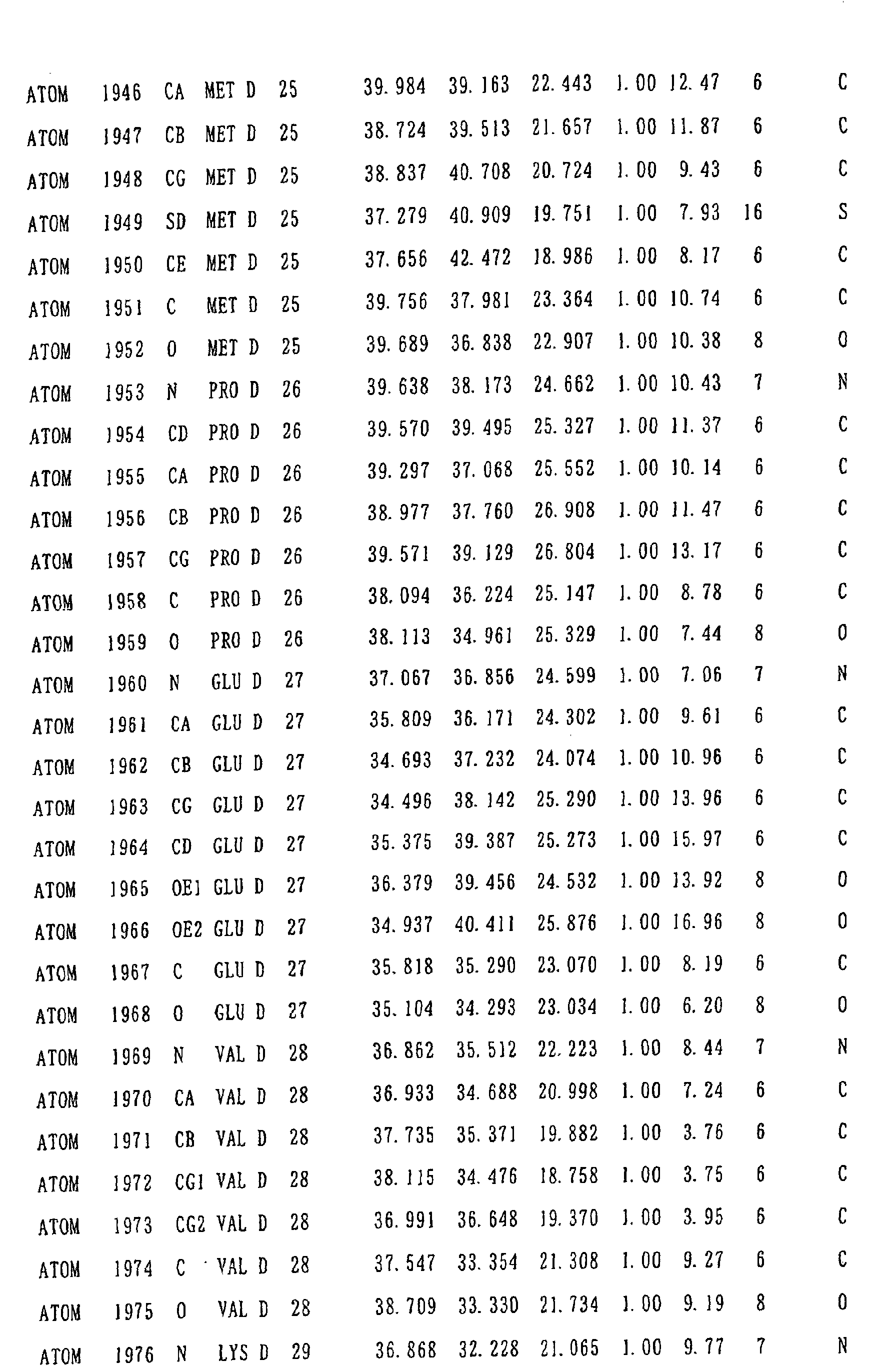

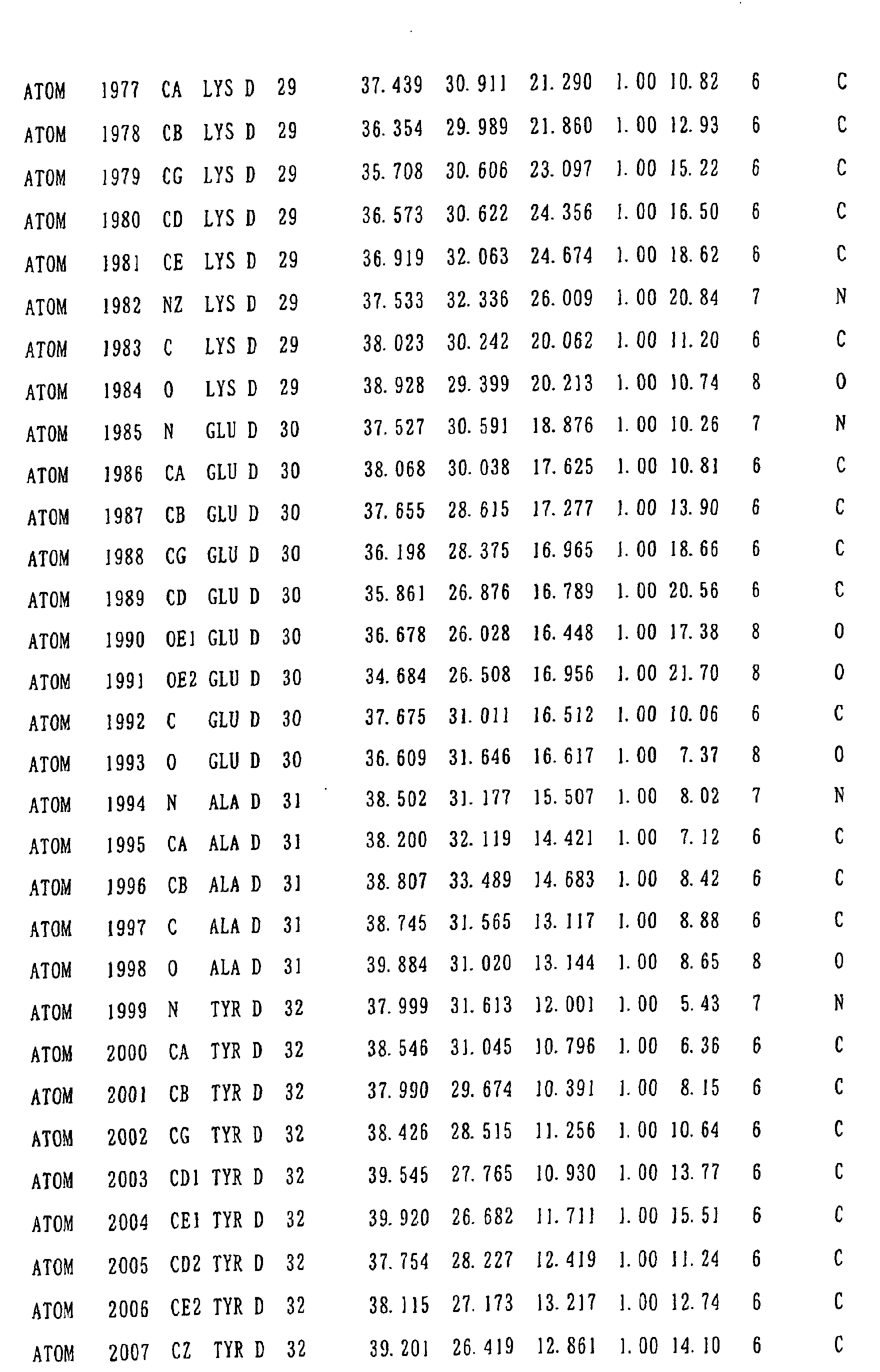

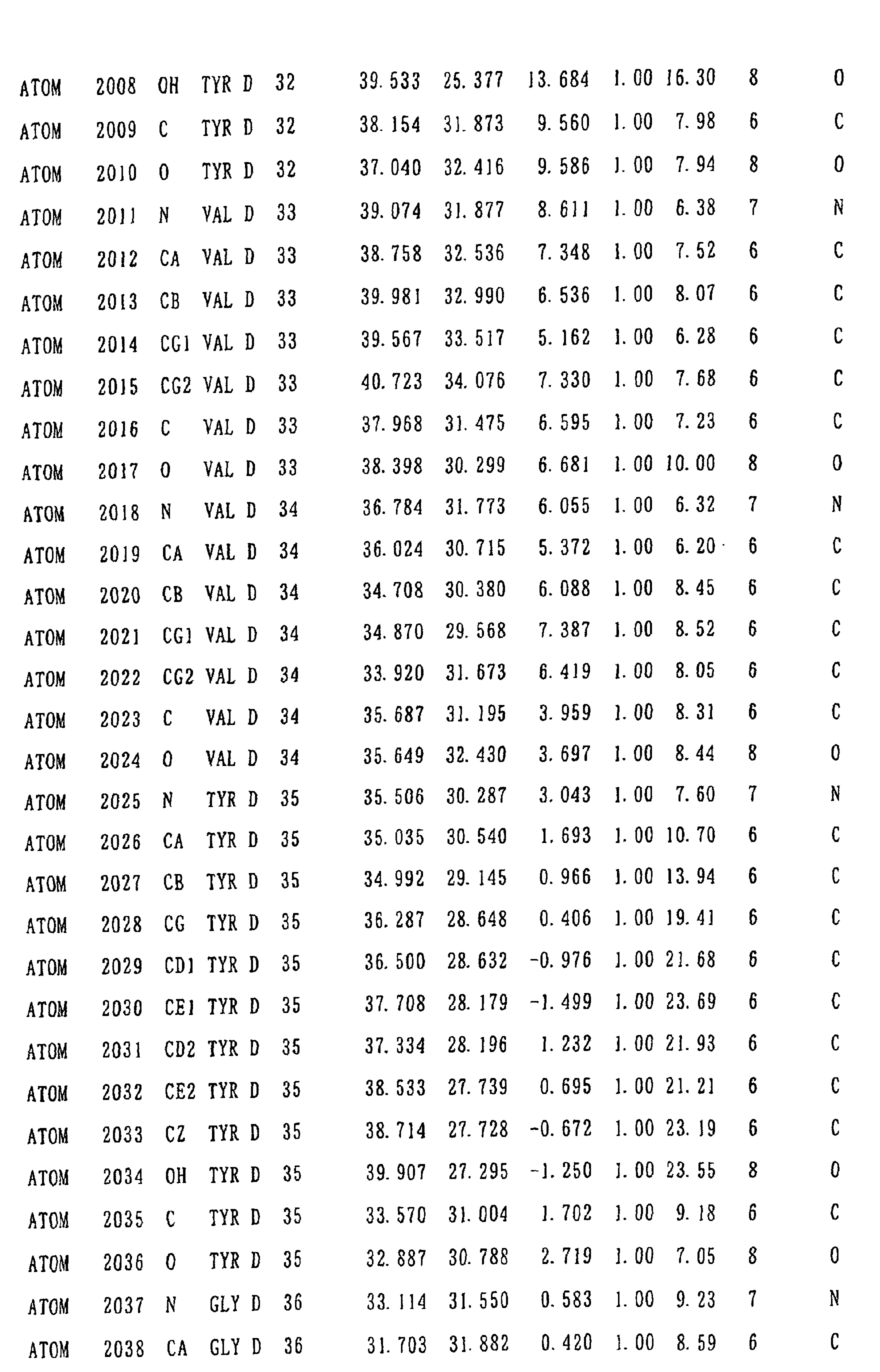

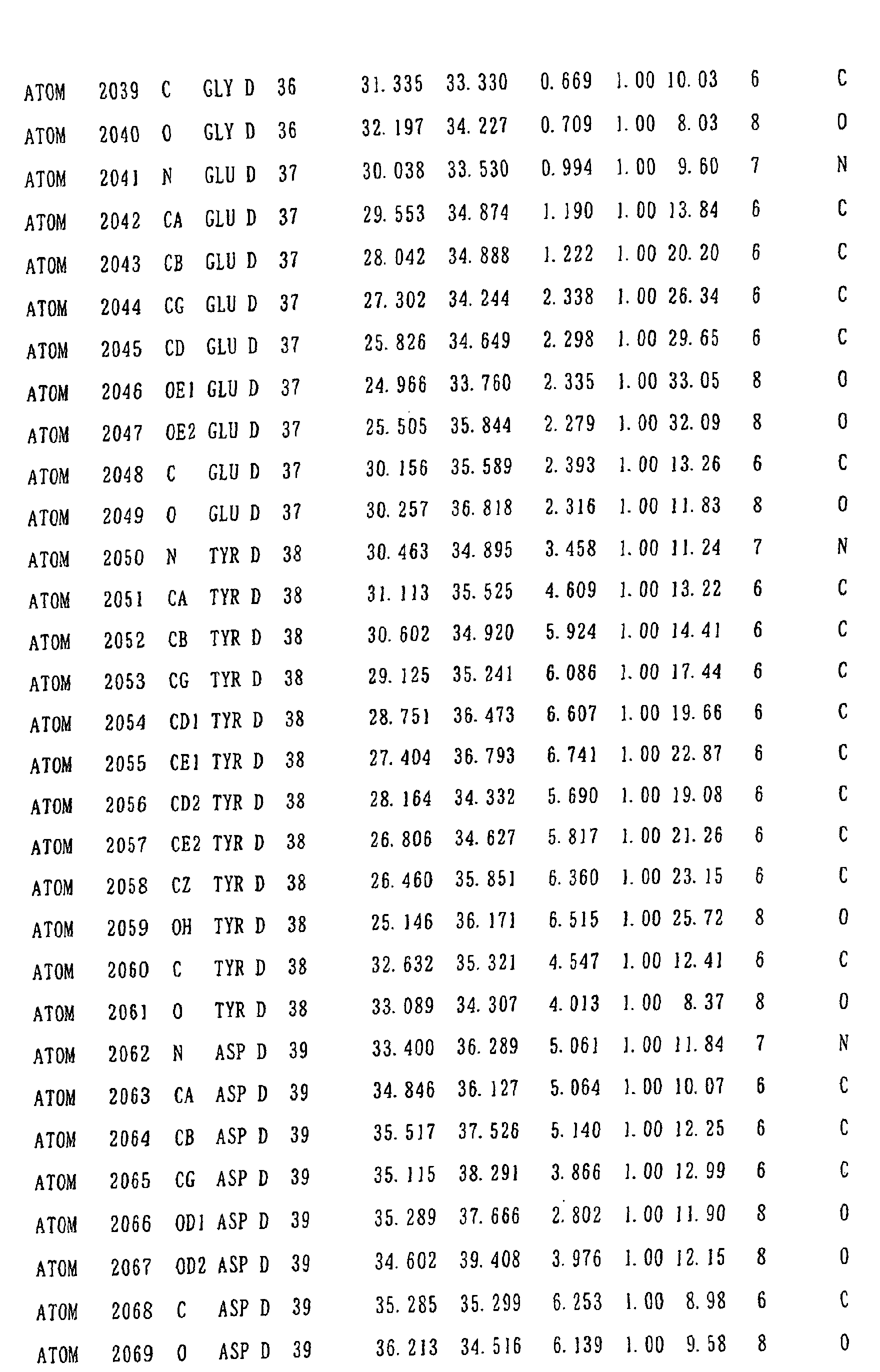

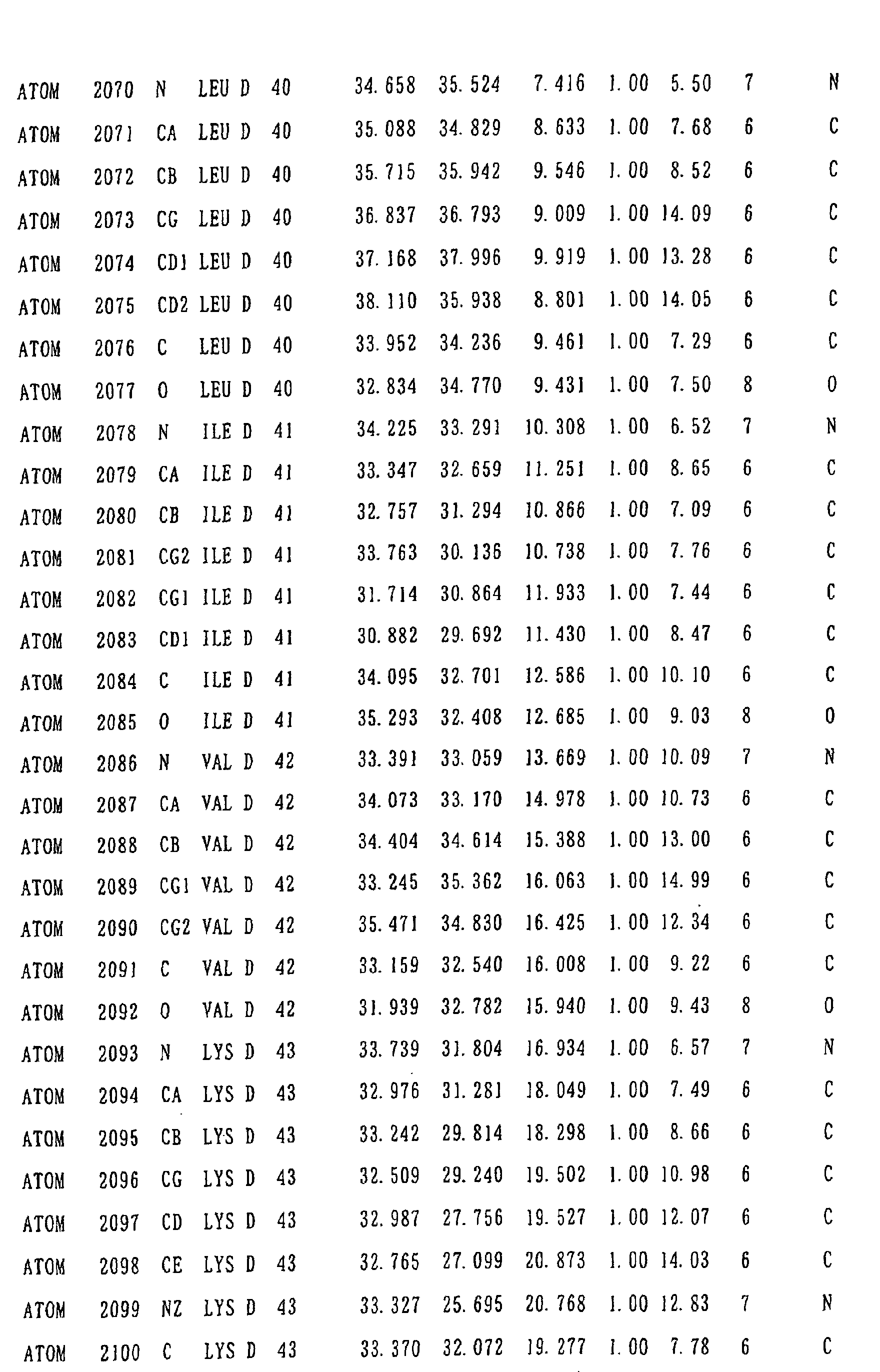

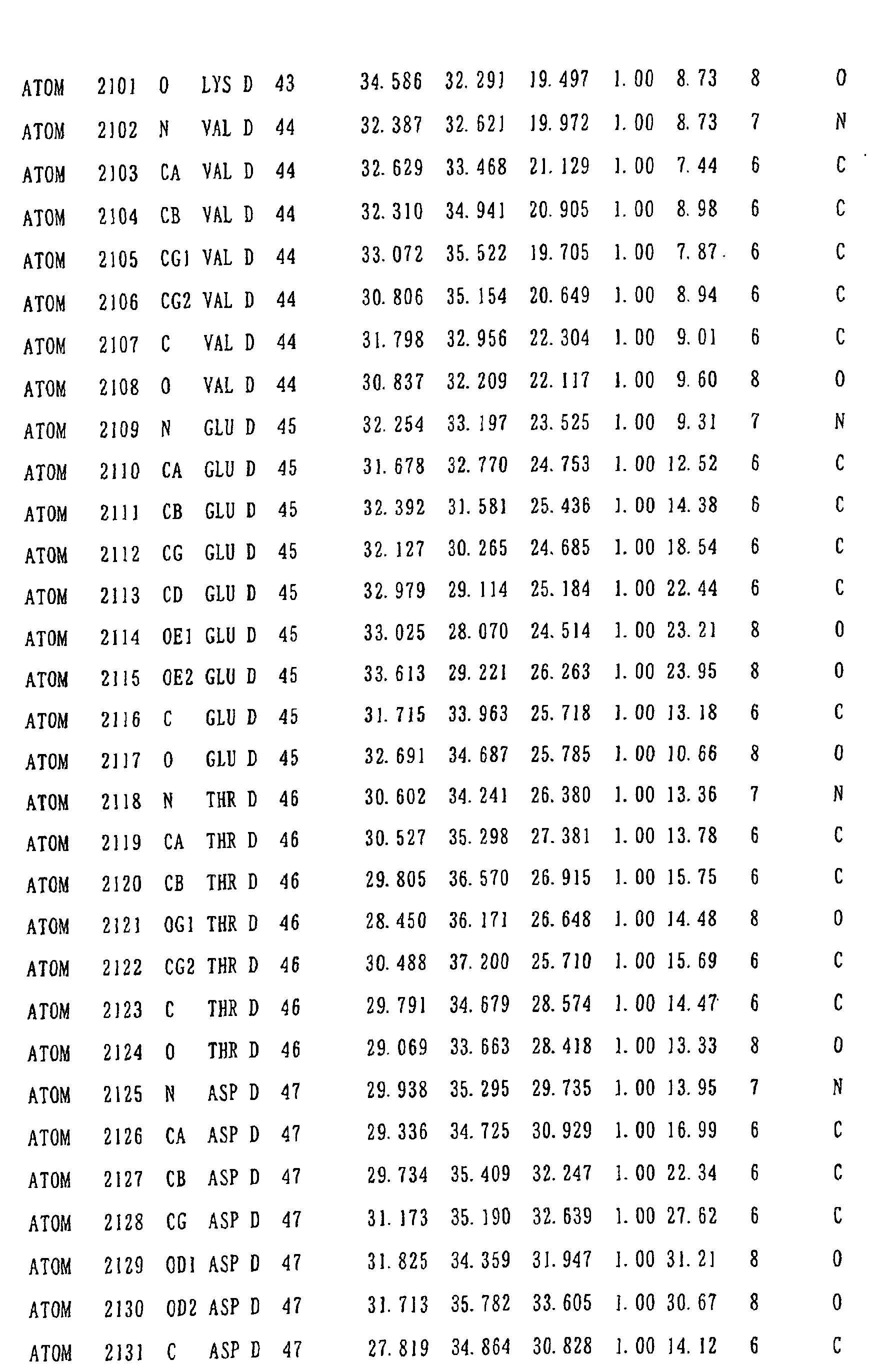

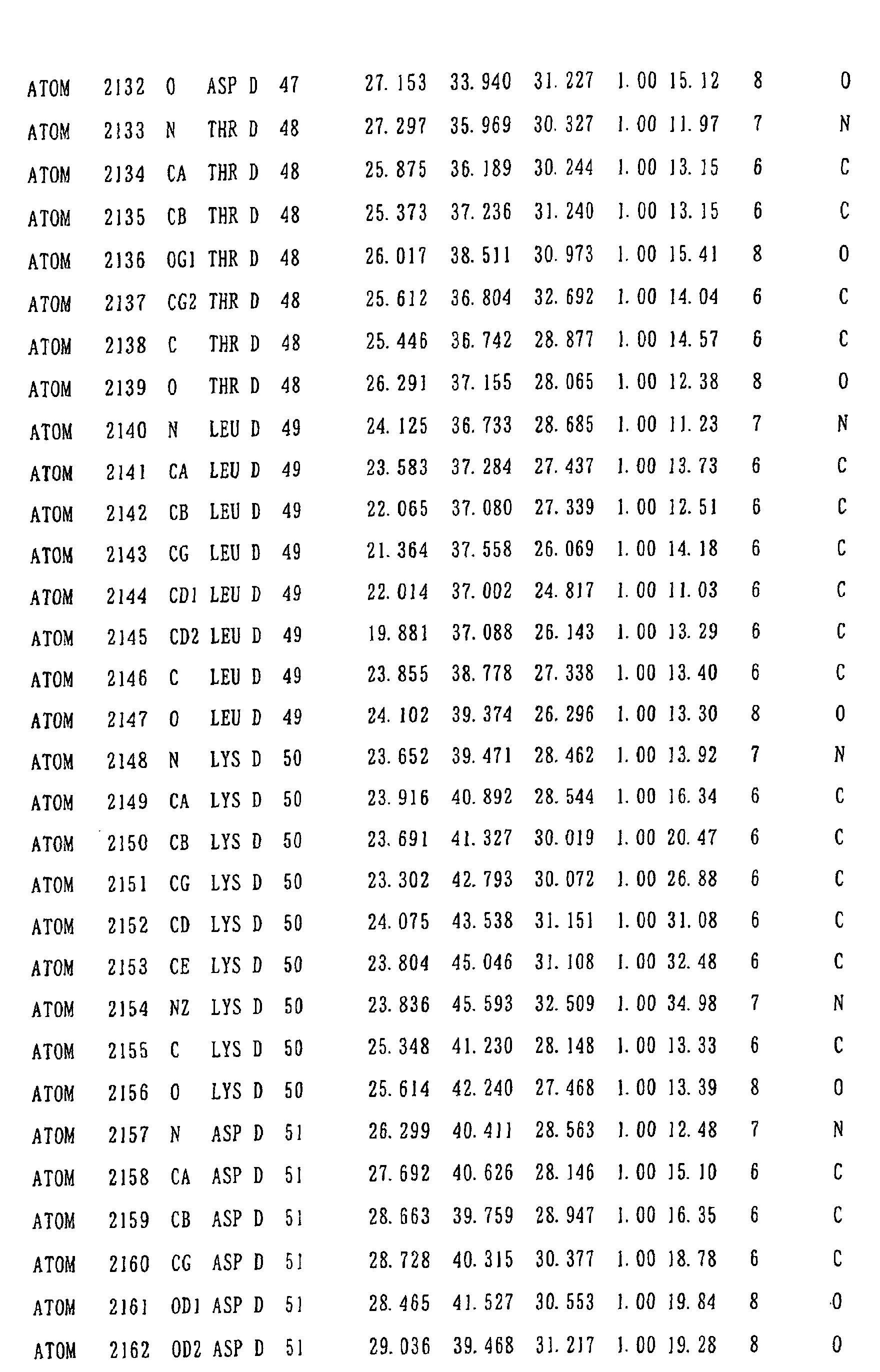

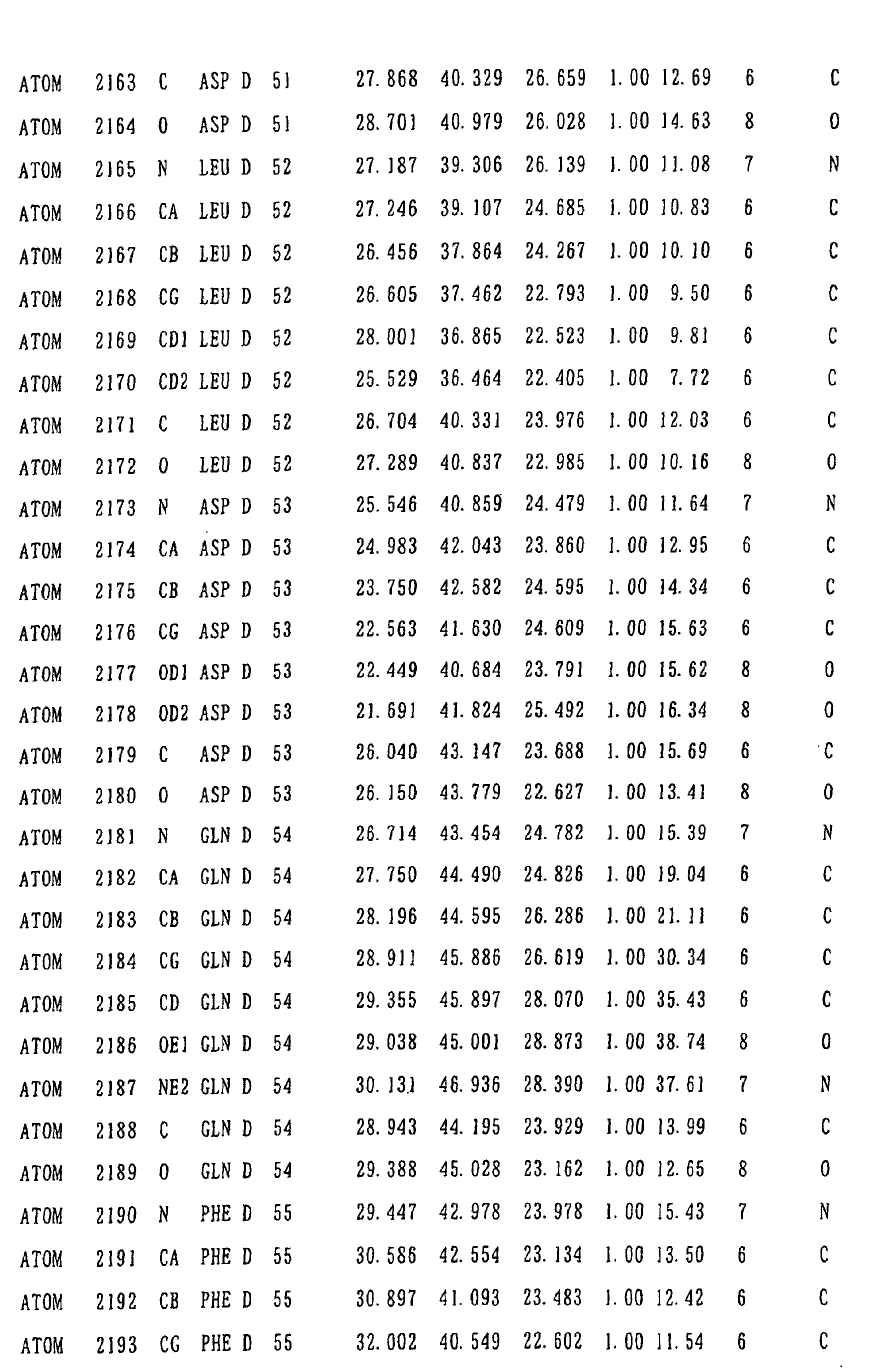

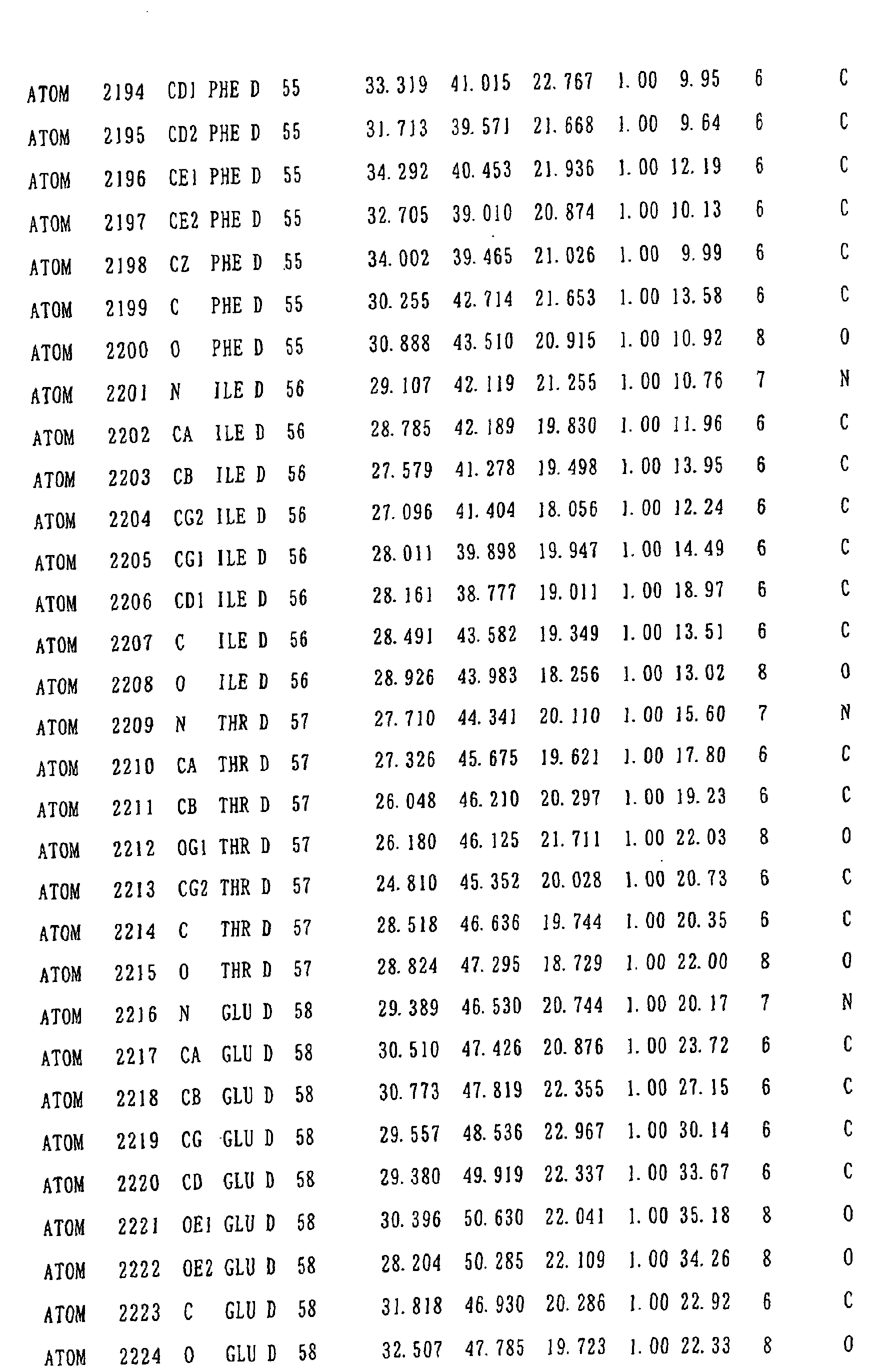

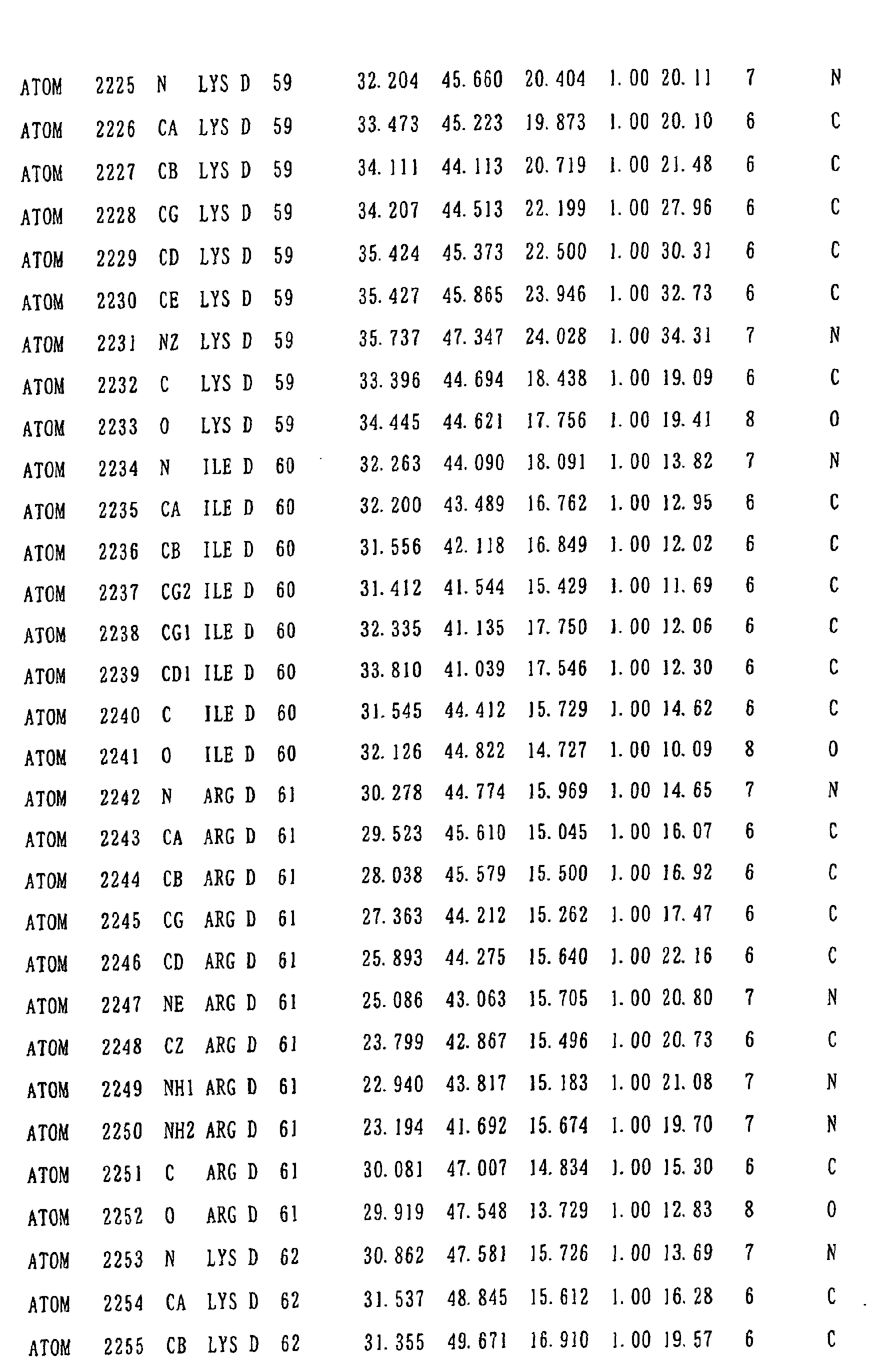

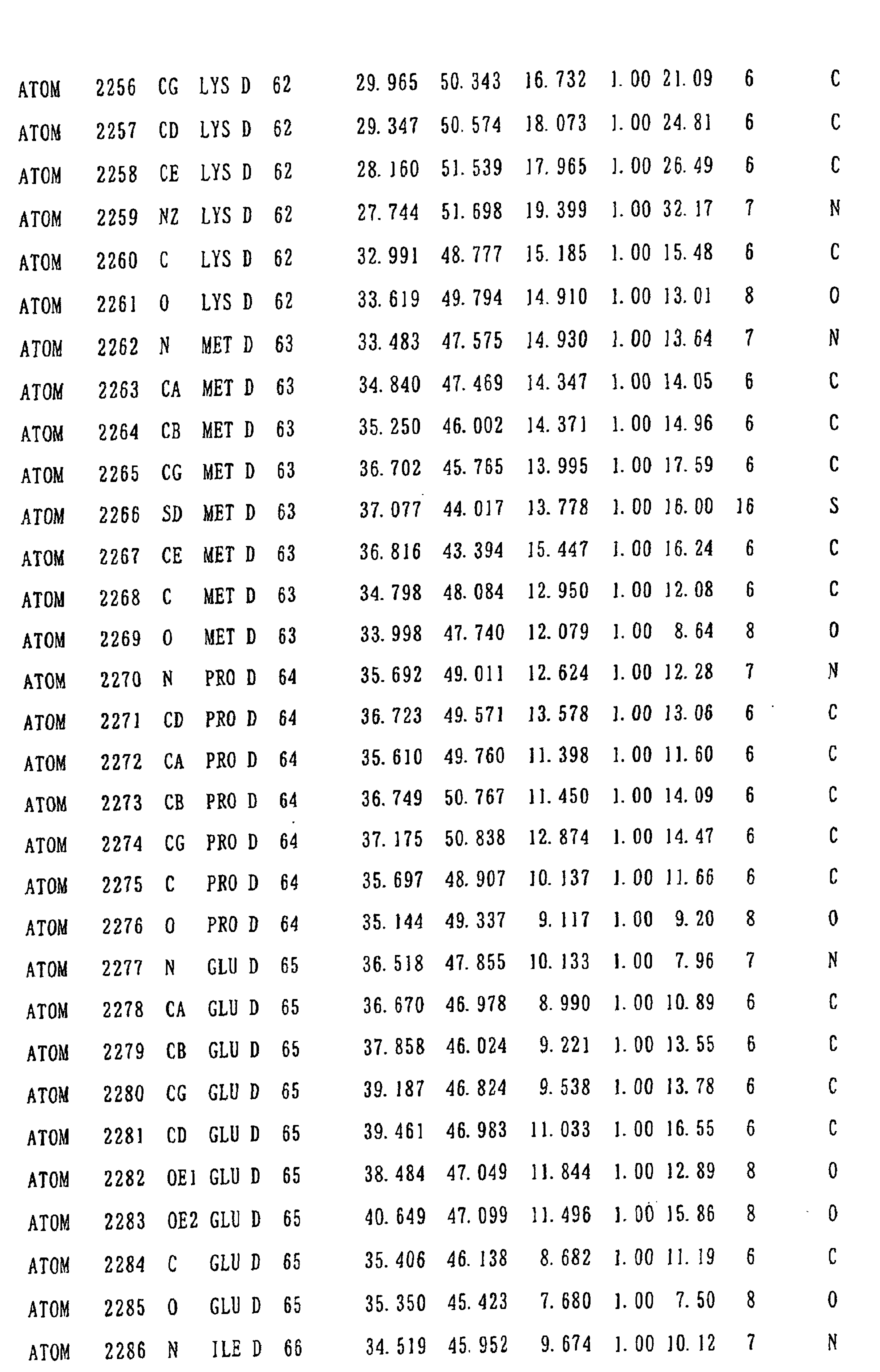

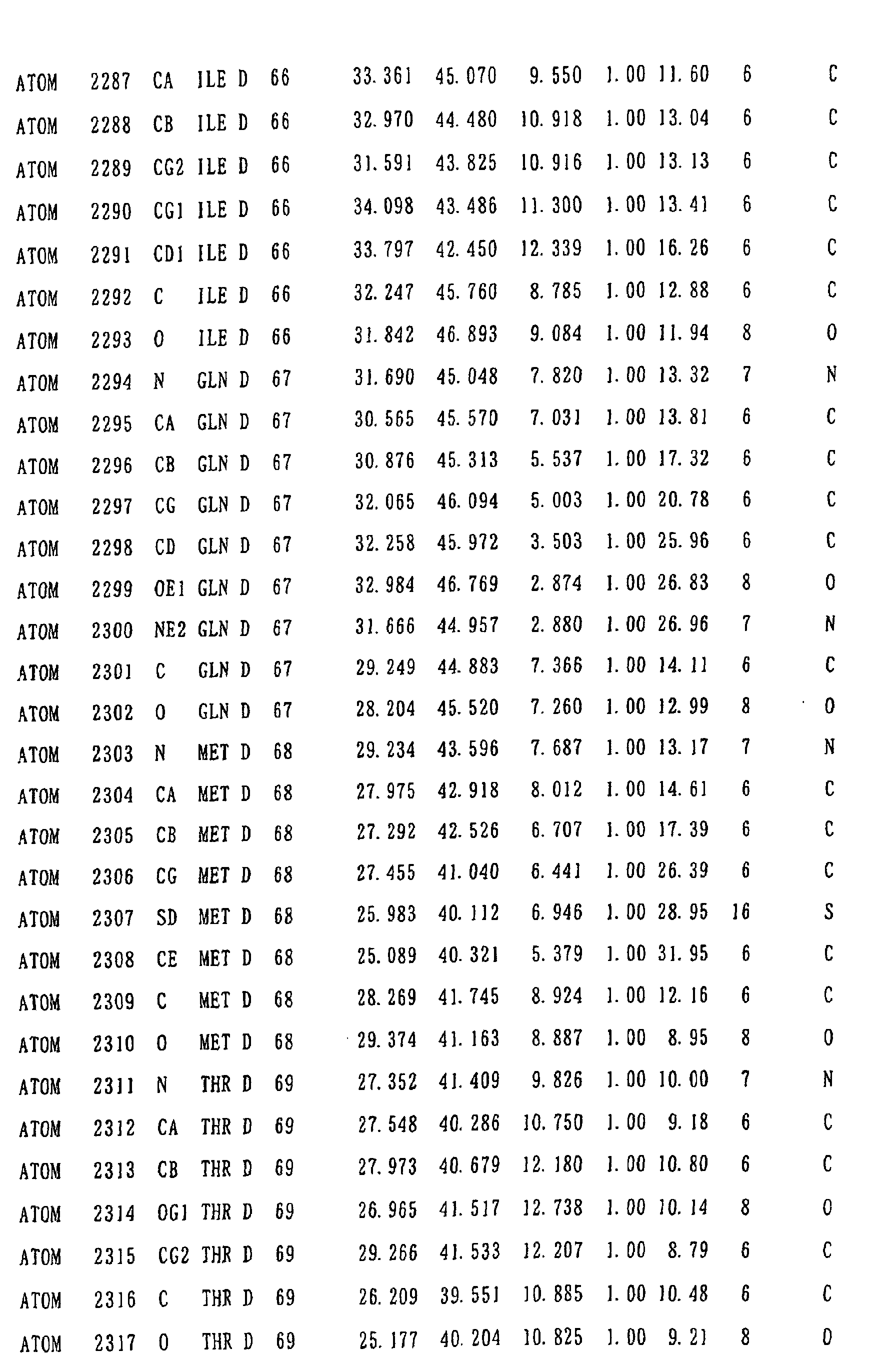

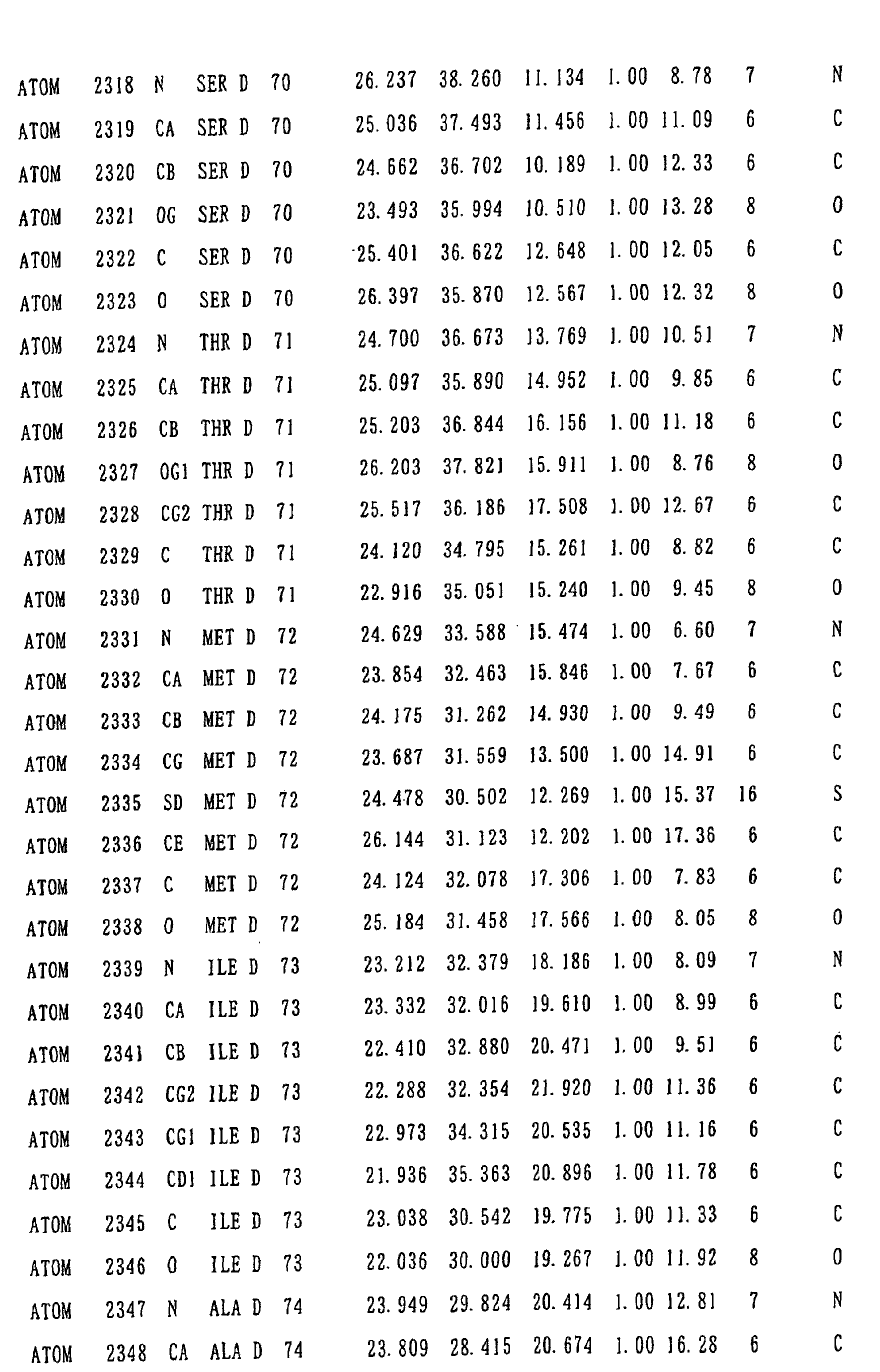

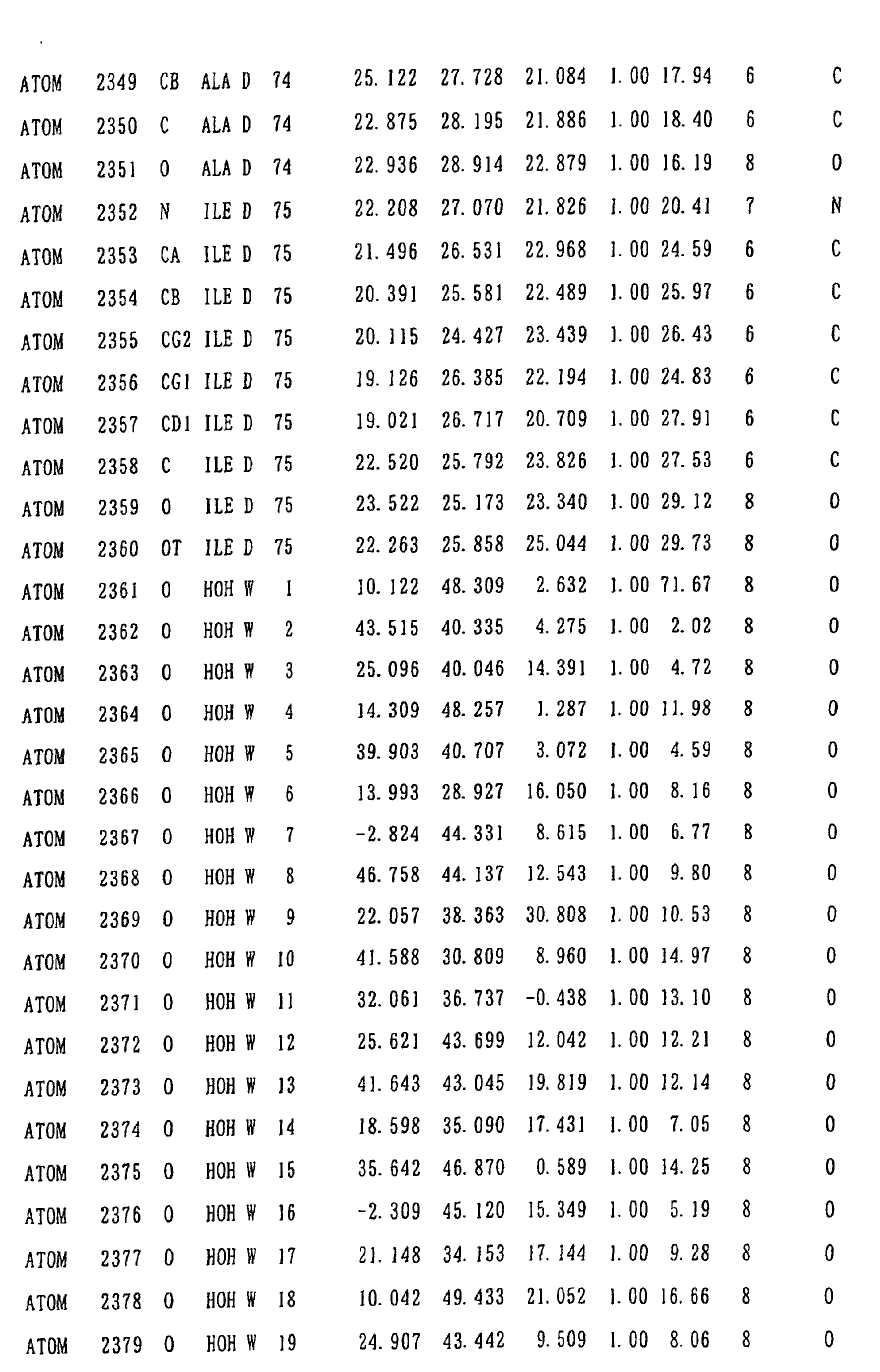

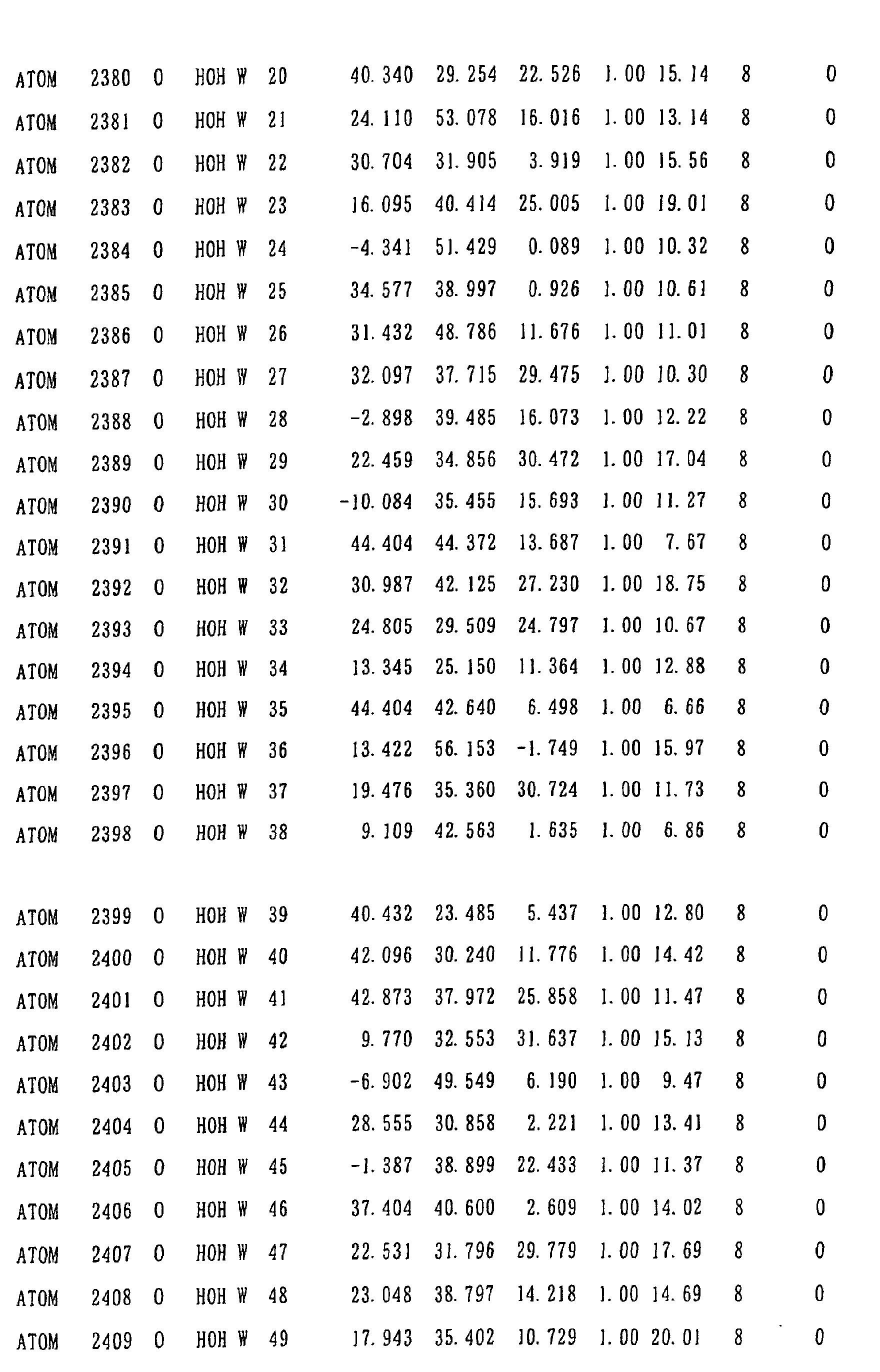

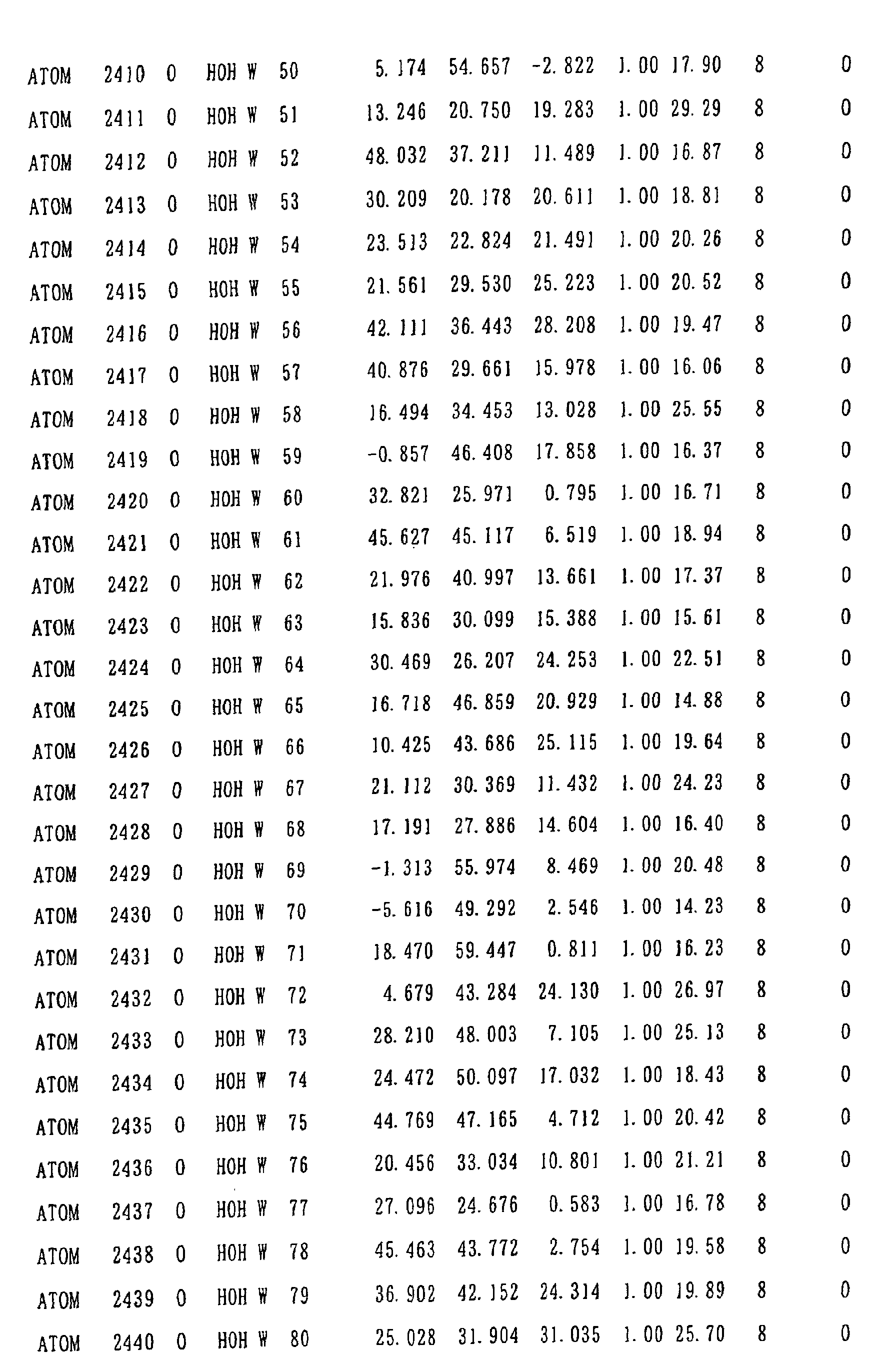

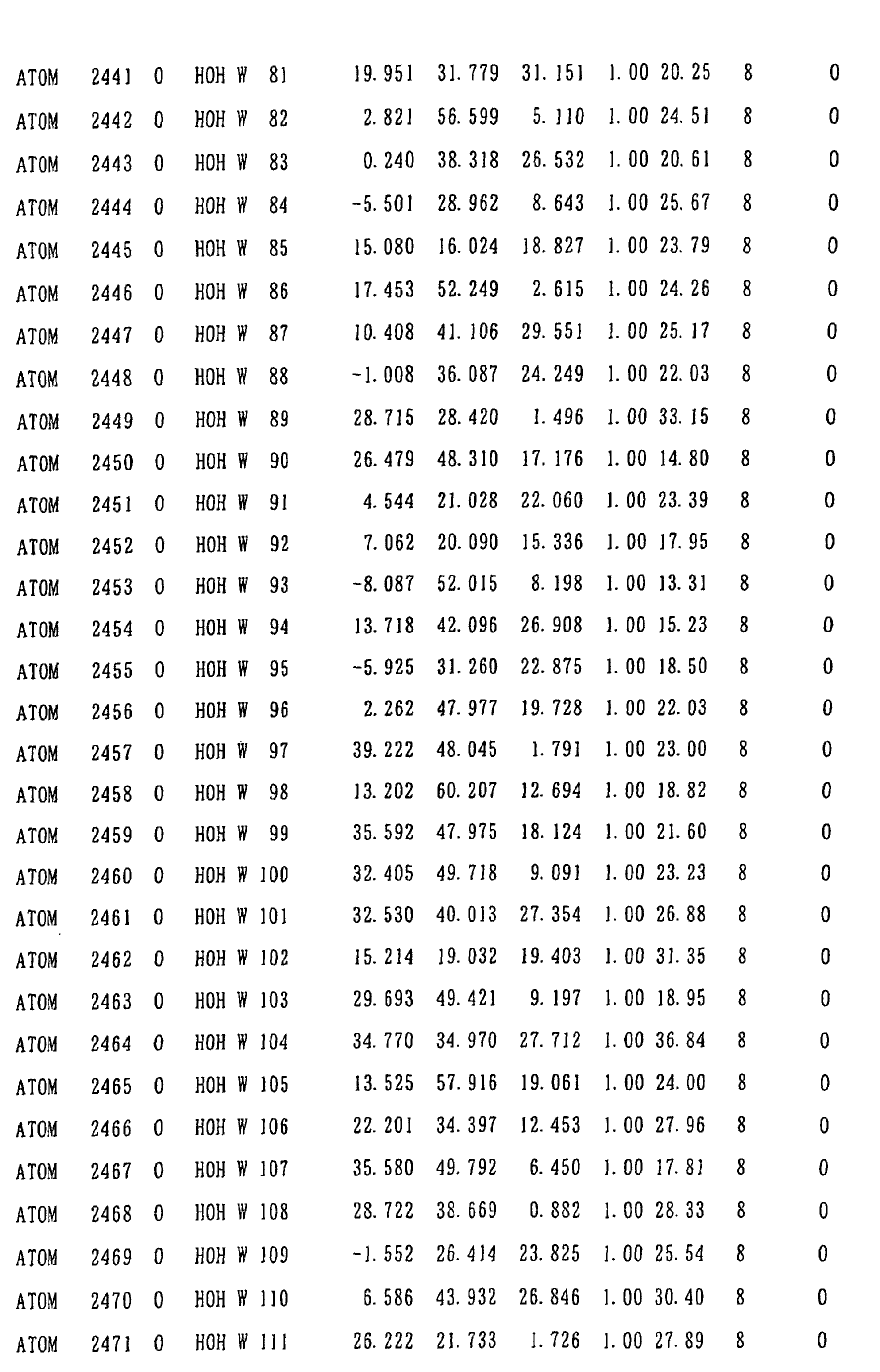

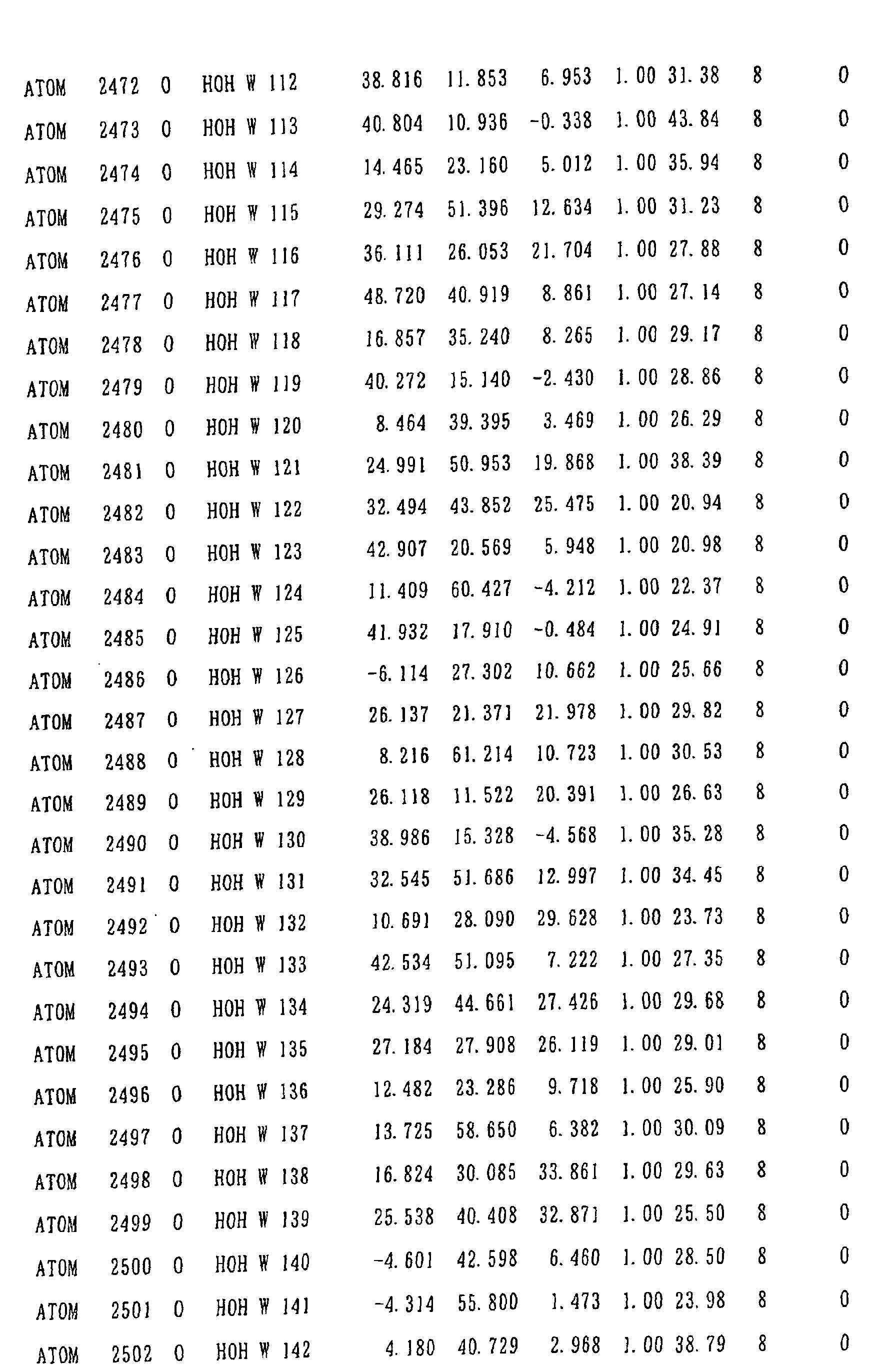

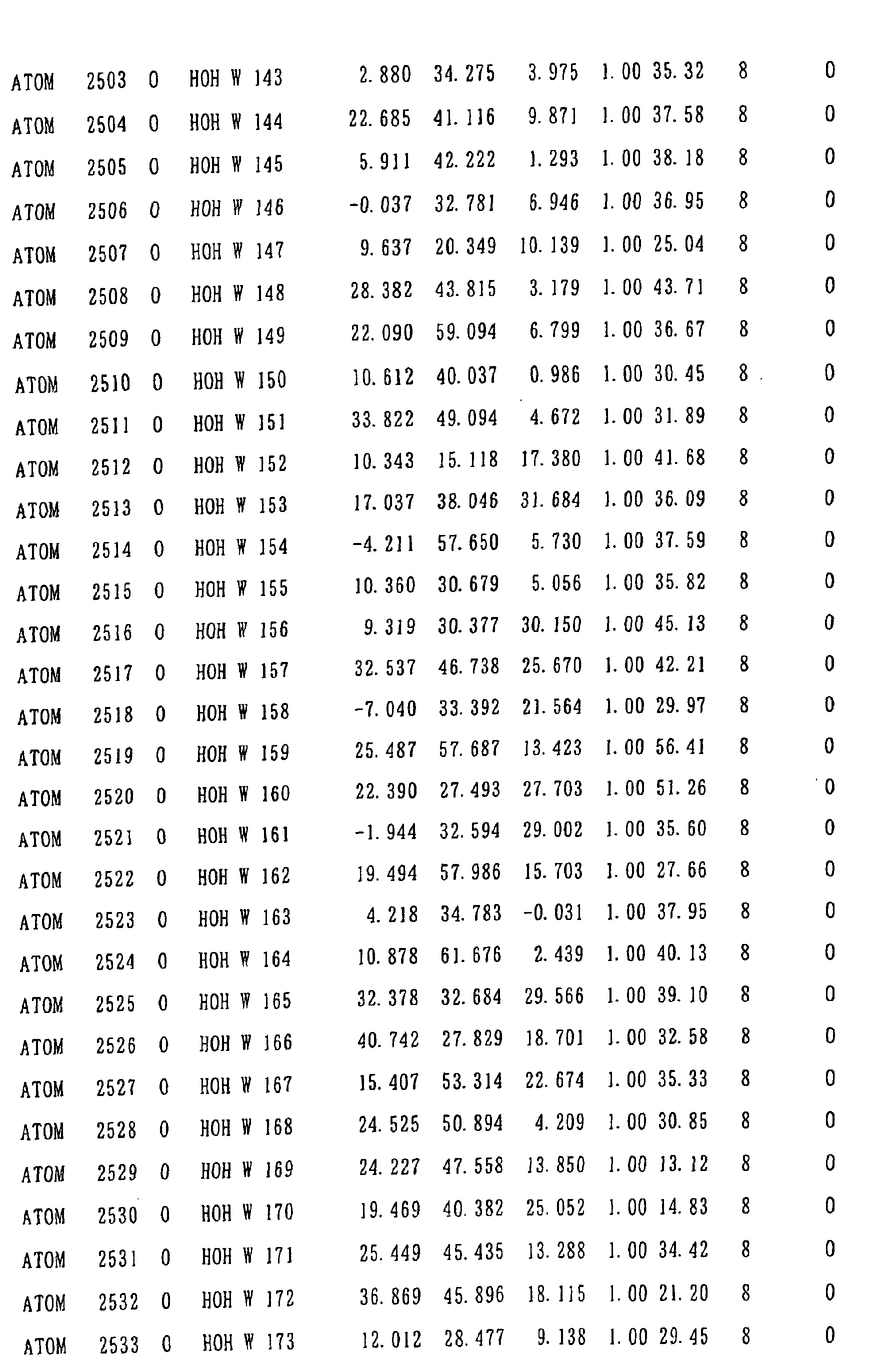

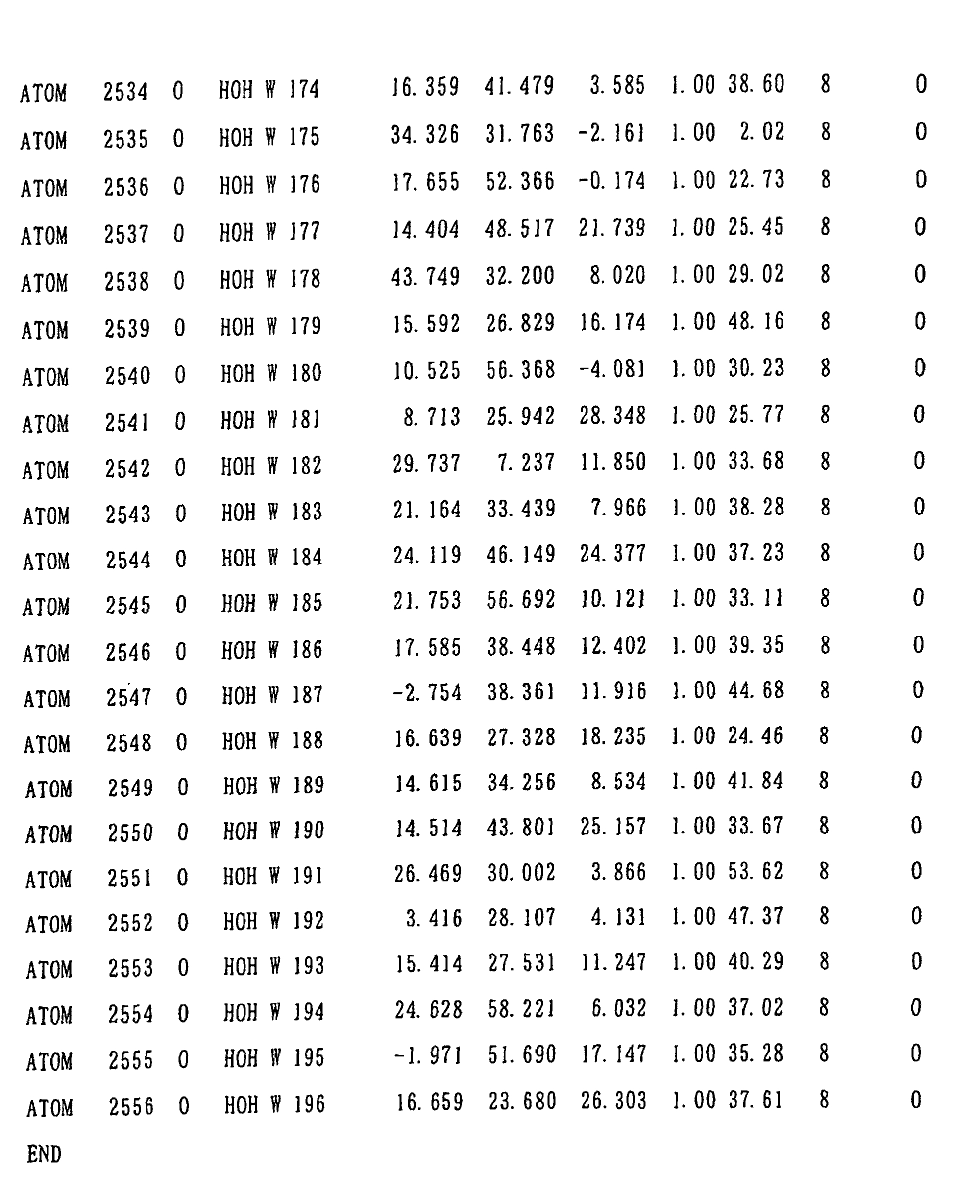

All publications, patents, and patent applications cited herein are incorporated herein by reference in their entirety.

### Industrial Applicability

Lrp-like proteins activate or inactivate transcription of a wide range of genes of microorganisms, which are involved in metabolism or growth of microorgansims, or their infection. Accordingly, a ligand that alters the 3D structure of an Lrp-like protein, or that regulates the function of an Lrp-like protein by changing its 3D structure or by affecting its ability to assemble, can be used as a pharmaceutical agent in order to regulate metabolism of, growth of, or infection by microorganisms.

So far no Lrp-like protein has been found in eukaryotes, including humans. Accordingly, by the use of such a ligand no serious side effects is expected to humans. The target of the ligand, i.e., an Lrp-like protein, and its mechanism of action are different from those of known antibiotics. This might prevent infection by microorganisms which are resistant to known antibiotics; such infection is becoming more and more serious.

The present invention provides a highly useful method for screening for the aforementioned ligand. Thus, this can serve as an innovative method for developing pharmaceutical agents that can complement known antibiotics through a radically new approach.

### Free Text of Sequence Listing

SEQ ID NO: 1: description of artificial sequence: primer
SEQ ID NO: 2: description of artificial sequence: primer

## Claims

1. A method of screening for pharmaceutical agents that regulates at least one of metabolism or growth of, or infection by microorganisms, which comprises selecting ligands of an Lrp-like protein based on 3D structural information of the protein.

2. The method according to claim 1, wherein the 3D structural information is information concerning gaps pockets generated by assembly of Lrp-like protein monomers.

3. The method according to claim 1 or 2, wherein the 3D structural information is information obtained from 3D atomic coordinates determined by X-ray diffraction analysis.

4. The method according to claim 3, which comprises the following steps:
1) determination of the 3D atomic coordinates of the Lrp-like protein by applying X-ray diffraction analysis to the protein crystallized;
2) processing of the atomic coordinates using a program designed for analyzing protein 3D structures, thereby obtaining graphical and/or numerical information concerning the 3D structure of the Lrp-like protein;
3) deduction of the 3D structural information on gaps or pockets generated by assembling of monomers of the Lrp-like protein, by using the graphical and/or numerical information; and
4) identification of ligands which will interact with the Lrp-like protein and thus will be usable as pharmaceutical agents, by using the 3D structural information.

5. The method according to any one of claims 1 to 4, wherein the pharmaceutical agents that regulate at least one of metabolism or growth of, or infection by microorganisms, are antimicrobial agents.

6. The method according to any one of claims 1 to 5, wherein the Lrp-like protein is derived from a microorganism belonging to the genus *Pyrococcus*.

7. The method according to claim 6, wherein the microorganism is *Pyrococcus* sp. OT3 (JCM 9974).

8. The method according to claim 7, wherein the 3D atomic coordinates comprise the data shown in Table 1.

9. A recording medium in which the following a) and/or b) are recorded:
a) 3D atomic coordinates of an Lrp-like protein; and
b) graphical and/or numerical information concerning the 3D structure of an Lrp-like protein obtained by analyzing the 3D atomic coordinates using a program designed for analyzing protein 3D structures.

10. The recording medium according to claim 9, wherein the Lrp-like protein is derived from a microorganism belonging to the genus *Pyrococcus.*

11. The recording medium according to claim 10, wherein the microorganism is *Pyrococcus* sp. OT3 (JCM 9974).

12. The recording medium according to claim 11, wherein the 3D atomic coordinates comprise the data shown in Table 1.


SEQUENCE LISTING

<110> National Institute of Advanced Industrial Science and Technology
      Japan Science and Technology Corporation

<120> Method of screening for pharmaceutical agents
<130> PH-1708-PCT

<150> JP 2001-384683
<151> 2001-12-18

<160> 2

<170> PatentIn Ver. 2.1

<210> 1
<211> 33
<212> DNA
<213> Artificial Sequence

<220>
<223> Description of Artificial Sequence:primer

<400> 1
tggtgatgac atatggtgac ggcctttatc ctg                                33

<210> 2
<211> 36

<212> DNA
<213> Artificial Sequence

<220>
<223> Description of Artificial Sequence:primer

<400> 2
gaacggatcc atcaaattgc tatcatagtc gaggtc 36